# EUROPEAN PATENT SPECIFICATION

(11) **EP 1 713 778 B1**
(45) Date of publication and mention of the grant of the patent: **16.01.2008**
(21) Application number: 04803989.5
(22) Date of filing: 15.12.2004
(51) Int. Cl.: C07D 223/16, C07D 405/12, C07D 401/04, C07D 413/12, C07D 403/12, C07D 409/12, C07D 471/04, C07D 417/12, C07D 401/14, C07D 401/12, C07D 403/14, C07D 405/14, A61K 31/55, A61P 25/28

(54) **BENZAZEPINE DERIVATIVES AS HISTAMINE H3 ANTAGONISTS**
BENZAZEPINDERIVATE ALS HISTAMIN-H3-ANTAGONISTEN
DERIVES DE BENZAZEPINE UTILISES COMME ANTAGONISTES DE L'HISTAMINE H3

(30) Priority: 17.12.2003 GB 0329214
(43) Date of publication of application: 25.10.2006
(73) Proprietor: GLAXO GROUP LIMITED, Greenford, Middlesex UB6 ONN (GB)
(72) Inventor: BAILEY, Nicholas GlaxoSmithKline, Harlow Essex CM19 5AW (GB); BAMFORD, Mark, James GlaxoSmithKline, Harlow Essex CM19 5AW (GB); DEAN, David Kenneth GlaxoSmithKline, Harlow Essex CM19 5AW (GB); PICKERING, Paula, Louise GlaxoSmithKline, Harlow Essex CM19 5AW (GB); WILSON, David, Matthew GlaxoSmithKline, Harlow Essex CM19 5AW (GB); WITHERINGTON, Jason GlaxoSmithKline, Harlow Essex CM19 5AW (GB)
(74) Representative: Goddard, Carolyn Janice
(86) International application number: PCT/EP2004/014380
(87) International publication number: WO 2005/058837

(56) References cited:
- EP-A- 1 331 010
- WO-A-96/05194
- WO-A-03/068751
- WO-A-03/095428
- WO-A-20/04056369
- STARK H: "Recent advances in histamine H3/H4 receptor ligands" EXPERT OPINION ON THERAPEUTIC PATENTS, ASHLEY PUBLICATIONS, GB, vol. 13, no. 6, 2003, pages 851-865, XP002298271 ISSN: 1354-3776

## Description

The present invention relates to novel benzazepine derivatives having pharmacological activity, processes for their preparation, to compositions containing them and to their use in the treatment of neurological and psychiatric disorders.

JP 2001226269 and WO 00/23437 (Takeda Chem Ind Ltd) describe a series of benzazepine derivatives which are claimed to be useful in the treatment of obesity. DE 2207430, US 4,210,749 and FR 2171879 (Pennwalt Corp) and GB 1268243 (Wallace and Tiernan Inc) all describe a series of benzazepine derivatives which are claimed as being antagonists for narcotics (such as morphine or codeine) and also anti-histamines and anticholinergic agents. WO 02/14513 (Takeda Chem Ind Ltd) describe a series of benzazepine derivatives with GPR12 activity which are claimed to be useful in the treatment of attention deficit disorder, narcolepsy or anxiety. WO 02/02530 (Takeda Chem Ind Ltd) describe a series of benzazepine derivatives as GPR14 antagonists which are claimed to be useful in the treatment of hypertension, atherosclerosis and cardiac infarction. WO 01/03680 (Isis Innovation Ltd) describe a series of benzazepine derivatives which are claimed as effective agents in the preparation of cells for transplantation in addition to the inhibition of diseases such as diabetes. WO 00/21951 (SmithKline Beecham plc) discloses a series of tetrahydrobenzazepine derivatives as modulators of dopamine D3 receptors which are claimed to be useful as antipsychotic agents. WO 01/87834 (Takeda Chem Ind Ltd) describe a series of benzazepine derivatives as MCH antagonists which are claimed to be useful in the treatment of obesity. WO 02/15934 (Takeda Chem Ind Ltd) describe a series of benzazepine derivatives as urotensin II receptor antagonists which are claimed to be useful in the treatment of neurodegenerative disorders.

WO9605194 discloses the compound 7-amino-3-cyclopropyl-2,3,4,5-tetrahydro-1H-3-benzazepine as a synthesis intermediate. Various histamine H3 receptor ligands are disclosed in H. Stark, "Recent advances in histamine H3/H4 receptor ligands", Expert Opinion on Therapeutic Patents, vol. 13 no.6, 2003 pages 851-865.

The histamine H3 receptor is predominantly expressed in the mammalian central nervous system (CNS), with minimal expression in peripheral tissues except on some sympathetic nerves (Leurs et al., (1998), Trends Pharmacol. Sci. 19, 177-183). Activation of H3 receptors by selective agonists or histamine results in the inhibition of neurotransmitter release from a variety of different nerve populations, including histaminergic and cholinergic neurons (Schlicker et al., (1994), Fundam. Clin. Pharmacol. 8, 128-137). Additionally, *in vitro* and *in vivo* studies have shown that H3 antagonists can facilitate neurotransmitter release in brain areas such as the cerebral cortex and hippocampus, relevant to cognition (Onodera et al., (1998), In: The Histamine H3 receptor, ed Leurs and Timmerman, pp255-267, Elsevier Science B.V.). Moreover, a number of reports in the literature have demonstrated the cognitive enhancing properties of H3 antagonists (e.g. thioperamide, clobenpropit, ciproxifan and GT-2331) in rodent models including the five choice task, object recognition, elevated plus maze, acquisition of novel task and passive avoidance (Giovanni et al., (1999), Behav. Brain Res. 104, 147-155). These data suggest that novel H3 antagonists and/or inverse agonists such as the current series could be useful for the treatment of cognitive impairments in neurological diseases such as Alzheimer's disease and related neurodegenerative disorders.

The present invention provides, in a first aspect, a compound of formula (I) or a pharmaceutically acceptable salt thereof: 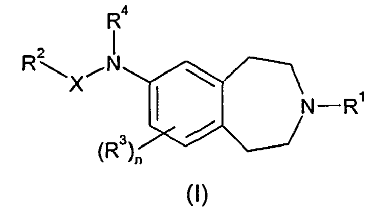 wherein:
R¹ represents -C₃₋₇ cycloalkyl optionally substituted by C₁₋₃ alkyl;
R² represents hydrogen, -C₁₋₆ alkyl, -C₃₋₈ cycloalkyl, -C₁₋₆ akyl-C₃₋₈ cycloalkyl; -aryl, -heterocyclyl, -heteroaryl, -C₃₋₈ cycloalkyl-Y-C₃₋₈ cycloalkyl, -C₃₋₈ cycloalkyl-Y-aryl, -C₃₋₈ cycloalkyl-Y-heteroaryl, -C₃₋₈ cycloalkyl-Y-heterocyclyl, -aryl-Y-C₃₋₈ cycloalkyl, -aryl-Y-aryl, -aryl-Y-heteroaryl, -aryl-Y-heterocyclyl, -heteroaryl-Y-C₃₋₈ cycloalkyl, -heteroaryl-Y-aryl, -heteroaryl-Y-heteroaryl, -heteroaryl-Y-heterocyclyl, -heterocyclyl- Y-C₃₋₈ cycloalkyl, -heterocyclyl-Y-aryl, -heterocyclyl-Y-heteroaryl, -heterocyclyl-Y-heterocyclyl;
X represents a bond, CO, SO₂, CONR⁵, COO or COC₂₋₆ alkenyl;
Y represents a bond, C₁₋₆ alkyl, CO, CONH, NHCO, O, SO₂, SO₂NH or NHSO₂;
R³ represents halogen, C₁₋₆alkyl, C₁₋₆ alkoxy, cyano, amino or trifluoromethyl;
R⁴ and R⁵ independently represent hydrogen, -C₁₋₆ alkyl, -C₃₋₈ cycloalkyl, -aryl, -heterocyclyl or -heteroaryl;
n is 0, 1 or 2;
wherein said alkyl, cycloalkyl, aryl, heteroaryl and heterocyclyl groups of R², R³ and R⁴ may be optionally substituted by one or more substituents (e.g. 1, 2 or 3) which may be the same or different, and which are selected from the group consisting of halogen, hydroxy, cyano, nitro, =O, haloC₁₋₆ alkyl, haloC₁₋₆ alkoxy, C₁₋₆ alkyl, C₁₋₆ alkoxy, arylC₁₋₆ alkoxy, C₁₋₆ alkylthio, C₁₋₆ alkoxyC₁₋₆ alkyl, C₃₋₇ cycloalkylC₁₋₆ alkoxy, C₁₋₆ alkanoyl, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, sulfonyl, arylsulfonyl, arylsulfonyloxy, arylsulfonylC₁₋₆ alkyl, aryloxy, C₁₋₆ alkylsulfonamido, C₁₋₆ alkylamino, C₁₋₆ alkylamido, -R⁸, -CO₂R⁸, -COR⁸, C₁₋₆ alkylsulfonamidoC₁₋₆ alkyl, C₁₋₆ alkylamidoC₁₋₆ alkyl, arylsulfonamido, arylcarboxamido, arylsulfonamidoC₁₋₆ alkyl, arylcarboxamidoC₁₋₆ alkyl, aryl, aroyl, aroylC₁₋₆ alkyl, arylC₁₋₆ alkanoyl, or a group -NR⁶R⁷, -C₁₋₆ alkyl-NR⁶R⁷, -C₃₋₈cycloalkyl-NR⁶R⁷, -CONR⁶R⁷, -NR⁶COR⁷, -NR⁶SO₂R⁷, -OCONR⁶R⁷, -NR⁶CO₂R⁷, -NR⁸CONR⁶R⁷ or -SO₂NR⁶R⁷(wherein R⁶, R⁷ and R⁸ independently represent hydrogen, C₁₋₆ alkyl, -C₃₋₈ cycloalkyl, -C₁₋₆ alkyl-C₃₋₈ cycloalkyl, aryl, heterocyclyl or heteroaryl or -NR⁶R⁷ may represent a nitrogen containing heterocyclyl group, wherein said R⁵, R⁶ and R⁷ groups may be optionally substituted by one or more substituents (e.g. 1, 2 or 3) which may be the same or different, and which are selected from the group consisting of halogen, hydroxy, C₁₋₆ alkyl, C₁₋₆ alkoxy, cyano, amino, =O or trifluoromethyl);
or solvates thereof, wherein the compound is not 7-amino-3-cyclopropyl-2,3,4,5-tetrahydro-1 H-3-benzazepine
Specific compounds which may be mentioned are those wherein
R² represents hydrogen, -C₁₋₆ alkyl, -C₃₋₈ cycloalkyl, -aryl, -heterocyclyl, -heteroaryl, -C₃₋₈ cycloalkyl-Y-C₃₋₈ cycloalkyl, -C₃₋₈ cycloalkyl-Y-aryl, -C₃₋₈ cycloalkyl-Y-heteroaryl, -C₃₋₈ cycloalkyl-Y-heterocyclyl, -aryl-Y-C₃₋₈ cycloalkyl, -aryl-Y-aryl, -aryl-Y-heteroaryl, -aryl-Y-heterocyclyl, -heteroaryl-Y-C₃₋₈ cycloalkyl, -heteroaryl-Y-aryl, -heteroaryl-Y-heteroaryl, - heteroaryl-Y-heterocyclyl, -heterocyclyl-Y-C₃₋₈ cycloalkyl, -heterocyclyl-Y-aryl, -heterocyclyl-Y-heteroaryl, -heterocyclyl-Y-heterocyclyl; and
X represents a bond, CO, SO₂ CONR⁵ or COO; and
wherein said alkyl, cycloalkyl, aryl, heteroaryl and heterocyclyl groups of R², R³ and R⁴ may be optionally substituted by one or more substituents (e.g. 1, 2 or 3) which may be the same or different, and which are selected from the group consisting of halogen, hydroxy, cyano, nitro, =O, trifluoromethyl, trifluoromethoxy, fluoromethoxy; difluoromethoxy, C₁₋₆ alkyl, pentafluoroethyl, C₁₋₆ alkoxy, arylC₁₋₆ alkoxy, C₁₋₆ alkylthio, C₁₋₆ alkoxyC₁₋₆ alkyl, C₃₋₇ cycloalkylC₁₋₆ alkoxy, C₁₋₆ alkanoyl, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, sulfonyl, arylsulfonyl, arylsulfonyloxy, arylsulfonylC₁₋₆ alkyl, aryloxy, C₁₋₆ alkylsulfonamido, C₁₋₆ alkylamino, C₁₋₆ alkylamido, -R⁸, -CO₂R⁸, -COR⁸, C₁₋₆ alkylsulfonamidoC₁₋₆ alkyl, C₁₋₆ alkylamidoC₁₋₆ alkyl, arylsulfonamido, arylcarboxamido, arylsulfonamidoC₁₋₆ alkyl, arylcarboxamidoC₁₋₆ alkyl, aroyl, aroylC₁₋₆ alkyl, arylC₁₋₆ alkanoyl, or a group -NR⁶R⁷, -C₁₋₆ alkyl-NR⁶R⁷, -C₃₋₈ cycloalkyl-NR⁶R⁷, -CONR⁶R⁷, -NR⁶COR⁷, -NR⁶SO₂R⁷, -OCONR⁶R⁷ , -NR⁶CO₂R⁷, -NR⁸CONR⁶R⁷ or -SO₂NR⁶R⁷ (wherein R⁶, R⁷ and R⁸ independently represent hydrogen, C₁₋₆ alkyl, -C₃₋₈cycloalkyl, -C₁₋₆ alkyl-C₃₋₈ cycloalkyl, aryl, heterocyclyl or heteroaryl or -NR⁶R⁷ may represent a nitrogen containing heterocyclyl group, wherein said R⁶, R⁷ and R⁸ groups may be optionally substituted by one or more substituents (e.g. 1, 2 or 3) which may be the same or different, and which are selected from the group consisting of halogen, hydroxy, C₁₋₆ alkyl, C₁₋₆ alkoxy, cyano, amino, =O or trifluoromethyl);
or solvates thereof.

The term 'C₁₋₆ alkyl' as used herein as a group or a part of the group refers to a linear or branched saturated hydrocarbon group containing from 1 to 6 carbon atoms. Examples of such groups include methyl, ethyl, n-propyl, isopropyl, n-butyl, isobutyl, sec-butyl, tert butyl, n-pentyl, isopentyl, neopentyl or hexyl and the like.

The term 'C₁₋₆ alkoxy' as used herein refers to an -O-C₁₋₆ alkyl group wherein C₁₋₆ alkyl is as defined herein. Examples of such groups include methoxy, ethoxy, propoxy, butoxy, pentoxy or hexoxy and the like.

The term 'C₃₋₈ cycloalkyl' as used herein refers to a saturated monocyclic hydrocarbon ring of 3 to 8 carbon atoms. Examples of such groups include cyclopropyl, cyclobutyl, cyclopentyl, cyclohexyl, cycloheptyl or cyclooctyl and the like.

The term 'halogen' as used herein refers to a fluorine, chlorine, bromine or iodine atom.

The term 'haloC₁₋₆ alkyl' as used herein refers to a C₁₋₆ alkyl group as defined herein wherein at least one hydrogen atom is replaced with halogen. Examples of such groups include fluoroethyl, trifluoromethyl or trifluoroethyl and the like.

The term 'haloC₁₋₆ alkoxy' as used herein refers to a C₁₋₆ alkoxy group as herein defined wherein at least one hydrogen atom is replaced with halogen. Examples of such groups include difluoromethoxy or trifluoromethoxy and the like.

The term 'aryl' as used herein refers to a C₆₋₁₂ monocyclic or bicyclic hydrocarbon ring wherein at least one ring is aromatic. Examples of such groups include phenyl, naphthyl or tetrahydronaphthalenyl and the like.

The term 'aryloxy' as used herein refers to an -O-aryl group wherein aryl is as defined herein. Examples of such groups include phenoxy and the like.

The term 'heteroaryl' as used herein refers to a 5-6 membered monocyclic aromatic or a fused 8-10 membered bicyclic aromatic ring containing 1 to 4 heteroatoms selected from oxygen, nitrogen and sulphur. Examples of such monocyclic aromatic rings include thienyl, furyl, furazanyl, pyrrolyl, triazolyl, tetrazolyl, imidazolyl, oxazolyl, thiazolyl, oxadiazolyl, isothiazolyl, isoxazolyl, thiadiazolyl, pyranyl, pyrazolyl, pyrimidyl, pyridazinyl, pyrazinyl, pyridyl, triazinyl, tetrazinyl and the like. Examples of such fused aromatic rings include quinolinyl, isoquinolinyl, quinazolinyl, quinoxalinyl, pteridinyl, cinnolinyl, phthalazinyl, naphthyridinyl, indolyl, isoindolyl, azaindolyl, indolizinyl, indazolyl, purinyl, pyrrolopyridinyl, furopyridinyl, benzofuranyl, isobenzofuranyl, benzothienyl, benzoimidazolyl, benzoxazolyl, benzoisoxazolyl, benzothiazolyl, benzoisothiazolyl, benzoxadiazolyl, benzothiadiazolyl and the like.

The term 'heterocyclyl' refers to a 4-7 membered monocyclic ring or a fused 8-12 membered bicyclic ring which may be saturated or partially unsaturated containing 1 to 4 heteroatoms selected from oxygen, nitrogen or sulphur. Examples of such monocyclic rings include pyrrolidinyl, azetidinyl, pyrazolidinyl, oxazolidinyl, piperidinyl, piperazinyl, morpholinyl, thiomorpholinyl, thiazolidinyl, hydantoinyl, valerolactamyl, oxiranyl, oxetanyl, dioxolanyl, dioxanyl, oxathiolanyl, oxathianyl, dithianyl, dihydrofuranyl, tetrahydrofuranyl, dihydropyranyl, tetrahydropyranyl, tetrahydropyridinyl, tetrahydropyrimidinyl, tetrahydrothiophenyl, tetrahydrothiopyranyl, diazepanyl, azepanyl and the like. Examples of such bicyclic rings include indolinyl, isoindolinyl, benzopyranyl, quinuclidinyl, 2,3,4,5-tetrahydro-1*H*-3-benzazepine, tetrahydroisoquinolinyl and the like.

Preferably, R¹ represents -C₃₋₇ cycloalkyl (e.g. cyclobutyl, cyclopentyl or cyclohexyl) optionally substituted by a C₁₋₃ alkyl (e.g. methyl) group.

Most preferably, R¹ represents cyclobutyl, cyclopentyl or cyclohexyl optionally substituted by one or more C₁₋₃ alkyl (e.g. methyl) groups, especially unsubstituted cyclobutyl.

Preferably, R² represents
- C₁₋₆ alkyl (e.g. methyl, isopropyl or -C(CH₂CH₃)₂);
- C₃₋₈ cycloalkyl (e.g. cyclopropyl or cyclohexyl);
- C₁₋₆ alkyl-C₃₋₈ cycloalkyl (e.g. -CH₂-cyclopropyl);
- aryl (e.g. phenyl, naphthyl or bicyclooctatriene) optionally substituted by one or more cyano, halogen (e.g. fluorine, chlorine or iodine), haloC₁₋₆alkyl (e.g. trifluoromethyl), haloC₁₋₆ alkoxy (e.g. trifluoromethoxy), C₁₋₆ alkyl (e.g. methyl), C₁₋₆ alkoxy (e.g. methoxy or ethoxy) or C₁-₈ alkylsulfonyl (e.g. -SO₂Me) groups;
- heteroaryl (e.g. pyridinyl, pyrazinyl, pyridazinyl, pyrimidinyl, pyrazolyl, triazolyl, benzotriazolyl, imidazolyl, benzimidazolyl, quinolinyl, isoquinolinyl, quinoxalinyl, naphthyridinyl, indolyl, indazolyl, tetrahydroindazolyl, pyrazolopyridinyl, pyrazolopyrimidinyl, dihydropyrrolopyridinyl, thiazolyl, benzothiazolyl, thiadiazolyl, benzothiadiazolyl, thiophenyl, benzothiophenyl, oxazolyl, isoxazolyl, benzisoxazolyl, oxadiazolyl, furanyl, benzofuranyl or chromenyl) optionally substituted by one or more -CONR⁶R⁷ (e.g. -CONMe), hydroxy, cyano, oxo, halogen (e.g. bromine or fluorine), haloC₁₋₆ alkyl (e.g. trifluoromethyl or trifluoroethyl), C₁₋₆ alkyl (e.g. methyl, ethyl, propyl or isopropyl or t-butyl) or C₁₋₆ alkoxy (e.g. methoxy) groups;
- heterocyclyl (e.g. pyrrolidinyl, piperidinyl, morpholinyl, tetrahydropyranyl, tetrahydroisoquinolinyl, tetrahydroquinolinyl, dihydroisoindolyl, dihydroindolyl, dihydrobenzofuranyl, dihydrochromenyl, dihydrobenzoxazinyl, dihydrobenzodioxinyl, benzodioxolyl or tetrahydrobenzazepinyl) optionally substituted by one or more cyano or halogen (e.g. fluorine or chlorine) groups;
- aryl-Y-aryl (e.g. -phenyl-phenyl or -phenyl-O-phenyl);
- aryl-Y-heteroaryl (e.g. -phenyl-pyridinyl, -phenyl-oxazolyl, -phenyl-imidazopyridinyl, -phenyl-pyrimidinyl, -phenyl-tetrazolyl, -phenyl-triazinyl, -phenyl-pyrazinyl, -phenyl-imidazolyl, -phenyl-pyrazolyl or -phenyl-triazolyl) optionally substituted by one or more cyano or C₁₋₆ alkyl (e.g. methyl) groups;
- aryl-Y-heterocyclyl (e.g. -phenyl-pyrrolidinyl, -phenyl-imidazolidinyl or -phenyl-dioxidoisothiazolidinyl) optionally substituted by one or more oxo groups;
- heteroaryl-Y-aryl (e.g. -furanyl-phenyl, -triazolyl-phenyl, -oxazolyl-phenyl, -pyridinyl-phenyl, -pyrazolyl-phenyl, -thiazolyl-phenyl, -pyrazinyl-phenyl, -pyrazolyl-CH₂-phenyl, -pyrazinyl-O-phenyl or -triazolyl-O-phenyl) optionally substituted by one or more cyano, halogen (e.g. fluorine or chlorine), oxo, haloC₁₋₆ alkyl (e.g. trifluoromethyl), C₁₋₆ alkyl (e.g. methyl, ethyl or propyl), C₁₋₆ alkoxy (e.g. methoxy) or -R⁸ (e.g. phenyl) groups;
- heteroaryl-Y-heteroaryl (e.g. -pyridinyl-pyrazolyl, -pyrazolyl-pyridinyl, -thiazolyl-benzoxadiazolyl, -pyridinyl-pyridinyl, -pyridinyl-imidazolyl, -pyridinyl-pyrazinyl, -pyridinyl-triazolyl, -pyridinyl-pyrimidinyl, -pyrazolyl-thienyl, -pyrazolyl-furanyl or -pyrazolyl-pyrrolyl) optionally substituted by one or more cyano, C₁₋₆ alkyl (e.g. ethyl) or -R⁸ (e.g. phenyl) groups;
- heteroaryl-Y-heterocyclyl (e.g. -pyridinyl-pyrrolidinyl, -pyridinyl-morpholinyl, -pyrazinyl-pyrrolidinyl, -pyrazinyl-piperidinyl or -pyrazinyl-O-tetrahydropyranyl) optionally substituted by one or more oxo groups;
- heterocyclyl-Y-aryl (e.g. -pyrrolidinyl-phenyl, -piperidinyl-phenyl, -piperazinyl-phenyl or-piperidinyl-O-phenyl) optionally substituted by one or more cyano, halogen (e.g. fluorine) or C₁₋₆ alkoxy (e.g. methoxy) groups; or
- heterocyclyl-Y-heteroaryl (e.g. -piperidinyl-pyridinyl, -piperidinyl-O-pyridinyl, -piperazinyl-pyrazinyl or -piperazinyl-pyridinyl) optionally substituted by one or more cyano groups.

More preferably, R² represents
- aryl (e.g. phenyl) optionally substituted by a cyano group;
- heteroaryl (e.g. pyridinyl) optionally substituted by a C₁₋₆ alkyl (e.g. methyl) group;
- heterocyclyl (e.g. tetrahydroquinolinyl);
- aryl-Y-heteroaryl (e.g. -phenyl-pyridinyl, -phenyl-pyrimidinyl or -phenyl-tetrazolyl) optionally substituted by a cyano group;
- aryl-Y-heterocyclyl (e.g. -phenyl-imidazolidinyl) optionally substituted by a oxo group;
- heteroaryl-Y-aryl (e.g. -pyridinyl-phenyl) optionally substituted by a cyano group; or
- heteroaryl-Y-heterocyclyl (e.g. -pyridinyl-morpholinyl or -pyrazinyl-piperidinyl).

Particularly preferably, R² represents -aryl-Y-heteroaryl (e.g. -phenyl-pyridinyl, -phenyl-pyrimidinyl or-phenyl-tetrazolyl) optionally substituted by a cyano group, most preferably -phenyl-pyridinyl substituted by a cyano group.

Preferably, X represents a bond, CO, SO₂, CONR⁵ (e.g. CONH) or COC₂₋₆ alkenyl (e.g. COCH=CH), more preferably CO.

Preferably, Y represents a bond, C₁₋₆ alkyl (e.g. CH₂) or O, more preferably a bond.

Preferably, R⁴ represents hydrogen.

Preferably, R⁵ represents hydrogen.

Preferably, R⁶ and R⁷ independently represent hydrogen and C₁₋₆ alkyl (e.g. methyl), more preferably one of R⁶ and R⁷ represents hydrogen and the other represents methyl.

Preferably, R⁸ represents aryl (e.g. phenyl).

Preferably, n represents 0 or 1, more preferably O.

When n represents 1, R³ is preferably a halogen (e.g. iodine) atom or a cyano group.

Preferred compounds according to the invention include examples E1-E280 as shown below, or a pharmaceutically acceptable salt thereof.

Compounds of formula (I) may form acid addition salts with acids, such as conventional pharmaceutically acceptable acids, for example maleic, hydrochloric, hydrobromic, phosphoric, acetic, fumaric, salicylic, sulphate, citric, lactic, mandelic, tartaric and methanesulphonic. Salts, solvates and hydrates of compounds of formula (I) therefore form an aspect of the invention.

Certain compounds of formula (I) are capable of existing in stereoisomeric forms. It will be understood that the invention encompasses all geometric and optical isomers of these compounds and the mixtures thereof including racemates. Tautomers also form an aspect of the invention.

The present invention also provides a process for the preparation of a compound of formula (I) or a pharmaceutically acceptable salt thereof, which process comprises:
(a) preparing a compound of formula (I) wherein X represents CO which comprises reacting a compound of formula (II) 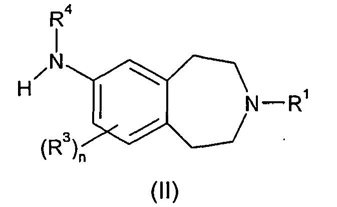
   wherein R¹, R³, R⁴ and n are as defined above, with a compound of formula R^{2'}-CO₂H,
   wherein R^{2'} is as defined above for R² or a group convertible thereto; or
(b) preparing a compound of formula (I) wherein X represents CO which comprises reacting a compound of formula (II) as defined above, with an activated compound of formula R^{2'}-COL¹ wherein R^{2'} is as defined above for R² or a group convertible thereto and L¹ represents a suitable leaving group such as a halogen atom (e.g. chlorine, bromine) or imidazole; or
(c) preparing a compound of formula (I) wherein X represents CONR⁵ which comprises reacting a compound of formula (II) as defined above, with an activated compound of formula R^{2'}-NR^{5'}COL² wherein R^{2'} and R^{5'} are as defined above for R² and R⁵, respectively or a group convertible thereto and L² represents a suitable leaving group such as a halogen atom (e.g. chlorine, bromine); or
(d) preparing a compound of formula (I) wherein X represents SO₂ which comprises reacting a compound of formula (II) as defined above, with an activated compound of formula R^{2'}-SO₂L³ wherein R^{2'} is as defined above for R² or a group convertible thereto and L³ represents a suitable leaving group such as a halogen atom (e.g. chlorine, bromine); or
(e) preparing a compound of formula (I) wherein X represents CONR⁵ which comprises reacting a compound of formula (II) as defined above, with a compound of formula R^{2'}-N=C=O wherein R^{2'} is as defined above for R² or a group convertible thereto; or
(f) preparing a compound of formula (I) wherein X represents COO which comprises reacting a compound of formula (II) as defined above, with an activated compound of formula R^{2'}-OCOL⁴ wherein R^{2'} is as defined above for R² or a group convertible thereto and L⁴ represents a suitable leaving group such as a halogen atom (e.g. chlorine, bromine); or
(g) preparing a compound of formula (I) wherein X represents a bond which comprises reacting a compound of formula (II) as defined above, with a compound of formula R^{2'}-L⁵, wherein R^{2'} is as defined above for R² or a group convertible thereto and L⁵ represents a suitable leaving group such as a halogen atom (e.g. chlorine, bromine or iodine);
(h) reacting a compound of formula (III) 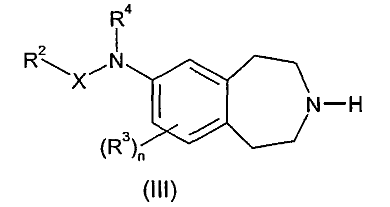
   wherein R², R³, R⁴, X and n are as defined above, with a compound of formula R^{1'}-L⁶,
   wherein R^{1'} is as defined above for R¹ or a group convertible thereto and L⁶ represents a suitable leaving group such as a halogen atom (e.g. bromine, iodine or tosylate); or
(i) reacting a compound of formula (III) as defined above, with a ketone of formula R^{1'}=O, wherein R^{1'} is as defined above for R¹ or a group convertible thereto; or
(j) deprotecting a compound of formula (I) which is protected; and
(k) interconversion to other compounds of formula (I).

Process (a) typically comprises the use of a coupling reagent, such as dicyclohexylcarbodiimide, in an appropriate solvent such as dichloromethane or dimethylformamide, optionally in the presence of a suitable activating agent, such as hydroxybenzotriazole at an appropriate temperature such as room temperature.

Processes (b), (c), (d) and (f) typically comprise the use of a base, such as triethylamine, in an appropriate solvent such as dichloromethane, at an appropriate temperature, for example room temperature.

Process (e) may typically be performed in a suitable solvent, such as dichloromethane, at an appropriate temperature, for example room temperature.

When the leaving group L⁵ is attached to an sp³ hybridised carbon, for example, R^{2'}-L⁵ is an alkyl halide, process (g) typically comprises the use of a suitable base, such as potassium hydroxide in an appropriate solvent such as methanol optionally in the presence of a catalyst such as potassium iodide at an appropriate temperature such as reflux.

When the leaving group L⁵ is attached to an sp² hybridised carbon, for example, R^{2'}-L⁵ is an aryl or heteroaryl halide, process (g) typically comprises the use of a transition metal catalyst, such as a palladium salt (e.g. Palladium (II) acetate), in combination with a suitable ligand, such a BINAP, in the presence of a base such as potassium carbonate, in an appropriate solvent such as toluene, at an appropriate temperature such as reflux.

Process (h) typically comprises the use of a suitable base, such as potassium carbonate in an appropriate solvent such as 2-butanone optionally in the presence of a catalyst such as potassium iodide at an appropriate temperature such as reflux.

Process (i) typically comprises the use of reductive conditions (such as treatment with a borohydride e.g. sodium triacetoxyborohydride), optionally in the presence of an acid, such as acetic acid, in an appropriate solvent such as dichloromethane at a suitable temperature such as room temperature.

In process (j), examples of protecting groups and the means for their removal can be found in T. W. Greene 'Protective Groups in Organic Synthesis' (J. Wiley and Sons, 1991). Suitable amine protecting groups include sulphonyl (e.g. tosyl), acyl (e.g. acetyl, 2',2',2'-trichloroethoxycarbonyl, benzyloxycarbonyl or t-butoxycarbonyl) and arylalkyl (e.g. benzyl), which may be removed by hydrolysis (e.g. using an acid such as hydrochloric acid in dioxan or trifluoroacetic acid in dichloromethane) or reductively (e.g. hydrogenolysis of a benzyl group or reductive removal of a 2',2',2'-trichloroethoxycarbonyl group using zinc in acetic acid) as appropriate. Other suitable amine protecting groups include trifluoroacetyl (-COCF₃) which may be removed by base catalysed hydrolysis or a solid phase resin bound benzyl group, such as a Merrifield resin bound 2,6-dimethoxybenzyl group (Ellman linker), which may be removed by acid catalysed hydrolysis, for example with trifluoroacetic acid.

Process (k) may be performed using conventional interconversion procedures such as epimerisation, oxidation, reduction, alkylation, nucleophilic or electrophilic aromatic substitution, ester hydrolysis, amide bond formation or transition metal mediated coupling reactions. Examples of transition metal mediated coupling reactions useful as interconversion procedures include the following: Palladium catalysed coupling reactions between organic electrophiles, such as aryl halides, and organometallic reagents, for example boronic acids (Suzuki cross-coupling reactions); Palladium catalysed amination and amidation reactions between organic electrophiles, such as aryl halides, and nucleophiles, such as amines and amides; Copper catalysed amidation reactions between organic electrophiles (such as aryl halides) and nucleophiles such as amides; and Copper mediated coupling reactions between phenols and boronic acids.
may be performed using conventional interconversion procedures such as epimerisation, oxidation, reduction, alkylation, nucleophilic or electrophilic aromatic substitution, ester hydrolysis, amide bond formation or transition metal mediated coupling reactions. Examples of transition metal mediated coupling reactions useful as interconversion procedures include the following: Palladium catalysed coupling reactions between organic electrophiles, such as aryl halides, and organometallic reagents, for example boronic acids (Suzuki cross-coupling reactions); Palladium catalysed amination and amidation reactions between organic electrophiles, such as aryl halides, and nucleophiles, such as amines and amides; Copper catalysed amidation reactions between organic electrophiles (such as aryl halides) and nucleophiles such as amides; and Copper mediated coupling reactions between phenols and boronic acids.

Compounds of formula (II) wherein R⁴ represents hydrogen may be prepared in accordance with the following scheme 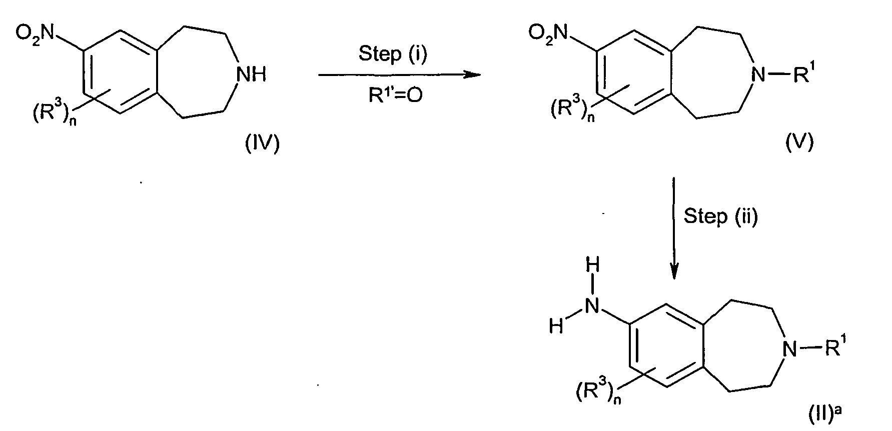 wherein R¹, R^{1'}, R³ and n are as defined above.

Step (i) can be performed under reducing conditions in an analogous manner to that described for process (i) above.

Step (ii) comprises a reduction reaction, for example reaction of a compound of formula (V) with hydrogen in the presence of a catalyst, for example palladium in methanol.

Compounds of formula (II) wherein R⁴ represents -C₁₋₆ alkyl, -C₃₋₈ cycloalkyl, -aryl, -heterocyclyl or -heteroaryl may be prepared in accordance with the following scheme: 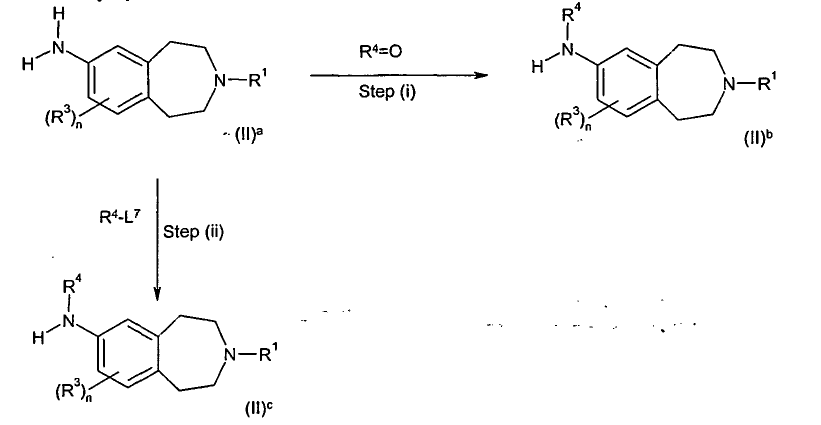 wherein R¹, R³ and n are as defined above and L⁷ represents a suitable leaving group such as a halogen atom (e.g. bromine or iodine).

Step (i) may be performed under reducing conditions in an analogous manner to that described for process (i) above.

When the leaving group L⁷ is attached to an sp³ hybridised carbon, for example, R⁴-L⁷ is an alkyl halide, step (ii) typically comprises the use of a suitable base, such as potassium hydroxide in an appropriate solvent such as methanol at an appropriate temperature such as reflux.

When the leaving group L⁷ is attached to an sp² hybridised carbon, for example, R⁴-L⁷ is an aryl or heteroaryl halide, step (ii) typically comprises the use of a transition metal catalyst, such as a palladium salt (e.g. Palladium (II) acetate), in combination with a suitable ligand, such a BINAP, in the presence of a base such as potassium carbonate, in an appropriate solvent such as toluene, at an appropriate temperature such as reflux.

Compounds of formula (III) wherein R⁴ represents hydrogen may be prepared in accordance with the following scheme: 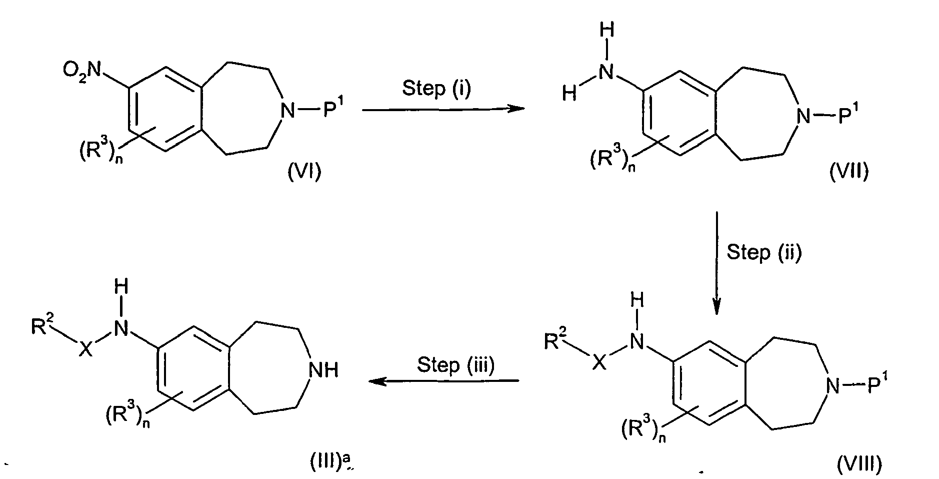 wherein R², R³, X and n are as defined above and P¹ represents a suitable protecting group such as Boc.

Step (i) comprises a reduction reaction, for example reaction of a compound of formula (VI) with hydrogen in the presence of a catalyst, for example palladium in methanol.

Step (ii) can be performed as highlighted in processes (a - g).

Step (iii) comprises a deprotection reaction and may be performed according to the process (j).

Compounds of formula (IV) may be prepared in accordance with procedures shown in WO 03/68752.

Compounds of formula (VI) may be prepared in accordance with procedures shown in WO 03/68751.

Compounds of formula (I) and their pharmaceutically acceptable salts have affinity for and are antagonists and/or inverse agonists of the histamine H3 receptor and are believed to be of potential use in the treatment of neurological diseases including Alzheimer's disease, dementia (including Lewy body dementia and vascular dementia), age-related memory dysfunction, mild cognitive impairment, cognitive deficit, epilepsy, neuropathic pain, inflammatory pain, migraine, Parkinson's disease, multiple sclerosis, stroke and sleep disorders (including narcolepsy and sleep deficits associated with Parkinson's disease); psychiatric disorders including schizophrenia (particularly cognitive deficit of schizophrenia), attention deficit hypereactivity disorder, depression, anxiety and addiction; and other diseases including obesity and gastro-intestinal disorders.

It will be appreciated that certain compounds of formula (I) believed to be of potential use in the treatment of Alzheimer's disease and cognitive deficit of schizophrenia will advantageously be CNS penetrant, e.g. have the potential to cross the blood-brain barrier.

It will also be appreciated that compounds of formula (I) may have the advantage of being selective for the histamine H3 receptor over other histamine receptor subtypes, such as the histamine H1 receptor. Generally, compounds of the invention may have the advantage of being at least 10 fold selective for H3 over H1, such as at least 100 fold selective.

Thus the invention also provides a compound of formula (I) or a pharmaceutically acceptable salt thereof, for use as a therapeutic substance in the treatment or prophylaxis of the above disorders, in particular cognitive impairments in diseases such as Alzheimer's disease and related neurodegenerative disorders.

In another aspect, the invention provides the use of a compound of formula (I) or a pharmaceutically acceptable salt thereof in the manufacture of a medicament for use in the treatment of the above disorders.

When used in therapy, the compounds of formula (I) are usually formulated in a standard pharmaceutical composition. Such compositions can be prepared using standard procedures.

Thus, the present invention further provides a pharmaceutical composition for use in the treatment of the above disorders which comprises the compound of formula (I) or a pharmaceutically acceptable salt thereof and a pharmaceutically acceptable carrier.

The present invention further provides a pharmaceutical composition which comprises the compound of formula (I) or a pharmaceutically acceptable salt thereof and a pharmaceutically acceptable carrier.

Compounds of formula (I) may be used in combination with other therapeutic agents, for example medicaments claimed to be useful as either disease modifying or symptomatic treatments of Alzheimer's disease. Suitable examples of such other therapeutic agents may be agents known to modify cholinergic transmission such as 5-HT₆ antagonists, M1 muscarinic agonists, M2 muscarinic antagonists or acetylcholinesterase inhibitors. When the compounds are used in combination with other therapeutic agents, the compounds may be administered either sequentially or simultaneously by any convenient route.

The invention thus provides, in a further aspect, a combination comprising a compound of formula (I) or a pharmaceutically acceptable derivative thereof together with a further therapeutic agent or agents.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations.

When a compound of formula (I) or a pharmaceutically acceptable derivative thereof is used in combination with a second therapeutic agent active against the same disease state the dose of each compound may differ from that when the compound is used alone. Appropriate doses will be readily appreciated by those skilled in the art.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusible solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colorants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1 % to 99% by weight, preferably from 10 to 60% by weight, of the active material, depending on the method of administration. The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 1.0 to 200 mg, and such unit doses may be administered more than once a day, for example two or three a day. Such therapy may extend for a number of weeks or months.

The following Descriptions and Examples illustrate the preparation of compounds of the invention.

### Description 1

### 3-Cyclobutyl-7-nitro-2,3,4,5-tetrahydro-1H-3-benzazepine (D1)

Sodium triacetoxyborohydride (320 mg, 1.5 mmol) was added to a stirred solution of 7-nitro-2,3,4,5-tetrahydro-1*H*-3-benzazepine (EP285323) (192 mg, 1 mmol), cyclobutanone (105 mg, 1.5 mmol) in dichloromethane (10 ml). After 1 hour at room temperature the reaction mixture was diluted with dichloromethane (100 ml) and washed with a saturated solution of sodium bicarbonate, water and brine. The organic layer was dried (sodium sulfate), filtered and concentrated *in vacuo* to afford the title compound (D1). MS (ES+) m/e 247 [M+H]⁺.

### Description 2

### 3-Cyclobutyl-2,3,4,5-tetrahydro-1H-3-benzazepin-7-amine (D2)

3-Cyclobutyl-7-nitro-2,3,4,5-tetrahydro-1*H*-3-benzazepine (D1) (2 g) was dissolved in methanol (2 ml) and tetrahydrofuran (30 ml). Palladium (200 mg, 10% on charcoal paste) was added and the reaction mixture was stirred at room temperature under hydrogen (1 atmosphere) for 5 hours. The reaction mixture was filtered through celite and the filtrate concentrated *in vacuo* to afford the title compound (D2). MS (ES+) m/e 217 [M+H]⁺.

### Description 3

### 1,1-Dimethylethyl 7-[(4-morpholinylcarbonyl)amino]-1,2,4,5-tetrahydro-3H-3-benzazepine-3-carboxylate (D3)

1,1-Dimethylethyl 7-amino-1,2,4,5-tetrahydro-3*H*-3-benzazepine-3-carboxylate (PCT Int. Appl. (1998), WO 98/30560) (393 mg, 1.5 mmol) was stirred in dichloromethane (5ml) with diethylaminomethyl polystyrene (3.2 mmol/g, 1.00 g, 3.2 mmol). Morpholine carbamoyl chloride (0.27 ml, 1.8 mmol) was added and the mixture stirred at room temperature for 16 hours. The resin was filtered, washed with dichloromethane and the filtrate concentrated *in vacuo.* The residue was purified by column chromatography (dichloromethane to 1:9:90 .880 ammonia: ethanol: dichloromethane) to afford the title compound (D3). MS (ES+) m/e 374 [M-H]⁻.

### Description 4

### N-(2,3,4,5-Tetrahydro-1H-3-benzazepin-7-yl)-4-morpholinecarboxamide (D4)

1,1-Dimethylethyl-7-[(4-morpholinylcarbonyl)amino]-1,2,4,5-tetrahydro-3*H*-3-benzazepine-3-carboxylate (D3) (562 mg, 1.5 mmol) was dissolved in dichloromethane (10 ml) and treated with trifluoroacetic acid (5 ml). The solution was stirred at room temperature for 1 hour, concentrated *in vacuo* and then twice co-evaporated with dichloromethane. The residue was dissolved in methanol and applied to a SCX (Varian bond-elute, 10g) and washed with methanol and then a mixture of .880 ammonia/ methanol. The combined basic fractions were concentrated *in vacuo* and the residue purified by column chromatography (1:9:40 .880 ammonia: ethanol: dichloromethane) to afford the title compound (D4). MS (ES+) m/e 254 [M+H]⁺.

### Description 5

### 3-(5-Pyrimidinyl)benzoic acid (D5)

A mixture of 3-carboxyphenylboronic acid (200 mg, 1.20 mmol), 5-bromopyrimidine (211 mg, 1.33 mmol), (1,1'bis-(diphenylphosphino)-ferrocene) palladium dichloride (88 mg, 0.12 mmol) and 2M sodium carbonate solution (1.2 ml) in 1,4-dioxane (10 ml) was heated at 80 °C for 1.5 hours. The mixture was allowed to cool to room temperature and left to stand for 18 hours. The mixture was poured into 0.5M hydrochloric acid (20 ml). This was extracted with ethyl acetate (x 3). The ethyl acetate layers were combined, washed with brine, dried under magnesium sulphate and evaporated *in vacuo* to afford the title compound (D5) MS (ES+), m/e 201 [M+H]⁺.

### Description 6

### 3-(2-Pyrazinyl)benzoic acid (D6)

A mixture of 3-carboxyphenylboronic acid (500 mg, 3.01 mmol), chloropyrazine (0.27 ml, 3.01 mmol), tetrakis(triphenylphosphine)palladium(0) (0.17 g, 0.15 mmol) and 0.5M sodium carbonate solution (15 ml) in acetonitrile (15 ml) was heated at 90 °C for 4 hours. The mixture was allowed to cool to room temperature and left to stand for 18 hours. The mixture was poured into 2M hydrochloric acid (25 ml). This was extracted with ethyl acetate (x 3). The ethyl acetate layers were combined, washed with brine, dried under magnesium sulphate and evaporated *in vacuo* to afford the title compound (D6) MS (ES+), m/e 201 [M+H]⁺.

### Descriptions 7 and 8

Descriptions 7 and 8 (D7 and D8) were synthesised in the same manner as Description 6 (D6) using either 3-carboxyphenylboronic acid or 4-carboxyphenylboronic acid and substituting chloropyrazine with 4-chloropyrmidine *(Biorganic Chem,* 2002, 30, 3, 188) as shown in the table:

| **Description** | **Boronic Acid** | **Mass Spectrum** |
|---|---|---|
| 3-(4-Pyrimidinyl)benzoic acid (D7) | 3-Carboxyphenylboronic acid | MS (ES+), m/e 201 (M+H)⁺. |
| 4-(4-Pyrimidinyl)benzoic acid (D8) | 4-Carboxyphenylboronic acid | MS (ES+), m/e 201 [M+H]⁺. |

### Description 9

### N-(3-Cyclobutyl-2,3,4,5-tetrahydro-1H-3-benzazepin-7-yl)-3-iodobenzamide (D9)

The title compound (D9) was synthesised in the same manner as Example 8 (E8) using dimethylformamide as solvent and substituting 4-(2-pyridinyl) benzoic acid with 3-iodobenzoic acid MS (ES+), m/e 447 [M+H]⁺.

### Description 10

### 5-{[(3-Cyclobutyl-2,3,4,5-tetrahydro-1H-3-benzazepin-7-yl)amino]carbonyl}-2-pyrazinecarboxylic acid (D10)

The title compound (D10) was synthesised in the same manner as Example 8 (E8) using dimethylformamide as solvent and substituting 4-(2-pyridinyl) benzoic acid with 2,5-pyrazinedicarboxylic acid MS (ES+), m/e 367 [M+H]⁺.

### Description 11

### 6-Chloro-N-(3-cyclobutyl-2,3,4,5-tetrahydro-1H-3-benzazepin-7-yl)-3-pyridinecarboxamide (D11)

A mixture of 3-cyclobutyl-2,3,4,5-tetrahydro-1*H*-3-benzazepin-7-amine (D2, 100 mg, 0.46mmol), 6-chloro-3-pyridinecarbonyl chloride (86 mg, 0.48 mmol) and polystyrene supported morpholine (125 mg of 3 mmol/g resin) in tetrahydrofuran (3 ml) was stirred at room temperature for 18 hours. The mixture was diluted with methanol and the resin removed by filtration. The filtrate was concentrated *in vacuo* to afford the title compound (D11) MS (ES+), m/e 356 & 358 [M+H]⁺

### Description 12

### 5-Bromo-N-(3-cyclobutyl-2,3,4,5-tetrahydro-1H-3-benzazepin-7-yl)-2-pyridinecarboxamide (D12)

A mixture of 5-bromo-2-pyridinecarboxylic acid (140 mg, 0.69 mmol), N-cyclohexylcarbodiimide N'-methyl polystyrene (544 mg, 0.92 mmol), and 1-hydroxybenzotriazole (124 mg, 0.92 mmol) in dry dimethylformamide (5 ml) were stirred under argon at room temperature for 10 mins. A solution of 3-cyclobutyl-2,3,4,5-tetrahydro-1*H*-3-benzazepin-7-amine (D2, 100 mg, 0.46 mmol) in dry dimethylformamide (1 ml) was added, and the reaction mixture left to stir at room temperature for 16h. The mixture was applied to a SCX ion exchange cartridge (Varian bond-elute, 10g), washed with methanol and then with a mixture of .880 ammonia: methanol (1: 9). The combined basic fractions were concentrated *in vacuo* and the resulting residue purified by column chromatography eluting with a mixture of 10% .880 ammonia in methanol: dichloromethane (0 to 5%) to afford the title product (D12); MS (ES+) m/e 400, 402 [M+H]⁺.

### Description 13

### 5-Chloro-N-(3-cyclobutyl-2,3,4,5-tetrahydro-1H-3-benzazepin-7-yl)-2-pyrazinecarboxamide (D13)

A mixture of *N*-(3-cyclobutyl-2,3,4,5-tetrahydro-1*H*-3-benzazepin-7-yl)-5-hydroxy-2-pyrazinecarboxamide (E9) (1.41 g, 4.2 mmol) and phosphorus oxychloride (6 ml) was heated at reflux for 1 hour. The mixture was allowed to cool and was poured onto ice. The resulting solution was basified using sodium carbonate and extracted with dichloromethane. The extracts were dried (sodium sulphate)and concentrated *in vacuo* to afford the title compound (D13) MS (ES+), m/e 357 & 359 [M+H]⁺

### Description 14

### 1,1-Dimethylethyl-7-[(4-pyridinylcarbonyl)amino]-1,2,4,5-tetrahydro-3H-3-benzazepine-3-carboxylate (D14)

A solution of 7-amino-1,2,4,5-tetrahydro-3-benzazepine-3-carboxylic acid tert-butyl ester (EP 284384) (0.50g, 1.9mmole) in dichloromethane (20ml) was treated with diethylaminomethyl-polystyrene (3.2mmole/g) (1.78g, 5.7mmole) followed by 4-pyridinecarbonyl chloride hydrochloride (0.41g, 2.3mmole) and the mixture was stirred at ambient temperature for 4 hours. The reaction mixture was filtered and the residue washed with dichloromethane. The filtrate was evaporated *in vacuo* and the residue purified by column chromatography eluting with a mixture of dichloromethane/ methanol (19:1) to afford the title compound (D14); MS (ES+), m/e 368 [M +H]⁺

### Description 15

### N-(2,3,4,5-Tetrahydro-1H-3-benzazepin-7-yl)-4-pyridinecarboxamide (D15)

1,1-Dimethylethyl-7-[(4-pyridinylcarbonyl)amino]-1,2,4,5-tetrahydro-3H-3-benzazepine-3-carboxylate (D14) (0.49g, 1.3mmole) in dichloromethane (10ml) was treated with trifluoroacetic acid (5ml). The solvent was removed *in vacuo* and the residue co evaporated with dichloromethane. The residue was dissolved in methanol and passed down an SCX column eluting with methanol, followed by 2M ammonia in methanol. The basic fractions were combined and concentrated *in vacuo* to afford the title compound (D15); MS (ES+), m/e 268 [M +H]⁺

### Description 16

### Ethyl 4-(1,3-oxazol-4-yl)benzoate (D16)

A mixture of ethyl 4-(bromoacetyl) benzoate (1.41g, 5.5 mmol) and ammonium formate (0.92g; 15.6 mmol) in formic acid 5 ml) was heated at reflux for 3 hours. The mixture was allowed to cool and poured into water. This mixture was basified using concentrated sodium hydroxide solution and extracted with ethyl acetate. The extracts were dried (sodium sulphate) and concentrated *in vacuo.* The residue was purified by column chromatography eluting with a mixture of pentane and ethyl acetate (4-1) to afford the title compound (D16). NMR (CDCl3) δ 8.09 (2H, m), 8.05 (H, s), 7.97 (H, s), 7.82 (2H, m), 4.39 (2H, q), 1.41 (3H, t).

### Description 17

### 4-(1,3-Oxazol-4-yl)benzoic acid (D17)

Ethyl 4-(1,3-oxazol-4-yl)benzoate (D16) (210 mg, 0.97 mmol) and sodium hydroxide (170 mg, 5 mmol) in a mixture of ethanol (6 ml) and water (1 ml) was heated at reflux for 30 minutes. The mixture was allowed to cool and concentrated *in vacuo.* The residue was dissolved in water and acidified with 2M hydrochloric acid and the mixture extracted with ethyl acetate. The extracts were dried (sodium sulphate) and concentrated *in vacuo* to afford the title compound (D17), MS (ES+), m/e 190 [M+H]⁺.

### Description 18

### 1-(6-Chloro-3-pyridinyl)-2-pyrrolidinone (D18)

A mixture of 2-chloro-5-iodopyridine (1 g, 4.2 mmol), potassium carbonate (2.1 g, 15 mmol), copper (1) iodide (80 mg, 0.42 mmol), 2-pyrrolidinone (338 mg, 4 mmol) and *N,N'-*dimethyl-1,2-ethanediamine (37 mg, 0.42 mmol) in 1,4-dioxan (20 ml) was heated at reflux for 18 hours. The mixture was filtered through Celite and the filtrate evaporated. The residue was purified by column chromatography eluting with 1-1 pentane - ethyl acetate to afford the title compound (D18) MS (ES+) m/e 197, 199 [M+H]⁺.

### Description 19

### 4-(6-Cyano-3-pyridinyl)benzoic acid (D19)

A mixture of 5-bromo-2-pyridinecarbonitrile (162 mg, 0.9 mmol), 4-(4,4,5,5-tetramethyl-1,3,2-dioxaborolan-2-yl)benzoic acid (200 mg, 0.8 mmol) and diphenylphosphino dichloro palladium (30 mg, 0.04 mmol) in 1,4-dioxan (5 ml) and 1 M sodium carbonate solution 2 ml) was heated at reflux for 18 hours. The mixture was poured into 1M sodium carbonate solution and washed with ethyl acetate. The aqueous portion was acidified with 2M hydrochloric acid and extracted with ethyl acetate. The extracts were dried with sodium sulphate and evaporated to give the title compound (D19), MS (ES-) m/e 223 [M-H].

### Description 20

### 1,1-Dimethylethyl 7-{[(5-methyl-1-phenyl-1H-pyrazol-4-yl)carbonyl]amino}-1,2,4,5-tetrahydro-3H-3-benzazepine-3-carboxylate (D20)

A mixture of 1,1-dimethylethyl 7-amino-1,2,4,5-tetrahydro-3*H*-3-benzazepine-3-carboxylate [EP284384] (1.0g, 3.82mmol), 5-methyl-1-phenyl-1*H* pyrazole-4-carboxylic acid (1.2g, 5.72mmol), triethylamine (0.80ml, 5.72mmol), O-(1*H*-benzotriazol-1-yl)-N,N,N',N'-tetramethyluronium hexafluorophosphate (2.17g, 5.72mmol) and hydroxybenzotriazole hydrate (0.29g, 1.91 mmol) in dimethylformamide (30ml) was stirred at room temperature for 18 hours. The reaction mixture was concentrated *in vacuo* and diluted with dichloromethane (50ml), and the resulting organic phase washed with a saturated solution of sodium bicarbonate. The organic phase was dried (magnesium sulphate) and concentrated *in vacuo.* The residue was purified by silica gel chromatography, eluting with ethyl acetate:hexane (3:7) to afford the title compound (D20) (1.65g, 97%).
MS (ES+) m/e 447 [M+H]⁺.

### Description 21

### 1,1-Dimethylethyl 7-{[(5-methyl-2-phenyl-2H-1,2,3-triazol-4-yl)carbonyl]amino}-1,2,4,5-tetrahydro-3H-3-benzazepine-3-carboxylate (D21)

Description 21 (D21) was prepared using an analogous method to that described for Description 20 (D20) using 5-methyl-2-phenyl-2H-1,2,3-triazole-4-carboxylic acid.
MS (ES+) m/e 448 [M+H]⁺.

### Description 22

### 5-Methyl-1-phenyl-N-(2,3,4,5-tetrahydro-1H-3-benzazepin-7-yl)-1H-pyrazole-4-carboxamide (D22)

Trifluoroacetic acid (4ml) was added dropwise to a solution of 1,1-dimethylethyl 7-{[(5-methyl-1-phenyl-1*H*-pyrazol-4-yl)carbonyl]amino}-1,2,4,5-tetrahydro-3*H*-3-benzazepine-3-carboxylate (D20) (960mg, 2.15mmol) and 1,3-dimethoxybenzene (4ml) in dichloromethane (12ml) at 0°C. The mixture was stirred at room temperature for 3 hours, applied to a SCX cartridge (Isolute-flash, 10g), and washed with methanol followed by a 1:4 mixture of 2M ammonia:methanol to elute the title compound (D22) (675 mg, 91%).
MS (ES+) m/e 347 [M+H]⁺.

### Description 23

### 5-Methyl-2-phenyl-N-(2,3,4,5-tetrahydro-1H-3-benzazepin-7-yl)-2H-1,2,3-triazole-4-carboxamide (D23)

Description 23 (D23) was prepared using an analogous method to that described for Description 22 (D22) from 1,1-dimethylethyl 7-{[(5-methyl-2-phenyl-2*H*-1,2,3-triazol-4-yl)carbonyl]amino}-1,2,4,5-tetrahydro-3H-3-benzazepine-3-carboxylate (D21) (1.24 g, 2.77 mmol) to give the title compound (D23) (0.869 g, 90%). MS (ES+) m/e 348 [M+H]⁺.

### Example 1

### N-(3-Cyclobutyl-2,3,4,5-tetrahydro-1H-3-benzazepin-7-yl)-4-morpholinecarboxamide (E1)

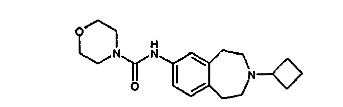

*N*-(2,3,4,5-Tetrahydro-1*H*-3-benzazepin-7-yl)-4-morpholinecarboxamide (D4) (250mg, 0.9mmol) was dissolved in 1% acetic acid in methanol (20ml) at 0 °C and treated dropwise with cyclobutanone (95mg, 1.35mmol). The mixture was stirred for 30 minutes and then (polystyrylmethyl)trimethylammonium borohydride (2mmol/g, 900mg, 1.8mmol) was added portion wise. The reaction mixture was stirred at room temperature for 18 hours, applied to a SCX (Varian bond-elute, 10g) and washed with methanol and then a mixture of .880 ammonia/ methanol. The combined basic fractions concentrated *in vacuo* and the residue purified by column chromatography eluting with a mixture of .880 ammonia: ethanol: dichloromethane (2:18:80) to afford the title compound (E1). MS (ES+) m/e 330 [M+H]⁺.

### Example 2

### 4-Cyano-N-(3-cyclobutyl-2,3,4,5-tetrahydro-1H-3-benzazepin-7-yl)benzamide (E2)

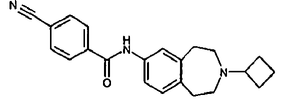

3-Cyclobutyl-2,3,4,5-tetrahydro-1*H*-3-benzazepin-7-amine (D2) (162mg, 0.75mmol) was stirred in dichloromethane (12ml) with diethylaminomethyl polystyrene (3.2mmol/g, 70mg, 2.25 mmol). 4-Cyanobenzoyl chloride (149mg, 0.9mmol) was added and the mixture stirred at room temperature for 1 hour. The reaction was diluted with methanol (10ml) and applied to a SCX ion exchange cartridge (Varian bond-elute, 10g), washed with methanol and then a mixture of .880 ammonia:methanol (1:9). The combined basic fractions were concentrated *in vacuo* and the residue purified by column chromatography .880 ammonia: ethanol: dichloromethane (2:18:80) to afford the title compound (E2). MS (ES+) m/e 346 [M+H]⁺.

### Examples 3-5

Examples 3-5 (E3-5) were prepared using an analogous method to that described for Example 2 (E2) from the appropriate acid chloride indicated in the table:

| **Example** | **Acid Chloride** | **LC/MS (M+H⁺)** |
|---|---|---|
| *N*-(3-Cyclobutyl-2,3,4,5-tetrahydro-1*H* 3-benzazepin-7-yl)tetrahydro-2*H*-pyran-4-carboxamide (E3) | Tetrahydro-2*H*-pyran-4-carbonyl chloride (Helv. Chim. Acta. 1997, 80(5), 1528) | 329 |
| *N*-(3-Cyclobutyl-2,3,4,5-tetrahydro-1*H*- 3-benzazepin-7-yl)acetamide (E4) | Acetyl chloride | 259 |
| *N*-(3-Cyclobutyl-2,3,4,5-tetrahydro-1*H-* 3-benzazepin-7-yl)cyclopropanecarboxamide (E5) | Cyclopropane carbonyl chloride. | 285 |

### Example 6

### N-(3-Cyclobutyl-2,3,4,5-tetrahydro-1H-3-benzazepin-7-yl)methanesulfonamide (E6)

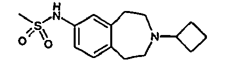

3-Cyclobutyl-2,3,4,5-tetrahydro-1*H*-3-benzazepin-7-amine (D2) (151mg, 0.75mmol) was stirred in dichloromethane (3ml). Methane sulfonyl chloride (103mg, 0.9mmol) was added and the mixture stirred at room temperature for 1 hour. Methanol (1ml) was added and the reaction was stirred for a further 0.5 hours then concentrated *in vacuo.* The crude mixture was triturated with diethyl ether, filtered and the solid was redissolved in methanol and concentrated *in vacuo* to afford the title compound (E6). MS (ES+) m/e 295 [M+H]⁺.

### Example 7

### 6-[(3-Cyclobutyl-2,3,4,5-tetrahydro-1H-3-benzazepin-7-yl)amino]-N-methyl-3-pyridinecarboxamide (E7)

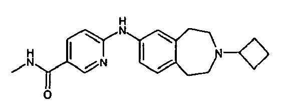

Palladium (II) acetate (11mg, 0.05mmol) was added to a stirred solution of 3-cyclobutyl-2,3,4,5-tetrahydro-1*H*-3-benzazepin-7-amine (D2) (216mg, 1.0mmol), 6-chloro-N-methyl-3-pyridinecarboxamide (PCT lnt. Appl. (2002), WO 02/0246186) (108mg, 1mmol), (±)-2,2'-bis(diphenylphosphino)-1,1'-binapthyl (31 mg, 0.05mmol) and potassium carbonate (276mg, 2mmol) in toluene (14ml). The reaction mixture was heated at reflux for 4 hours, cooled to room temperature and applied to a SCX ion exchange cartridge (Varian bond-elute, 10g), washed with methanol and then a mixture of .880 ammonia:methanol. The combined basic fractions were concentrated *in vacuo* and the residue purified by column chromatography .880 ammonia: ethanol: dichloromethane (1:9:90) to afford the title compound (E7). MS (ES+) m/e 346 [M+H]⁺.

### Example 8

### .N-(3-Cyclobutyl-2,3,4,5-tetrahydro-1H-3-benzazepin-7-yl)-4-(2-pyridinyl)benzamide (E8)

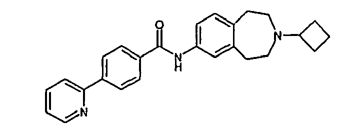

A mixture 4-(2-pyridinyl)benzoic acid (219 mg, 1.1 mmol), 1-hydroxybenzotriazole hydrate (297 mg, 2.2 mmol) and N-cyclohexylcarbodiimide-N'-methyl polystyrene (1.3 g, 2.2 mmol, resin loading 1.8mmol/g) in dimethylformamide (5ml) was stirred at room temperature for 30 minutes. A solution of 3-cyclobutyl-2,3,4,5-tetrahydro-1*H*-3-benzazepin-7-amine (D2) (200 mg, 0.9 mmol) in dimethylformamide (3ml) was added and the mixture stirred at room temperature for 18 hours. The mixture was filtered through an SCX ion exchange column eluting with methanol followed by 2.0 M ammonia solution in methanol to elute the product. The residue was purified by silica gel chromatography eluting with a 1:9:90 mixture of 0.880 ammonia solution:methanol:dichloromethane to afford the title compound (E8) (199 mg, 54%); MS(ES+) m/e 398 [M+H]⁺.

### Examples 9-12

Examples 9-12 (E9-E12) were synthesised in the same manner as Example 8 (E8) from 3-cyclobutyl-2,3,4,5-tetrahydro-1*H*-3-benzazepin-7-amine (D2) using dichloromethane and/or dimethylformamide as solvent and substituting 4-(2-pyridinyl) benzoic acid with the appropriate acid as shown in the table:

| **Example** | **Acid** | **Mass Spectrum** |
|---|---|---|
| *N*-(3-Cyclobutyl-2,3,4,5-tetrahydro-1*H*-3- benzazepin-7-yl)-5-hydroxy-2- pyrazinecarboxamide (E9) | 5-Hydroxy-2- pyrazinecarboxylic acid | MS (ES+), m/e 339 [M+H]⁺. |
| *N*-(3-Cyclobutyl-2,3,4,5-tetrahydro-1*H*-3- benzazepin-7-yl)-4-(1,3-oxazol-4- yl)benzamide (E10) | 4-(1,3-Oxazol-4- yl)benzoic acid (D17) | MS (ES+), m/e 388 [M+H]⁺. |
| *N*-(3-Cyclobutyl-2,3,4,5-tetrahydro-1*H*-3- benzazepin-7-yl)-4-iodobenzamide (E11) | 4-lodobenzoic acid | MS (ES+), m/e 447 [M+H]⁺. |
| *N*-(3-Cyclobutyl-2,3,4,5-tetrahydro-1*H*-3- benzazepin-7-yl)-4-(3-pyridinyl)benzamide (E12) | 4-(3-Pyridinyl) benzoic acid | MS (ES+), m/e 398 [M+H]⁺. |

### Example 13

Example 13 (E13) was prepared from 5-methyl-1-phenyl-*N*-(2,3,4,5-tetrahydro-1*H*-3-benzazepin-7-yl)-1*H*-pyrazole-4-carboxamide (D22) using an analogous method to that described for Example 277 (E277) from the appropriate ketone indicated in the table, except after the SCX column, the residues were purified by MDAP.

| **Example** | **Ketone** | **LC/MS (M+H⁺)** |
|---|---|---|
| *N*-(3-Cyclohexyl-2,3,4,5-tetrahydro-1*H*-3-benzazepin-7-yl)-5-methyl-1-phenyl- 1*H*-pyrazole-4-carboxamide trifluoroacetate (E13) | Cyclohexanone | 429 |

### Examples 14-85

Examples 14-85 (E14-E85) were synthesised in the same manner as Example 8 (E8) from 3-cyclobutyl-2,3,4,5-tetrahydro-1 H-3-benzazepin-7-amine (D2) using dichloromethane and/or dimethylformamide as solvent and substituting 4-(2-pyridinyl) benzoic acid with the appropriate acid as shown in the table:

| **Example** | **Acid** | **Mass Spectrum** |
|---|---|---|
| *N*-(3-Cyclobutyl-2,3,4,5-tetrahydro-1*H*-3- benzazepin-7-yl)-4-(1,3-oxazol-5-yl) benzamide (E14) | 4-(1,3-Oxazol-5-yl) benzoic acid | MS (ES+), m/e 388 [M+H]⁺. |
| *N*-(3-Cyclobutyl-2,3,4,5-tetrahydro-1*H*-3- benzazepin-7-yl)-4-(3,5-dimethyl-4- isoxazolyl)benzamide (E15) | 4-(3,5-Dimethyl-4- isoxazolyl) benzoic acid (Oriental Journal of Chemistry (1998), 14(1), 151-152) | MS (ES+), m/e 416 [M+H]⁺. |
| *N*-(3-Cyclobutyl-2,3,4,5-tetrahydro-1*H*-3- benzazepin-7-yl)-4-imidazo[1,2-a]pyridin-2- ylbenzamide (E16) | 4-Imidazo[1,2-a] pyridin-2-ylbenzoic acid (WO 95/34540) | MS (ES+), m/e 437 [M+H]⁺. |
| *N*-(3-Cyclobutyl-2,3,4,5-tetrahydro-1*H*-3- benzazepin-7-yl)-1,2,5-oxadiazole-3- carboxamide (E17) | 1,2,5-Oxadiazole- 3-carboxylic acid | MS (ES+), m/e 313 [M+H]⁺. |
| *N*-(3-Cyclobutyl-2,3,4,5-tetrahydro-1*H*-3- benzazepin-7-yl)-3-isoxazolecarboxamide (E18) | 3-lsoxazole carboxylic acid | MS (ES+), m/e 312 [M+H]⁺. |
| *N*-(3-Cyclobutyl-2,3,4,5-tetrahydro-1*H*-3- benzazepin-7-yl) cyclohexane carboxamide (E19) | Cyclohexane carboxylic acid | MS (ES+), m/e 327 [M+H]⁺. |
| *N*-(3-Cyclobutyl-2,3,4,5-tetrahydro-1*H*-3- benzazepin-7-yl)-2-cyclopentyl acetamide (E20) | 2-Cyclopentyl acetic acid | MS (ES+), m/e 327 [M+H]⁺. |
| *N*-(3-Cyclobutyl-2,3,4,5-tetrahydro-1*H*-3- benzazepin-7-yl)-3-methylbenzamide (E21) | 3-Methyl benzoic acid | MS (ES+), m/e 335 [M+H]⁺. |
| *N*-(3-Cyclobutyl-2,3,4,5-tetrahydro-1*H*-3- benzazepin-7-yl)-4-fluorobenzamide (E22) | 4-Fluorobenzoic acid | MS (ES+), m/e 339 [M+H]⁺. |
| *N*-(3-Cyclobutyl-2,3,4,5-tetrahydro-1*H*-3- benzazepin-7-yl)-3-methyl-2- thiophenecarboxamide (E23) | 3-Methyl-2- thiophene carboxylic acid | MS (ES+), m/e 341 [M+H]⁺. |
| *N*-(3-Cyclobutyl-2,3,4,5-tetrahydro-1*H*-3- benzazepin-7-yl)bicyclo[4.2.0]octa-1,3,5- triene-7-carboxamide (E24) | Bicyclo[4.2.0]octa- 1,3,5-triene-7- carboxylic acid | MS (ES+), m/e 347 [M+H]⁺. |
| *N*-(3-Cyclobutyl-2,3,4,5-tetrahydro-1*H*-3-benzazepin-7-yl)-2-methylbenzamide (E25) | 2-Methylbenzoic acid | MS (ES+), m/e 335 [M+H]⁺. |
| *N*-(3-Cyclobutyl-2,3,4,5-tetrahydro-1*H*-3- benzazepin-7-yl)-4-methylbenzamide (E26) | 4-Methylbenzoic acid | MS (ES+), m/e 335 [M+H]⁺. |
| *N*-(3-Cyclobutyl-2,3,4,5-tetrahydro-1*H*-3- benzazepin-7-yl)-2-ethylbutanamide (E27) | 2-Ethylbutanoic acid | MS (ES+), m/e 315 [M+H]⁺. |
| *N*-(3-Cyclobutyl-2,3,4,5-tetrahydro-1*H*-3- benzazepin-7-yl)-3-fluorobenzamide (E28) | 3-Fluorobenzoic acid | MS (ES+), m/e 339 [M+H]⁺. |
| *N*-(3-Cyclobutyl-2,3,4,5-tetrahydro-1*H*-3- benzazepin-7-yl)-4-(5- pyrimidinyl)benzamide (E29) | 4-(5-Pyrimidinyl) benzoic acid | MS (ES+), m/e 399 [M+H]⁺. |
| *N*-(3-Cyclobutyl-2,3,4,5-tetrahydro-1*H*-3- benzazepin-7-yl)-4- (methylsulfonyl)benzamide (E30) | 4-(Methylsulfonyl) benzoic acid | MS (ES+), m/e 399 [M+H]⁺. |
| *N*-(3-Cyclobutyl-2,3,4,5-tetrahydro-1*H*-3- benzazepin-7-yl)-6- quinoxalinecarboxamide (E31) | 6-Quinoxaline carboxylic acid | MS (ES+), m/e 373 [M+H]⁺. |
| *N*-(3-Cyclobutyl-2,3,4,5-tetrahydro-1*H*-3- benzazepin-7-yl)-2-methyl-1,6- naphthyridine-3-carboxamide (E32) | 2-Methyl-1,6- naphthyridine-3- carboxylic acid | MS (ES+), m/e 387 [M+H]⁺. |
| *N*-(3-Cyclobutyl-2,3,4,5-tetrahydro-1*H*-3- benzazepin-7-yl)-4-(1*H*-tetrazol-1- yl)benzamide (E33) | 4-(1*H*-Tetrazol-1- yl)benzoic acid | MS (ES+), m/e 389 [M+H]⁺. |
| 4-(6-Cyano-3-pyridinyl)-N-(3-cyclobutyl- 2,3,4,5-tetrahydro-1*H*-3-benzazepin-7- yl)benzamide (E34) | 4-(6-Cyano-3- pyridinyl)benzoic acid (D19) | MS (ES+), m/e 423 [M+H]⁺. |
| *N*-(3-Cyclobutyl-2,3,4,5-tetrahydro-1*H*-3- benzazepin-7-yl)-2-(trifluoromethyl)-1,8- naphthyridine-3-carboxamide (E35) | 2- (Trifluoromethyl)- 1,8-naphthyridine-3-carboxylic acid | MS (ES+), m/e 441 [M+H]⁺. |
| *N*-(3-Cyclobutyl-2,3,4,5-tetrahydro-1*H*-3- benzazepin-7-yl)-4-methylnicotinamide (E36) | 4-Methylnicotinic | MS (ES+), m/e 336 [M+H]⁺. |
| *N*-(3-Cyclobutyl-2,3,4,5-tetrahydro-1*H*-3- benzazepin-7-yl)-6-methylnicotinamide (E37) | 6-Methyl nicotinic acid | MS (ES+), m/e 336 [M+H]⁺. |
| *N*-(3-Cyclobutyl-2,3,4,5-tetrahydro-1*H*-3- benzazepin-7-yl)nicotinamide (E38) | Nicotinic acid | MS (ES+), m/e 322 [M+H]⁺. |
| *N*-(3-Cyclobutyl-2,3,4,5-tetrahydro-1*H*-3- benzazepin-7-yl)-4- (trifluoromethyl)nicotinamide (E39) | 4-(Trifluoromethyl) nicotinic acid | MS (ES+), m/e 390 [M+H]⁺. |
| *N*-(3-Cyclobutyl-2,3,4,5-tetrahydro-1*H*-3- benzazepin-7-yl)-6-(1*H*-pyrazol-1- yl)nicotinamide (E40) | 6-(1*H*-Pyrazol-1- yl)nicotinic acid | MS (ES+), m/e 388 [M+H]⁺. |
| *N*-(3-Cyclobutyl-2,3,4,5-tetrahydro-1*H-*3*-* benzazepin-7-yl)-6- (trifluoromethyl)nicotinamide (E41) | 6-(Trifluoromethyl) nicotinic acid | MS (ES+), m/e 390 [M+H]⁺. |
| *N*-(3-Cyclobutyl-2,3,4,5-tetrahydro-1*H*-3- benzazepin-7-yl)-4-(5-methyl-1*H*-tetrazol- 1-yl)benzamide (E42) | 4-(5-Methyl-1*H*- tetrazol-1- yl)benzoic acid *(*J. Org. Chem, 1956, 21, 767) | MS (ES+), m/e 403 [M+H]⁺. |
| *N*-(3-Cyclobutyl-2,3,4,5-tetrahydro-1*H*-3- benzazepin-7-yl)-5-(3-pyridinyl)-1*H*- pyrazole-3-carboxamide (E43) | 5-(3-Pyridinyl)-1*H*- pyrazole-3- carboxylic acid (J. Chem. Soc, 1933, 350) | MS (ES+), m/e 388 [M+H]⁺. |
| *N*-(3-Cyclobutyl-2,3,4,5-tetrahydro-1*H*-3- benzazepin-7-yl)-6-(4-morpholinyl)-3- pyridinecarboxamide (E44) | 6-(4-Morpholinyl)- 3-pyridine carboxylic acid | MS (ES+), m/e 407 [M+H]⁺. |
| *N*-(3-Cyclobutyl-2,3,4,5-tetrahydro-1*H*-3- benzazepin-7-yl)-1*H*-1,2,3-benzotriazole- 5-carboxamide (E45) | 1*H*-1,2,3- Benzotriazole-5- carboxylic acid *(*Synth. Commun, 1993,23,14, 2019) | MS (ES+), m/e 362 [M+H]⁺. |
| *N*-(3-Cyclobutyl-2,3,4,5-tetrahydro-1*H*-3- benzazepin-7-yl)-4-(2-pyrazinyl)benzamide (E46) | 4-(2- Pyrazinyl)benzoic acid *(*Syn. Lett, 2000, 6, 829) | MS (ES+), m/e 399 [M+H]⁺. |
| *N*-(3-Cyclobutyl-2,3,4,5-tetrahydro-1*H*-3- benzazepin-7-yl)-2-methyl-1,8- naphthyridine-3-carboxamide (E47) | 2-Methyl-1,8- naphthyridine-3- carboxylic acid | MS (ES+), m/e 387 [M+H]⁺. |
| *N*-(3-Cyclobutyl-2,3,4,5-tetrahydro-1*H*-3- benzazepin-7-yl)-5-quinoxaline carboxamide (E48) | 5-Quinoxaline carboxylic acid | MS (ES+), m/e 373 [M+H]⁺. |
| *N*-(3-Cyclobutyl-2,3,4,5-tetrahydro-1*H*-3- benzazepin-7-yl)pyrazolo[1,5-a]pyrimidine- 3-carboxamide (E49) | Pyrazolo[1,5- a]pyrimidine-3- carboxylic acid | MS (ES+), m/e 362 [M+H]⁺. |
| *N*-(3-Cyclobutyl-2,3,4,5-tetrahydro-1*H*-3- benzazepin-7-yl)-5-pyrimidinecarboxamide (E50) | 5-Pyrimidine carboxylic acid | MS (ES+), m/e 323 [M+H]⁺. |
| *N*-(3-Cyclobutyl-2,3,4,5-tetrahydro-1*H*-3-benzazepin-7-yl)-4-pyridazinecarboxamide (E51) | 4-Pyridazine carboxylic acid | MS (ES+), m/e323 [M+H]⁺. |
| *N*-(3-Cyclobutyl-2,3,4,5-tetrahydro-1*H*-3- benzazepin-7-yl)-3-(5-pyrimidinyl) benzamide (E52) | 3-(5-Pyrimidinyl) benzoic acid (D5) | MS (ES+), m/e 399 [M+H]⁺. |
| *N*-(3-Cyclobutyl-2,3,4,5-tetrahydro-1*H*-3- benzazepin-7-yl)-3-(2-pyrazinyl)benzamide (E53) | 3-(2- Pyrazinyl)benzoic acid (D6) | MS (ES+), m/e 399 [M+H]⁺. |
| *N*-(3-Cyclobutyl-2,3,4,5-tetrahydro-1*H*-3- benzazepin-7-yl)-3-(4-pyrimidinyl) benzamide (E54) | 3-(4-Pyrimidinyl) benzoic acid (D7) | MS (ES+), m/e 399 [M+H]⁺. |
| *N*-(3-Cyclobutyl-2,3,4,5-tetrahydro-1*H*-3- benzazepin-7-yl)-4-(4- pyrimidinyl)benzamide (E55) | 4-(4-Pyrimidinyl) benzoic acid (D8) | MS (ES+), m/e 399 [M+H]⁺. |
| *N*-(3-Cyclobutyl-2,3,4,5-tetrahydro-1H-3- benzazepin-7-yl)-5-methyl-3- isoxazolecarboxamide (E56) | 5-Methylisoxazole- 3-carboxylic acid | MS (ES+), m/e 326 [M +H]⁺ |
| 6-Cyano-N-(3-cyclobutyl-2,3,4,5- tetrahydro-1H-3-benzazepin-7-yl)-3- pyridinecarboxamide (E57) | 6-Cyanonicotinic acid | MS (ES+), m/e 347 [M +H]⁺ |
| N-(3-Cyclobutyl-2,3,4,5-tetrahydro-1H-3- benzazepin-7-yl)-4-(1 H-imidazol-1- yl)benzamide (E58) | 4-(1 H-Imidazol-1- yl)benzoic acid | MS (ES+), m/e 387 [M +H]⁺ |
| N-(3-Cyclobutyl-2,3,4,5-tetrahydro-1H-3- benzazepin-7-yl)-2-pyrazinecarboxamide (E59) | 2-Pyrazine carboxylic acid | MS (ES+), m/e 323 [M +H]⁺ |
| N-(3-Cyclobutyl-2,3,4,5-tetrahydro-1H-3- benzazepin-7-yl)-2-methyl-3- pyridinecarboxamide (E60) | 2-Methylnicotinic acid | MS (ES+), m/e 336 [M +H]⁺ |
| N-(3-Cyclobutyl-2,3,4,5-tetrahydro-1H-3- benzazepin-7-yl)-3-methyl-2- pyridinecarboxamide (E61) | 3-Methylpicolinic acid | MS (ES+), m/e 336 [M +H]⁺ |
| N-(3-Cyclobutyl-2,3,4,5-tetrahydro-1H-3- benzazepin-7-yl)-3-thiophenecarboxamide (E62) | Thiophene-3- carboxylic acid | MS (ES+), m/e 327 [M +H]⁺ |
| N-(3-Cyclobutyl-2,3,4,5-tetrahydro-1H-3- benzazepin-7-yl)-3-furancarboxamide (E63) | Furan-3-carboxylic acid | MS (ES+), m/e 311 [M +H]⁺ |
| N-(3-Cyclobutyl-2,3,4,5-tetrahydro-1H-3- benzazepin-7-yl)-2-methyl-3- furancarboxamide (E64) | 2-Methylfuran-3- carboxylic acid | MS (ES+), m/e 325 [M +H]⁺ |
| N-(3-Cyclobutyl-2,3,4,5-tetrahydro-1H-3- | 2,5-Dimethylfuran- | MS (ES+), m/e |
| benzazepin-7-yl)-2,5-dimethyl-3- furancarboxamide (E65) | 3-carboxylic acid | 339 [M +H]⁺ |
| N-(3-Cyclobutyl-2,3,4,5-tetrahydro-1H-3- benzazepin-7-yl)-2-methyl-5-phenyl-3- furancarboxamide (E66) | 2-Methyl-5-phenyl- 3-furan carboxylic acid | MS (ES+), m/e 401 [M +H]⁺ |
| N-(3-Cyclobutyl-2,3,4,5-tetrahydro-1H-3- benzazepin-7-yl)-1,2,3-thiadiazole-4- carboxamide (E67) | 1,2,3-Thiadiazole- 4-carboxylic acid | MS (ES+), m/e 329 [M +H]⁺ |
| N-(3-Cyclobutyl-2,3,4,5-tetrahydro-1H-3- benzazepin-7-yl)-1,3-thiazole-4- carboxamide (E68) | 1,3-Thiazole-4- carboxylic acid | MS (ES+), m/e 328 [M +H]⁺ |
| N-(3-Cyclobutyl-2,3,4,5-tetrahydro-1H-3- benzazepin-7-yl)-5-methyl-2-phenyl-2H- 1,2,3-triazole-4-carboxamide (E69) | 5-Methyl-2-phenyl- 2H-1,2,3-triazole- 4-carboxylic acid | MS (ES+), m/e 402 [M +H]⁺ |
| N-(3-Cyclobutyl-2,3,4,5-tetrahydro-1H-3- benzazepin-7-yl)-1-methyl-1H-imidazole-4- carboxamide (E70) | 1-Methyl-1H- imidazole-4- carboxylic acid | MS (ES+), m/e 325 [M +H]⁺ |
| N-(3-Cyclobutyl-2,3,4,5-tetrahydro-1H-3-benzazepin-7-yl)-1-methyl-1H-1,2,3-triazole-4-carboxamide (E71) | 1-Methyl-1H-1,2,3-triazole-4-carboxylic acid (J. Org. Chem. 41 (6), 1041-1051 (1976) | MS (ES+), m/e 326 [M +H]⁺ |
| N-(3-Cyclobutyl-2,3,4,5-tetrahydro-1H-3- benzazepin-7-yl)-5-phenyl-1,3-oxazole-4- carboxamide (E72) | 5-Phenyl-1,3- oxazole-4- carboxylic acid | MS (ES+), m/e 388 [M +H]⁺ |
| N-(3-Cyclobutyl-2,3,4,5-tetrahydro-1H-3- benzazepin-7-yl)-2-phenyl-2H-1,2,3- triazole-4-carboxamide (E73) | 2-Phenyl-2H- 1,2,3-triazole-4- carboxylic acid | MS (ES+), m/e 388 [M +H]⁺ |
| 2-(2,1,3-Benzoxadiazol-5-yl)-N-(3- cyclobutyl-2,3,4,5-tetrahydro-1H-3- benzazepin-7-yl)-1,3-thiazole-4- carboxamide (E74) | 2-(2,1,3- Benzoxadiazol-5- yl)-1,3-thiazole-4-carboxylic acid | MS (ES+), m/e 446 [M +H]⁺ |
| N-(3-Cyclobutyl-2,3,4,5-tetrahydro-1H-3- benzazepin-7-yl)-1,5-dimethyl-1H- pyrazole-3-carboxamide (E75) | 1,5-Dimethyl-1H- pyrazole-3-, carboxylic acid | MS (ES+), m/e 339 [M+H]⁺ |
| *N*-(3-Cyclobutyl-2,3,4,5-tetrahydro-1*H*-3- benzazepin-7-yl)-1,2,5-thiadiazole-3- carboxamide (E76) | 1,2,5-Thiadiazole- 3-carboxylic acid | MS (ES+), m/e 329 [M +H]⁺ |
| N-(3-Cyclobutyl-2,3,4,5-tetrahydro-1H-3- benzazepin-7-yl)-6-(1H-1,2,4-triazol-1-yl)- 3-pyridinecarboxamide (E77) | 6-(1H-1,2,4- Triazol-1-yl)-3- pyridinecarboxylic acid | MS (ES+), m/e 389 [M +H]⁺ |
| N-(3-Cyclobutyl-2,3,4,5-tetrahydro-1H-3- benzazepin-7-yl)-6-phenyl-3- pyridinecarboxamide (E78) | 6-Phenyl-3- pyridinecarboxylic acid | MS (ES+), m/e 398 [M +H]⁺ |
| N-(3-Cyclobutyl-2,3,4,5-tetrahydro-1H-3- benzazepin-7-yl)-3,3'-bipyridine-5- carboxamide (E79) | 3,3'-Bipyridine-5- carboxylic acid | MS (ES+), m/e 399 [M +H]⁺ |
| N-(3-Cyclobutyl-2,3,4,5-tetrahydro-1H-3- benzazepin-7-yl)-6-oxo-1,6-dihydro-3- pyridinecarboxamide (E80) | 6-Hydroxynicotinic acid | MS (ES+), m/e 338 [M+H]⁺ |
| N-(3-Cyclobutyl-2,3,4,5-tetrahydro-1H-3- benzazepin-7-yl)-3-methyl-5- isoxazolecarboxamide (E81) | 3-Methylisoxazole- 4-carboxylic acid | MS (ES+), m/e 326 [M +H]⁺ |
| N-(3-Cyclobutyl-2,3,4,5-tetrahydro-1H-3- benzazepin-7-yl)-5-methyl-2- pyrazinecarboxamide (E82) | 2-Methylpyrazine- 5-carboxylic acid | MS (ES+), m/e 337 [M +H]⁺ |
| N-(3-Cyclobutyl-2,3,4,5-tetrahydro-1H-3- benzazepin-7-yl)-6-oxo-1,6-dihydro-2- pyridinecarboxamide (E83) | 6-Hydroxypicolinic acid | MS (ES+), m/e 338 [M +H]⁺ |
| N-(3-Cyclobutyl-2,3,4,5-tetrahydro-1H-3- benzazepin-7-yl)-2,4-dimethyl-1,3-thiazole- 5-carboxamide (E84) | 2,4- Dimethylthiazole- 5-carboxylic acid | MS (ES+), m/e 356 [M+H]⁺ |
| N-(3-Cyclobutyl-2,3,4,5-tetrahydro-1H-3- benzazepin-7-yl)-2-phenyl-1,3-thiazole-4- carboxamide (E85) | 2-Phenyl-1,3- thiazole-4- carboxylic acid | MS (ES+), m/e 404 [M +H]⁺ |

### Example 86

### N-(3-Cyclobutyl-2,3,4,5-tetrahydro-1H-3-benzazepin-7-yl)-4-(1,1-dioxido-2-isothiazolidinyl)benzamide (E86)

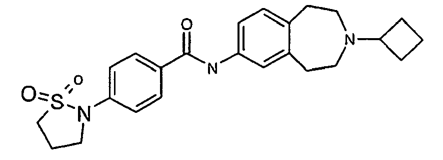

A mixture of *N*-(3-cyclobutyl-2,3,4,5-tetrahydro-1*H*-3-benzazepin-7-yl)-4-iodobenzamide (E11) (150mg, 0.34 mmol), potassium carbonate (169 mg, 1.22 mmol), copper (1) iodide (19 mg, 0.1 mmol), *N,N'*-dimethyl-1,2-ethanediamine (0.01 ml, 0.1 mmol) and isothiazolidine 1,1-dioxide (123 mg, 1.0 mmol) in dioxan (3 ml) was heated in a microwave reactor at 140 °C for 20 minutes. The mixture was diluted with methanol and purified on an SCX ion exchange cartridge eluting with methanol and then a 2M methanolic ammonia solution. The basic fractions were concentrated *in vacuo* and the residue purified by column chromatography eluting with a mixture of 2M methanolic ammonia solution and dichloromethane (3-97) to afford the title compound (E86) MS (ES+), m/e 440 [M+H]⁺.

### Examples 87-90

Examples 87-90 (E87-E90) were synthesised from E11 substituting isothiazolidine 1, 1-dioxide with the appropriate nitrogen containing heterocycle as shown in the table:

| **Example** | **Heterocycle** | **Mass Spectrum** |
|---|---|---|
| *N*-(3-Cyclobutyl-2,3,4,5-tetrahydro-1*H*-3- benzazepin-7-yl)-4-(2-oxo-1- pyrrolidinyl)benzamide (E87) | Pyrrolidinone | MS (ES+), m/e 404 [M+H]⁺. |
| *N*-(3-Cyclobutyl-2,3,4,5-tetrahydro-1*H*-3- benzazepin-7-yl)-4-(2-oxo-1- imidazolidinyl)benzamide (E88) | 2-Imidazolidinone | MS (ES+), m/e 405 [M+H]⁺. |
| *N*-(3-Cyclobutyl-2,3,4,5-tetrahydro-1*H*-3- benzazepin-7-yl)-4-(1*H*-pyrazol-1- yl)benzamide (E89) | 1*H*-Pyrazole | MS (ES+), m/e 387 [M+H]⁺. |
| *N*-(3-Cyclobutyl-2,3,4,5-tetrahydro-1*H*-3- benzazepin-7-yl)-4-(1*H*-1,2,4-triazol-1 - yl)benzamide (E90) | 1*H*-1,2,4-Triazole | MS (ES+), m/e 388 [M+H]⁺. |

### Examples 91-93

Examples 91-93 (E91-E93) were synthesised from *N*-(3-cyclobutyl-2,3,4,5-tetrahydro-1*H*-3-benzazepin-7-yl)-3-iodobenzamide (D9) using the method of Example 86 substituting isothiazolidine 1,1-dioxide with the appropriate nitrogen containing heterocycle as shown in the table:

| **Example** | **Heterocycle** | **Mass Spectrum** |
|---|---|---|
| *N*-(3-Cyclobutyl-2,3,4,5-tetrahydro-1*H*-3- benzazepin-7-yl)-3-(2-oxo-1- pyrrolidinyl)benzamide (E91) | Pyrrolidinone | MS (ES+), m/e 404 [M+H]⁺. |
| *N*-(3-Cyclobutyl-2,3,4,5-tetrahydro-1*H*-3- benzazepin-7-yl)-3-(1,1-dioxido-2- isothiazolidinyl)benzamide (E92) | Isothiazolidine 1,1- dioxide *(*J. Org. Chem, 1961, 26, 4841) | MS (ES+), m/e 440 [M+H]⁺. |
| *N*-(3-Cyclobutyl-2,3,4,5-tetrahydro-1*H*-3- benzazepin-7-yl)-3-(2-oxo-1- imidazolidinyl)benzamide (E93) | 2-Imidazolidinone | MS (ES+), m/e 405 [M+H]⁺. |

### Example 94

### N-(3-Cyclobutyl-2,3,4,5-tetrahydro-1H-3-benzazepin-7-yl)-3-(1H-pyrazol-1-yl)benzamide (E94)

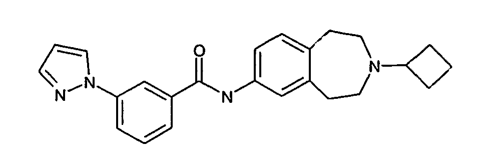

A mixture of *N*-(3-cyclobutyl-2,3,4,5-tetrahydro-1*H*-3-benzazepin-7-yl)-3-iodobenzamide (D9) (150 mg, 0.34 mmol), potassium phosphate (259 mg, 1.22 mmol), copper (1) iodide (19 mg, 0.1 mmol), trans-1,2-cyclohexanediamine (0.01 ml, 0.1 mmol) and 1*H*-pyrazole (46 mg, 0.68 mmol) in 1,4-dioxane (3 ml) was heated in a microwave reactor at 140 °C for 20 minutes. The mixture was diluted with methanol and purified on an SCX ion exchange cartridge eluting with methanol and then a 2M methanolic ammonia solution. The basic fractions were concentrated *in vacuo* and the residue purified by column chromatography eluting with a mixture of 2M methanolic ammonia solution and dichloromethane (5-95) to afford the title compound (E94) MS (ES+), m/e 387 [M+H]⁺.

### Example 95

### N-(3-Cyclobutyl-2,3,4,5-tetrahydro-1H-3-benzazepin-7-yl)-3-(1H-1,2,4-triazol-1-yl)benzamide (E95)

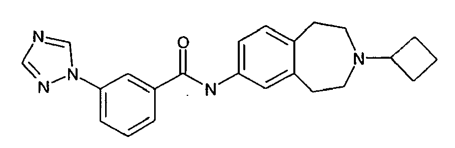

A mixture of *N*-(3-cyclobutyl-2,3,4,5-tetrahydro-1*H*-3-benzazepin-7-yl)-3-iodobenzamide (D9) (150 mg, 0.34 mmol), potassium carbonate (94 mg, 0.68 mmol), copper (1) iodide (7 mg, 0.03 mmol) and 1*H*-1,2,4-triazole (47 mg, 0.68 mmol) in N-methyl-2-pyrrolidone (3 ml) was heated in a microwave reactor at 190 °C for 2 hours. The mixture was diluted with methanol and purified on an SCX ion exchange cartridge eluting with methanol and then a 2M methanolic ammonia solution. The basic fractions were concentrated *in vacuo* and the residue purified by column chromatography eluting with a mixture of 2M methanolic ammonia solution and dichloromethane (5-95) to afford the title compound (E95) MS (ES+), m/e 388 [M+H]⁺.

### Example 96

### N-(3-Cyclobutyl-2,3,4,5-tetrahydro-1H-3-benzazepin-7-yl)-N'-methyl-2,5-pyrazinedicarboxamide (E96)

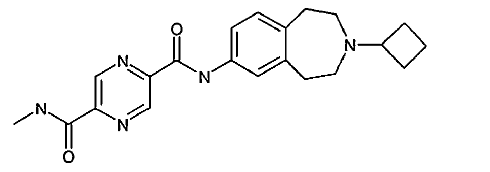

5-{[(3-Cyclobutyl-2,3,4,5-tetrahydro-1*H*-3-benzazepin-7-yl)amino]carbonyl}-2-pyrazinecarboxylic acid (D10) (118 mg, 0.31 mmol) was dissolved in N,N-dimethylformamide (4 ml), treated with N,N'-carbonyldiimidazole (151 mg, 0.93 mmol) and stirred for 5 hours at room temperature. Methylamine (2M solution in tetrahydrofuran) (0.93 ml, 1.86 mmol) was added and the mixture stirred for 18 hours. The solvent was evaporated *in vacuo* and the residue purified by column chromatography eluting with a mixture of 2M methanolic ammonia solution and dichloromethane (5-95) to afford the title compound (E96) MS (ES+), m/e 380 [M+H]⁺.

### Example 97

### N-(3-Cyclobutyl-2,3,4,5-tetrahydro-1H-3-benzazepin-7-yl)-4-(4-pyridinyl)benzamide (E97)

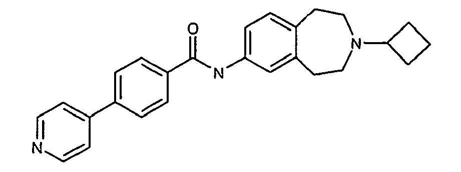

A mixture of *N*-(3-cyclobutyl-2,3,4,5-tetrahydro-1*H*-3-benzazepin-7-yl)-4-iodobenzamide (E11) (200 mg, 0.45 mmol), 4-pyridinylboronic acid (59 mg, 0.49 mmol) and 2M sodium carbonate solution (6 ml) in 1,2-bis(methyloxy)ethane (6 ml) was heated at 80 °C for 18 hours. The mixture was extracted with diethyl ether and the extracts dried and concentrated *in vacuo.* The residue was purified by column chromatography eluting with a mixture of 2M methanolic ammonia solution and dichloromethane (1-9) to afford the title compound (E97) MS (ES+), m/e 398 [M+H]⁺.

### Examples 98-101

Examples 98-101 (E98-E101) were synthesised from 6-chloro-N-(3-cyclobutyl-2,3,4,5-tetrahydro-1*H*-3-benzazepin-7-yl)-3-pyridinecarboxamide (D11) in the same manner as E97 substituting 4-pyridinylboronic acid with the boronic acids shown in the table:

| **Example** | **Boronic Acid** | **Mass Spectrum** |
|---|---|---|
| 6-(4-Cyanophenyl)-N-(3-cyclobutyl-2,3,4,5- tetrahydro-1*H*-3-benzazepin-7-yl)-3- pyridinecarboxamide (E98) | (4-Cyanophenyl) boronic acid | MS (ES+), m/e 423 [M+H]⁺. |
| *N*-(3-Cyclobutyl-2,3,4,5-tetrahydro-1*H*-3- benzazepin-7-yl)-2,3'-bipyridine-5- carboxamide (E99) | 3-Pyridinylboronic MS acid | (ES+), m/e 399 [M+H]⁺. |
| *N*-(3-Cyclobutyl-2,3,4,5-tetrahydro-1*H*-3- benzazepin-7-yl)-6-(5-pyrimidinyl)-3- pyridinecarboxamide (E100) | 5-Pyrimidinyl boronic acid | MS (ES+), m/e 400 [M+H]⁺. |
| 6-(3-Cyanophenyl)-N-(3-cyclobutyl-2,3,4,5- tetrahydro-1*H*-3-benzazepin-7-yl)-3- pyridinecarboxamide (E101) | 3-Cyanophenyl boronic acid | MS (ES+), m/e 423 [M+H]⁺. |

### Example 102

### N-(3-Cyclobutyl-2,3,4,5-tetrahydro-1H-3-benzazepin-7-yl)-5-(4-fluorophenyl)-2-pyridinecarboxamide (E102)

5-bromo-*N*-(3-cyclobutyl-2,3,4,5-tetrahydro-1*H*-3-benzazepin-7-yl)-2-pyridinecarboxamide (D12) (178mg, 0.44mmol), 4-(fluorophenyl)boronic acid (92mg, 0.66mmol), tetrakistriphenylphosphine palladium (0) 15mg, 0.013mmol), sodium carbonate (2N, 0.5ml) and 1,2-dimethoxyethane/water/ethanol 7:3:1 (5ml)) were heated in a microwave reactor at 160°c for 600 seconds at 200W. The mixture was applied to a SCX ion exchange cartridge (Varian bond-elute; 10g), washed with methanol and then with a mixture of .880 ammonia: methanol (1: 9). The combined basic fractions were concentrated *in vacuo* and the resulting residue purified by column chromatography eluting with a mixture of 10% .880 ammonia in methanol: dichloromethane (0 to 5%) to afford the title product (E102); MS (ES+) m/e 416 [M+H]⁺.

### Examples 103-105

Examples 103-105 (E103-E105) were prepared from the appropriate boronic acid, as shown in the table, with 5-bromo-*N*-(3-cyclobutyl-2,3,4,5-tetrahydro-1*H*-3-benzazepin-7-yl)-2-pyridinecarboxamide (D12) using an analogous method to that described for Example E102

| **Example** | **Boronate** | **Heating time** | **LC/MS** |
|---|---|---|---|
| 5-(4-Cyanophenyl)-*N*-(3-cyclobutyl-2,3,4,5-tetrahydro-1*H*-3-benzazepin-7-yl)-2-pyridinecarboxamide (E103) | (4-Cyanophenyl) boronic acid | 600 secs | MS (ES+), m/e 423 [M+H]⁺. |
| 6'-Cyano-*N*-(3-cyclobutyl-2,3,4,5- tetrahydro-1*H*-3-benzazepin-7-yl)- 3,3'-bipyridine-6-carboxamide (E104) 3,3'-bipyridine-6-carboxamide | 5-(4,4,5,5-Tetramethyl- 1,3,2-dioxaborolan-2- yl)-2- pyridinecarbonitrile | 900 mins | MS (ES+), m/e 424 [M+H]⁺. |
| N-(3-Cyclobutyl-2,3,4,5-tetrahydro- 1H-3-benzazepin-7-yl)-5-(5- pyrimidinyl)-2-pyridinecarboxamide (E105) | 5-Pyrimidinyl boronic acid | 900 | MS (ES+), m/e 400 [M+H]⁺. |

### Example 106

### N-(3-Cyclobutyl-2,3,4,5-tetrahydro-1H-3-benzazepin-7-yl)-5-(2-pyrazinyl)-2-pyridinecarboxamide (E106)

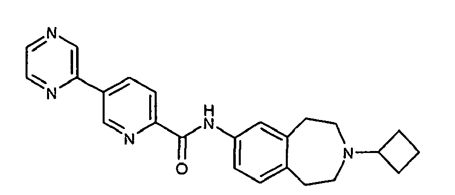

5-Bromo-*N*-(3-cyclobutyl-2,3,4,5-tetrahydro-1*H*-3-benzazepin-7-yl)-2-pyridinecarboxamide (D12) (268mg, 0.67mmol), 2-(tributylstannanyl)pyrazine (741 mg, 2.0mmol), tetrakistriphenylphosphine palladium (0) (7 mg, 0.02 mmol), in toluene (15ml)) was heated at 110°c for 4h. The cooled mixture was purified by column chromatography eluting with a mixture of 10% .880 ammonia in methanol: dichloromethane (1 to 10%) to afford the title product (E106) ; MS (ES+) m/e 400 [M+H]⁺.

### Example 107 (E107)

Example 107 was prepared from the appropriate stannane with 5-bromo-*N*-(3-cyclobutyl-2,3,4,5-tetrahydro-1*H*-3-benzazepin-7-yl)-2-pyridinecarboxamide (D12) using an analogous method to that described for Example 106:

| **Example** | **Stannane** | **Heating time** | **LC/MS** |
|---|---|---|---|
| *N*-(3-Cyclobutyl-2,3,4,5-tetrahydro- 1*H*-3-benzazepin-7-yl)-2,3'- bipyridine-6'-carboxamide (E107) | 2-(Tributylstannanyl) pyridine | 48h | MS (ES+). m/e 399 [M+H]⁺. |

### Example 108

### N-(3-Cyclobutyl-2,3,4,5-tetrahydro-1H-3-benzazepin-7-yl)-5-(1H-pyrazol-1-yl)-2-pyridinecarboxamide (E108)

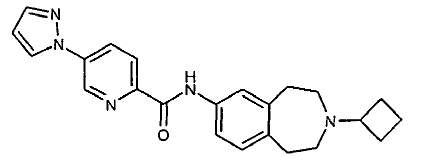

5-Bromo-*N*-(3-cyclobutyl-2,3,4,5-tetrahydro-1*H*-3-benzazepin-7-yl)-2-pyridinecarboxamide (D12) (200mg, 0.49mmol), 1*H*-pyrazole (68mg, 1.23mmol), copper (1) iodide (19mg, 0.025mmol), sodium carbonate (138mg, 0.69mmol) and N-methylpyrrolidine (5ml)) were heated in a microwave reactor at 190°c for 60 minutes at 200W. The cooled mixture was diluted with methanol (5ml), acidified with glacial acetic acid then applied to a SCX ion exchange cartridge (Varian bond-elute, 10g), washed with methanol and then with a mixture of .880 ammonia: methanol (1: 9). The combined basic fractions were concentrated *in vacuo* and the resulting residue purified by column chromatography eluting with a mixture of 10% .880 ammonia in methanol: dichloromethane (0 to 10%) to afford the title product (E108); MS (ES+) m/e 388 [M+H]⁺.

### Examples 109-110

Examples 109-110 (E109-E110) were prepared from the appropriate amine with 5-bromo-*N*-(3-cyclobutyl-2,3,4,5-tetrahydro-1*H*-3-benzazepin-7-yl)-2-pyridinecarboxamide (D12) using an analogous method to that described for Example E108:

| **Example** | **Heterocycle** | **Mass Spectrum** |
|---|---|---|
| *N*-(3-Cyclobutyl-2,3,4,5-tetrahydro-1*H*-3- benzazepin-7-yl)-5-(1*H*-imidazol-1-yl)-2- pyridinecarboxamide (E109) | 1*H*-Imidazole | MS (ES+), m/e 388 [M+H]⁺. |
| *N*-(3-Cyclobutyl-2,3,4,5-tetrahydro-1*H*-3- benzazepin-7-yl)-5-(1*H*-1,2,4-triazol-1-yl)- 2-pyridinecarboxamide (E110) | 1*H*-1,2,4-Triazole | MS (ES+), m/e 389 [M+H]⁺. |

### Example 111

### N-(3-Cyclobutyl-2,3,4,5-tetrahydro-1H-3-benzazepin-7-yl)-5-(4-morpholinyl)-2-pyrazinecarboxamide (E111)

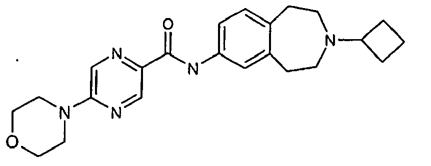

A mixture of 5-chloro-*N*-(3-cyclobutyl-2,3,4,5-tetrahydro-1*H*-3-benzazepin-7-yl)-2-pyrazinecarboxamide (D13) (200 mg, 0.56 mmol) and morpholine (0.2 ml, 2.24 mmol) in tetrahydrofuran (6 ml) was heated at reflux for 3 hours. The mixture was concentrated *in vacuo* and the residue purified by column chromatography eluting with a mixture of 2M methanolic ammonia in dichloromethane (5-95) to afford the title compound (E111) MS (ES+), m/e 408 [M+H]⁺

### Examples 112-113

Examples E112-E113 (E112-E113) were synthesised in the same manner as E111 from D13 substituting the appropriate amine for morpholine as shown in the table:

| **Example** | **Amine** | **Mass Spectrum** |
|---|---|---|
| *N*-(3-Cyclobutyl-2,3,4,5-tetrahydro-1*H*-3- benzazepin-7-yl)-5-(1-pyrrolidinyl)-2- pyrazinecarboxamide (E112) | Pyrrolidine | MS (ES+), m/e 392 [M+H]⁺. |
| *N*-(3-Cyclobutyl-2,3,4,5-tetrahydro-1*H*-3- benzazepin-7-yl)-5-(1-piperidinyl)-2- pyrazinecarboxamide (E113) | Piperidine | MS (ES+), m/e 406 [M+H]⁺. |

### Example 114

### N-(3-Cyclobutyl-2,3,4,5-tetrahydro-1H-3-benzazepin-7-yl)-5-(tetrahydro-2H-pyran-4-yloxy)-2-pyrazinecarboxamide (E114)

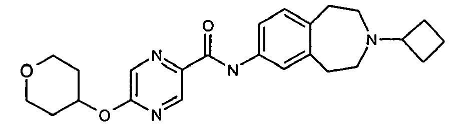

Sodium hydride (56 mg of 60% dispersion in oil, 1.4 mmol) was added to a solution of tetrahydro-2*H*-pyran-4-ol (146 mg, 104 mmol) in 1,2-bis(methyloxy)ethane (4 ml) and the mixture stirred for 30 minutes. 5-Chloro-*N*-(3-cyclobutyl-2,3,4,5-tetrahydro-1*H*-3-benzazepin-7-yl)-2-pyrazinecarboxamide (D13) (100 mg, 0.28 mmol) was added and the mixture heated at 60 °C for 18 hours. The mixture was concentrated *in vacuo* and the residue purified on an SCX ion exchange cartridge eluting with methanol and then a 2M methanolic ammonia solution. The basic fractions were concentrated *in vacuo* and the residue purified by column chromatography eluting with a mixture of 2M methanolic ammonia solution and dichloromethane (3-97) to afford the title compound (E114) MS (ES+), m/e 423 [M+H]⁺.

### Example 115

### Example 115 (E115) was prepared in the same manner as Example 114 (E114) substituting phenol for tetrahydro-2H-pyran-4-ol:

| **Example** | **Alcohol** | **Mass Spectrum** |
|---|---|---|
| *N-*(3-Cyclobutyl-2,3,4,5-tetrahydro-1*H*-3- benzazepin-7-yl)-5-(phenyloxy)-2- pyrazinecarboxamide (E115) | Phenol | MS (ES+), m/e 415 [M+H]⁺. |

### Example 116

### 5-(4-Cyanophenyl)-N-(3-cyclobutyl-2,3,4,5-tetrahydro-1H-3-benzazepin-7-yl)-2-pyrazinecarboxamide (E116)

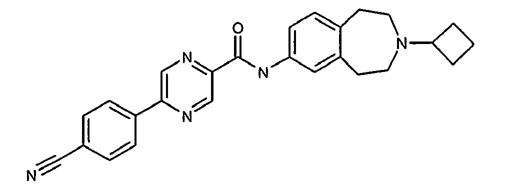

A mixture of tetrakis(triphenylphosphino)palladium (0) (32 mg, 0.03 mmol), 5-chloro-N-(3-cyclobutyl-2,3,4,5-tetrahydro-1*H*-3-benzazepin-7-yl)-2-pyrazinecarboxamide (D13) (100 mg, 0.28 mmol) and 4-cyanophenyl boronic acid (123 mg, 0.84 mmol) in 1,2-bis(methyloxy)ethane (4 ml) and 1 M sodium carbonate solution (1 ml) was heated at reflux for 3 hours. The mixture was concentrated *in vacuo* and the residue purified on an SCX ion exchange cartridge eluting with methanol and then a 2M methanolic ammonia solution. The basic fractions were concentrated *in vacuo* and the residue purified by column chromatography eluting with a mixture of 2M methanolic ammonia solution and dichloromethane (3-97) to afford the title compound (E116), NMR (CDCl₃) δ9.58 (2H, m), 9.04 (H, s), 8.24 (2H, m), 7.85 (2H, m), 7.56-7.50 (2H, m), 7.14 (H, m), 2.94 (4H, m), 2.79 (H, m), 2.47 (4H, m), 2.10 (2H, m), 1.92 (2H, m), 1.75-1.61 (2H, m)

### Example 117

Example 117 (E117) was prepared in the same manner as Example 116 (E116) substituting the appropriate boronic acid for 4-cyanophenyl boronic acid as shown in the table:

| **Example** | **Boronic Acid** | **Mass Spectrum** |
|---|---|---|
| 5-(3-Cyanophenyl)-*N*-(3-cyclobutyl-2,3,4,5- tetrahydro-1*H*-3-benzazepin-7-yl)-2- pyrazinecarboxamide (E117) | 3-Cyanophenyl boronic acid | MS (ES+), m/e 424 [M+H]⁺. |

### Example 118

### N-(3-Cyclobutyl-2,3,4,5-tetrahydro-1 H-3-benzazepin-7-yl)-4-pyridinecarboxamide (E118)

N-(2,3,4,5-Tetrahydro-1*H*-3-benzazepin-7-yl)-4-pyridinecarboxamide (D15) (0.32g, 1.2mmole) in methanol (20ml) and acetic acid (0.2ml) was treated with (polystyrylmethyl)trimethylammonium cyanoborohydride (4.1 mmole/g) (0.58g, 2.4mmole) and stirred overnight at room temperature. The residue was poured onto an SCX column and eluted with methanol followed by 2M ammonia/methanol. The basic fractions were combined and concentrated *in vacuo.* The residue was purified by column chromatography eluting with dichloromethane/(2M ammonia/methanol) (19:1) to afford the title compound (E118); MS (ES+), m/e 322 [M +H]⁺

### Examples 119-120

Examples 119-120 (E119-E120) were synthesised in the same manner as Example 118 from an analogue of D15 obtained by substituting 4-pyridinecarbonyl chloride hydrochloride in D14 with the appropriate acid chloride as shown in the table:

| **Example** | **Acid chloride** | **Mass Spectrum** |
|---|---|---|
| N-(3-Cyclobutyl-2,3,4,5-tetrahydro- 1H-3-benzazepin-7-yl)-4- biphenylcarboxamide (E119) | 4-Biphenyl carbonyl chloride | MS (ES+), m/e 397 [M +H]⁺ |
| 3-Cyano-N-(3-cyclobutyl-2,3,4,5- tetrahydro-1H-3-benzazepin-7- yl)benzamide (E120) | 3-Cyanobenzoyl chloride | MS (ES+), m/e 346 [M +H]⁺ |

### Example 121

### N-(3-Cyclobutyl-2,3,4,5-tetrahydro-1H-3-benzazepin-7-yl)-1,3-dimethyl-1H-pyrazole-5-carboxamide (E121)

3-Cyclobutyl-2,3,4,5-tetrahydro-1H-3-benzazepin-7-amine D2 (0.25g, 1.2mmole) in dichloromethane (10ml) was treated with diethylaminomethyl-polystyrene (3.2mmole/g) (1.09g, 3.5mmole) and 1,3-dimethylpyrazole-5-carbonyl chloride (0.22g, 1.4mmole) and stirred at room temperature for 4 hours. The resulting slurry was diluted with methanol, poured onto an SCX column and eluted with methanol followed by 2M ammonia/methanol. The basic fractions were combined and concentrated *in vacuo.* The residue was purified by column chromatography eluting with dichloromethane/ (2M ammonia/methanol) (32:1) to afford the title compound (E121); MS (ES+), m/e 339 [M +H]⁺

### Example 122

Example 122 (E122) was synthesised in the same manner as Example 121 substituting 1,3-dimethylpyrazole-5-carbonyl chloride with 3,5-dimethylisoxazole-4-carbonyl chloride as shown in the table:

| **Example** | **Acid Chloride** | **Mass Spectrum** |
|---|---|---|
| N-(3-Cyclobutyl-2,3,4,5-tetrahydro- 1H-3-benzazepin-7-yl)-3,5- dimethyl-4-isoxazolecarboxamide (E122) | 3,5- Dimethylisoxazole-4- carbonyl chloride | MS (ES+), m/e 340 [M +H]⁺ |

### Example 123

### 5-Bromo-N-(3-cyclobutyl-2,3,4,5-tetrahydro-1H-3-benzazepin-7-yl)-3-pyridinecarboxamide (E123)

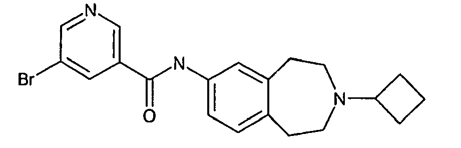

The title compound was prepared from 5-bromonicotinic acid and 3-cyclobutyl-2,3,4,5-tetrahydro-1H-3-benzazepin-7-amine (D2) using the method as described in Example 8 (E8); MS (ES+), m/e 400/402 [M +H]⁺

### Example 124

### N-(3-Cyclobutyl-2,3,4,5-tetrahydro-1H-3-benzazepin-7-yl)-5-(1H-imidazol-1-yl)-3-pyridinecarboxamide (E124)

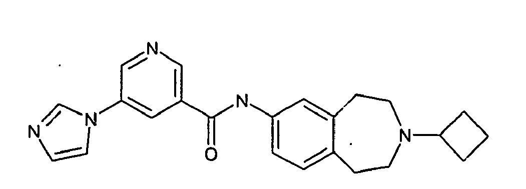

5-Bromo-N-(3-cyclobutyl-2,3,4,5-tetrahydro-1H-3-benzazepin-7-yl)-3-pyridinecarboxamide (E123) (0.20g, 0.5mmole), imidazole (0.068g, 1.0mmole), copper (I) iodide (0.019g, 0.1mmole) and potassium carbonate (0.14g, 1.0mmole) in n-methyl-2-pyrrolidone were heated together in a microwave reactor at 190°C and high absorption for 2.5 hours. The reaction mixture was diluted with methanol and passed down an SCX column eluting with methanol followed by 2M ammonia/ methanol. The basic fractions were combined and concentrated *in vacuo.* The residue was purified by column chromatography eluting with dichloromethane/ (2M ammonia/ methanol) (9:1) to afford the title compound (E124); MS (ES+), m/e 388 [M +H]⁺

### Example 125

### N-(3-Cyclobutyl-2,3,4,5-tetrahydro-1H-3-benzazepin-7-yl)-N-methyl-4-(2-pyrazinyl)benzamide (E125)

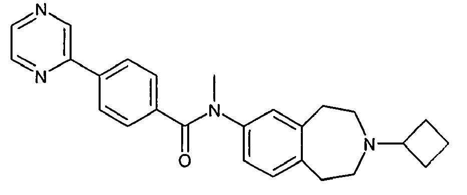

Sodium hydride (60%; 11 mg, 0.27mmol) was added to a solution of *N*-(3-cyclobutyl-2,3,4,5-tetrahydro-1*H*-3-benzazepin-7-yl)-4-(2-pyrazinyl)benzamide (E46, 66mg, 0.17mmol) in DMF (5ml). The mixture was stirred for 5 mins then methyl iodide (56mg, 0.36mmol) was added and the mixture stirred for 3h at room temperature. The reaction mixture was quenched with ammonia (0.880; 1ml) and the solvent was then evaporated. The mixture was dissolved in methanol (3ml) containing acetic acid (0.5ml) then applied to a SCX ion exchange cartridge (Varian bond-elute, 10g) and washed with methanol and then with a mixture of .880 ammonia: methanol (1: 9). The combined basic fractions were concentrated *in vacuo* to afford the title product (E125); MS (ES+) m/e 413 [M+H]⁺.

### Examples 126-128

### Examples 126-128 (E126-E128) were prepared from the appropriate secondary amide with methyl iodide using an analogous method to that described for Example 125 (see table)

| **Example** | **Starting material** | **LC/MS (M+H⁺)** |
|---|---|---|
| N-(3-Cyclobutyl-2,3,4,5-tetrahydro-1H-3- benzazepin-7-yl)-N-methyl-4-(2- tetrahydro-1*H*-3- pyridinyl)benzamide (E126) | *N*-(3-Cyclobutyl-2,3,4,5- benzazepin-7-yl)-4-(2- pyridinyl)benzamide (E8) | MS (ES+), m/e 412 [M+H]⁺. |
| *N*-(3-Cyclobutyl-2,3,4,5-tetrahydro-1*H*-3- benzazepin-7-yl)-N-methyl-4-(5- pyrimidinyl)benzamide (E127) | *N*-(3-Cyclobutyl-2,3,4,5-tetrahydro-1*H*-3- benzazepin-7-yl)-4-(5- pyrimidinyl)benzamide (E29) | MS (ES+), m/e 413 [M+H]⁺. |
| *N*-(3-Cyclobutyl-2,3,4,5-tetrahydro-1*H*-3- benzazepin-7-yl)-*N*,6-dimethyl-3- pyridinecarboxamide (E128) | *N*-(3-Cyclobutyl-2,3,4,5-tetrahydro-1*H*-3- benzazepin-7-yl)-6- methyl-3-pyridin3 carboxamide (E46) | MS (ES+), m/e 350 [M+H]⁺. |

### Example 129

### N-(3-Cyclobutyl-2,3,4,5-tetrahydro-1H-3-benzazepin-7-yl)-N'-phenylurea (E129)

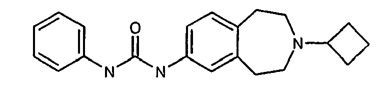

Phenyl isocyanate (92 mg, 0.77 mmol) was added to a solution of 3-cyclobutyl-2,3,4,5-tetrahydro-1*H*-3-benzazepin-7-amine (D2, 150 mg, 0.70 mmol) in dichloromethane (3 ml) and the mixture stirred for 2 hours. The mixture was diluted with methanol and purified on an SCX ion exchange cartridge eluting with methanol and then a 2M methanolic ammonia solution. The basic fractions were concentrated *in vacuo* and the residue purified by column chromatography eluting with a mixture of 2M methanolic ammonia solution and dichloromethane (3-97) to afford the title compound (E129), MS (ES+), m/e 336 [M+H]⁺.

### Examples 130-133

Examples 130-133 (E130-E133) were prepared in the same manner as E129 from 3-cyclobutyl-2,3,4,5-tetrahydro-1 H-3-benzazepin-7-amine (D2), substituting phenyl isocyanate with the isocyanates shown in the table:

| **Example** | **Isocyanate** | **Mass Spectrum** |
|---|---|---|
| *N*-(4-Cyanophenyl)-*N*'-(3-cyclobutyl- 2,3,4,5-tetrahydro-1*H*-3-benzazepin-7- yl)urea (E130) | 4-Isocyanato benzonitrile | MS (ES+), m/e 361 [M+H]⁺. |
| *N*-1,3-Benzodioxol-5-yl-*N*'-(3-cyclobutyl- 2,3,4,5-tetrahydro-1*H*-3-benzazepin-7- yl)urea (E131) | 5-Isocyanato-1,3- benzodioxole | MS (ES+), m/e 380 [M+H]⁺. |
| *N*-(3-Cyclobutyl-2,3,4,5-tetrahydro-1*H*-3- | Isocyanato | MS (ES+), m/e |
| benzazepin-7-yl)-*N'*-cyclohexylurea (E132) | cyclohexane | 342 [M+H]⁺. |
| *N*-(3-Cyclobutyl-2,3,4,5-tetrahydro-1*H*-3- benzazepin-7-yl)-*N'*-(1-methylethyl)urea (E133) | 2-Isocyanato propane | MS (ES+), m/e 302 [M+H]⁺. |

### Example 134

### N-(3-Cyclobutyl-2,3,4,5-tetrahydro-1H-3-benzazepin-7-yl)-N'-[2-(methyloxy)phenyl]urea (E134)

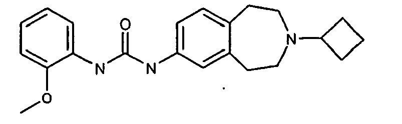

A solution of 3-cyclobutyl-2,3,4,5-tetrahydro-1 H-3-benzazepin-7-amine (D2, 100 mg, 0.46 mmol) in dichloromethane (3 ml) was added slowly to a 2M solution of phosgene in toluene (0.6 ml, 1.2 mmol) and the mixture stirred for 2 hours. The solvent was removed *in vacuo* and the residue dissolved in dichloromethane (3 ml). This solution was treated with triethylamine (0.06 ml, 0.46 mmol) and then 2-(methyloxy) aniline (113 mg, 0.92 mmol) and stirred for 2 hours. The mixture was diluted with methanol and purified on an SCX ion exchange cartridge eluting with methanol and then a 2M methanolic ammonia solution. The basic fractions were concentrated *in vacuo* and the residue purified by column chromatography eluting with a mixture of 2M methanolic ammonia solution and dichloromethane (3-97) to afford the title compound (E134), MS (ES+), m/e 366 [M+H]⁺.

### Examples 135-175

Examples 135-175 (E135-E175) were prepared in the same manner as E134, substituting 2-(methyloxy) aniline with the amines shown in the table:

| **Example** | **Amine** | **Mass Spectrum** |
|---|---|---|
| *N*-(3-Cyclobutyl-2,3,4,5-tetrahydro-1*H*-3- benzazepin-7-yl)-N'-8-quinolinylurea (E135) | 8-Quinolinamine | MS (ES+), m/e 387 [M+H]⁺. |
| *N*-(3-Cyclobutyl-2,3,4,5-tetrahydro-1*H*-3- benzazepin-7-yl)-N'-(2,2-difluoro-1,3- benzodioxol-4-yl)urea (E136) | (2,2-Difluoro-1,3- benzodioxol-4- yl)amine | MS (ES+), m/e 416 [M+H]⁺. |
| *N*-(3-Cyclobutyl-2,3,4,5-tetrahydro-1*H*-3- benzazepin-7-yl)-*N*'-3-pyridinylurea (E137) | 3-Pyridinamine | MS (ES+), m/e 337 [M+H]⁺. |
| *N*-(3-Cyclobutyl-2,3,4,5-tetrahydro-1*H*-3- benzazepin-7-yl)-*N*'-4-pyridinylurea (E138) | 4-Pyridinamine | MS (ES+), m/e 337 [M+H]⁺. |
| *N*-(3-Cyclobutyl-2,3,4,5-tetrahydro-1*H*-3- benzazepin-7-yl)-*N*'-[2-(ethyloxy) phenyl]urea (E139) | 1-(Ethyloxy)-2- methylbenzene | MS (ES+), m/e 380 [M+H]⁺. |
| *N*-(3-Cyclobutyl-2,3,4,5-tetrahydro-1*H*-3- benzazepin-7-yl)-*N*'-{2- [(trifluoromethyl)oxy]phenyl}urea (E140) | 2- [(Trifluoromethyl) oxy]aniline | MS (ES+), m/e 420 [M+H]⁺. |
| *N*-(3-Cyclobutyl-2,3,4,5-tetrahydro-1*H*-3- benzazepin-7-yl)-*N*'-[2-methyl-6- (methyloxy)phenyl]urea (E141) | 2-Methyl-6- (methyloxy)aniline | MS (ES+), m/e 380 [M+H]⁺. |
| *N*-(3-Cyanophenyl)-*N*'-(3-cyclobutyl- 2,3,4,5-tetrahydro-1*H*-3-benzazepin-7- yl)urea (E142) | 3-Amino benzonitrile | MS (ES+), m/e 361 [M+H]⁺. |
| *N*-[5-Chloro-2-(methyloxy)phenyl]-*N*'-(3- cyclobutyl-2,3,4,5-tetrahydro-1*H*-3- benzazepin-7-yl)urea (E143) | 5-Chloro-2- (methyloxy)aniline | MS (ES+), m/e 400 [M+H]⁺. |
| *N*-(3-Cyclobutyl-2,3,4,5-tetrahydro-1*H*-3- benzazepin-7-yl)-4-pyrazin-2-ylpiperazine- 1-carboxamide (E144) | 2-Piperazin-1- ylpyrazine | MS (ES+), m/e 407 [M+H]⁺. |
| 4-(5-Cyanopyridin-2-yl)-N-(3-cyclobutyl- 2,3,4,5-tetrahydro-1*H*-3-benzazepin-7- yl)piperazine-1-carboxamide (E145) | 6-Piperazin-1- ylnicotinonitrile | MS (ES+), m/e 431 [M+H]⁺. |
| 5-Chloro-*N*-(3-cyclobutyl-2,3,4,5- tetrahydro-1*H*-3-benzazepin-7-yl)indoline- 1-carboxamide (E146) | 5-Chloroindoline | MS (ES+), m/e 396 & 398 [M+H]⁺. |
| *N*-(3-Cyclobutyl-2,3,4,5-tetrahydro-1*H*-3- benzazepin-7-yl)-2,3-dihydro-1*H*-indole-1- carboxamide (E147) | 2,3-Dihydro-1*H*- indole | MS (ES+), m/e 362 [M+H]⁺. |
| *N*-(3-Cyclobutyl-2,3,4,5-tetrahydro-1*H*-3- benzazepin-7-yl)-3,4-dihydro-1(2*H*)- quinolinecarboxamide (E148) | 1,2,3,4-Tetrahydro quinoline | MS (ES+), m/e 376 [M+H]⁺. |
| *N*-(3-Cyclobutyl-2,3,4,5-tetrahydro-1*H*-3- benzazepin-7-yl)-1,3-dihydro-2*H*-isoindole- 2-carboxamide (E149) | 2,3-Dihydro-1*H*- isoindole | MS (ES+), m/e 362 [M+H]⁺. |
| *N*-(3-Cyclobutyl-2,3,4,5-tetrahydro-1*H*-3- benzazepin-7-yl)-3,4-dihydro-2(1*H*)- isoquinolinecarboxamide (E150) | 1,2,3,4-Tetrahydro isoquinoline | MS (ES+), m/e 376 [M+H]⁺. |
| *N*-(3-Cyclobutyl-2,3,4,5-tetrahydro-1*H*-3- benzazepin-7-yl)-4-phenyl-1- piperidinecarboxamide (E151) | 4-Phenylpiperidine | MS (ES+), m/e 404 [M+H]⁺. |
| 4-[(4-Cyanophenyl)oxy]-*N*-(3-cyclobutyl- 2,3,4,5-tetrahydro-1*H*-3-benzazepin-7-yl)- 1-piperidinecarboxamide (E152) | 4-(4- Piperidinyloxy) benzonitrile (WO 2002/012190) | MS (ES+), m/e 445 [M+H]⁺. |
| *N*-(3-Cyclobutyl-2,3,4,5-tetrahydro-1*H*-3- benzazepin-7-yl)-4-(2-pyridinyl)-1-piperidinecarboxamide (E153) | 2-(4-Piperidinyl) pyridine *(*Tetrahedron Lett, 1993, 34, 33, 5287) | MS (ES+), m/e 405 [M+H]⁺. |
| *N*-(3-Cyclobutyl-2,3,4,5-tetrahydro-1*H*-3- benzazepin-7-yl)-4-(4-pyridinyl)-1- piperidinecarboxamide (E154) | 4-(4-Piperidinyl) pyridine *(*Bioorg. Med. Chem. Lett, 2001, 11, 16, 2213) | MS (ES+), m/e 405 [M+H]⁺. |
| *N*-(3-Cyclobutyl-2,3,4,5-tetrahydro-1*H*-3- benzazepin-7-yl)-4-phenyl-1- piperazinecarboxamide (E155) | 1- Phenylpiperazine | MS (ES+), m/e 405 [M+H]⁺. |
| 4-(4-Cyanophenyl)-*N*-(3-cyclobutyl-2,3,4,5- tetrahydro-1*H*-3-benzazepin-7-yl)-1- piperazinecarboxamide (E156) | 4-(1-Piperazinyl) benzonitrile | MS (ES+), m/e 430 [M+H]⁺. |
| *N*-(3-Cyclobutyl-2,3,4,5-tetrahydro-1*H*-3- benzazepin-7-yl)-1-piperidinecarboxamide (E157) | Piperidine | MS (ES+), m/e 328 [M+H]⁺. |
| *N*-(3-Cyclobutyl-2,3,4,5-tetrahydro-1*H*-3- benzazepin-7-yl)-1-pyrrolidinecarboxamide (E158) | Pyrrolidine | MS (ES+), m/e 314 [M+H]⁺. |
| *N*-(3-Cyclobutyl-2,3,4,5-tetrahydro-1*H*-3- benzazepin-7-yl)-5-fluoro-2,3-dihydro-1*H*- indole-1-carboxamide (E159) | 5-Fluoro-2,3- dihydro-1*H*-indole | MS (ES+), m/e 380 [M+H]⁺. |
| *N*-(3-Cyclobutyl-2,3,4,5-tetrahydro-1*H*-3- benzazepin-7-yl)-4-(4-fluorophenyl)-1- piperidinecarboxamide (E160) | 4-(4-Fluorophenyl) piperidine | MS (ES+), m/e 422 [M+H]⁺. |
| *N*-(3-Cyclobutyl-2,3,4,5-tetrahydro-1*H*-3- benzazepin-7-yl)-2-phenyl-1- pyrrolidinecarboxamide (E161) | 2- Phenylpyrrolidine | MS (ES+), m/e 390 [M+H]⁺. |
| 5-Cyano-*N*-(3-cyclobutyl-2,3,4,5- tetrahydro-1*H*-3-benzazepin-7-yl)-2,3- dihydro-1*H*-indole-1-carboxamide (E162) | 2,3-Dihydro-1*H*- indole-5- carbonitrile *(*Tetrahedron, 1967,23,3823) | MS (ES+), m/e 387 [M+H]⁺. |
| 4-(4-Cyanophenyl)-N-(3-cyclobutyl-2,3,4,5- tetrahydro-1*H*-3-benzazepin-7-yl)-1- piperidinecarboxamide (E163) | 4-(4-Piperidinyl) benzonitrile | MS (ES+), m/e 429 [M+H]⁺. |
| *N*-(3-Cyclobutyl-2,3,4,5-tetrahydro-1*H*-3- benzazepin-7-yl)-1,2,4,5-tetrahydro-3*H*-3- benzazepine-3-carboxamide (E164) | 2,3,4,5- Tetrahydro-1*H*-3- benzazepine (J. Med. Chem, 2003, 46,23,4952) | MS (ES+), m/e 390 [M+H]⁺. |
| 4-(3-Cyano-2-pyrazinyl)-N-(3-cyclobutyl- 2,3,4,5-tetrahydro-1*H*-3-benzazepin-7-yl)- 1-piperazinecarboxamide (E165) | 3-(1-Piperazinyl)- 2-pyrazine carbonitrile | MS (ES+), m/e 432 [M+H]⁺. |
| *N*-(3-Cyclobutyl-2,3,4,5-tetrahydro-1*H*-3- benzazepin-7-yl)-2,3-dihydro-4*H*-1,4- benzoxazine-4-carboxamide (E166) | 3,4-Dihydro-2*H*- 1,4-benzoxazine | MS (ES+), m/e 378 [M+H]⁺. |
| N-(3-Cyclobutyl-2,3,4,5-tetrahydro-1*H*-3- benzazepin-7-yl)-4-{[4- (methyloxy)phenyl]oxy}-1- piperidinecarboxamide (E167) | 4-{[4- (Methyloxy)phenyl] oxy}piperidine | MS (ES+), m/e 450 [M+H]⁺. |
| *N*-(3-Cyclobutyl-2,3,4,5-tetrahydro-1*H*-3- benzazepin-7-yl)-4-[(4-fluorophenyl)oxy]-1- piperidinecarboxamide (E168) | 4-[(4- Fluorophenyl)oxy] piperidine | MS (ES+), m/e 438 [M+H]⁺. |
| *N*-(3-Cyclobutyl-2,3,4,5-tetrahydro-1*H*-3- benzazepin-7-yl)-4-(phenyloxy)-1- piperidinecarboxamide (E169) | 4-(Phenyloxy) piperidine | MS (ES+), m/e 420 [M+H]⁺. |
| *N*-(3-Cyclobutyl-2,3,4,5-tetrahydro-1*H*-3- benzazepin-7-yl)-4-(2-pyridinyloxy)-1- piperidinecarboxamide (E170) | 2-(4- Piperidinyloxy) pyridine | MS (ES+), m/e 421 [M+H]⁺. |
| *N*-(3-Cyclobutyl-2,3,4,5-tetrahydro-1*H*-3- benzazepin-7-yl)-4-(3-pyridinyloxy)-1- piperidinecarboxamide (E171) | 3-(4- Piperidinyloxy) pyridine *(*Bioorg. Med. Chem. Lett, 2000, 10, 10, 1063) | MS (ES+), m/e 421 [M+H]⁺. |
| 7-Cyano-*N*-(3-cyclobutyl-2,3,4,5- tetrahydro-1*H*-3-benzazepin-7-yl)-2,3- dihydro-4*H*-1,4-benzoxazine-4- carboxamide (E172) | 3,4-Dihydro-2*H*- 1,4-benzoxazine- 7-carbonitrile (WO 2003/059269) | MS (ES+), m/e 403 [M+H]⁺. |
| *N*-(4-Cyanophenyl)-*N*'-(3-cyclobutyl- 2,3,4,5-tetrahydro-1*H*-3-benzazepin-7-yl)- *N*-methylurea (E173) | 4-(Methylamino) benzonitrile | MS (ES+), m/e 375 [M+H]⁺. |
| *N*-(3-Cyclobutyl-2,3,4,5-tetrahydro-1*H*-3- benzazepin-7-yl)-2,3-dihydro-1*H*- pyrrolo[2,3-b]pyridine-1-carboxamide (E174) | 2,3-Dihydro-1*H*- pyrrolo[2,3- b]pyridine *(*Tetrahedron Lett, 1987,28,4,379) | MS (ES+), m/e 363 [M+H]⁺. |
| 5-Cyano-*N*-(3-cyclobutyl-2,3,4,5- tetrahydro-1*H*-3-benzazepin-7-yl)-1,3- dihydro-2*H*-isoindole-2-carboxamide (E175) | 2,3-Dihydro-1*H*- isoindole-5- carbonitrile *(*Bioorg. Med. Chem. Lett, 2001,11, 5, 685) | MS (ES+), m/e 387 [M+H]⁺. |

### Example 176

### 1-{6-[(3-Cyclobutyl-2,3,4,5-tetrahydro-1H-3-benzazepin-7-yl)amino]-3-pyridinyl}-2-pyrrolidinone (E176)

A mixture of 3-cyclobutyl-2,3,4,5-tetrahydro-1*H*-3-benzazepin-7-amine (D2, 200 mg, 0.93 mmol), 1-(6-chloro-3-pyridinyl)-2-pyrrolidinone (D18, 272 mg, 1.39 mmol), sodium tert-butoxide (133 mg, 1.39 mmol), (±)-2,2'-bis(diphenylphosphino)-1,1'-binapthyl (52 mg, 0.09 mmol) and dipalladium *tris*-dibenzylidene acetone (82 mg, 0.09 mmol) in 1,4-dioxan (5 ml) was heated at reflux for 18 hours. The reaction mixture was diluted with methanol and passed down an SCX column eluting with methanol followed by 2M ammonia/ methanol. The basic fractions were combined and concentrated *in vacuo.* The residue was purified by column chromatography eluting with dichloromethane/ (2M ammonia/ methanol) (9:1). The residue was further purified by MDAP to afford the title compound (E176),
MS (ES+) m/e 377 [M+H]⁺.

### Example 177

### N-(3-Cyclobutyl-2,3,4,5-tetrahydro-1H-3-benzazepin-7-yl)-1,3-benzodioxole-5-carboxamide trifluoroacetate (E177)

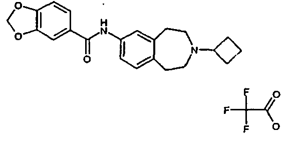

A mixture of 3-cyclobutyl-2,3,4,5-tetrahydro-1*H*-3-benzazepin-7-amine (D2) (30mg, 0.14mmol), 1,3-benzodioxole-5-carboxylic acid (35mg, 0.21mmol), O-(1*H*-benzotriazol-1-yl)-N,N,N',N'-tetramethyluronium hexafluorophosphate (79mg, 0.21mmol) and triethylamine (0.03ml, 0.22mmol) in dimethylformamide (3.5ml) was shaken at room temperature for 18 hours. The mixture was quenched with water (0.6ml), concentrated *in vacuo* and the residue purified by MDAP to afford the title compound (E177). MS (ES+) m/e 365 [M+H]⁺.

### Examples 178-266

Examples 178-266 (E178-266) were prepared from 3-cyclobutyl-2,3,4,5-tetrahydro-1*H*-3-benzazepin-7-amine (D2), using an analogous method to that described for Example 177 (E177) from the appropriate acid indicated in the table:

| **Example** | **Acid** | **LC/MS (M+H⁺)** |
|---|---|---|
| *N*-(3-Cyclobutyl-2,3,4,5-tetrahydro-1*H*-3-benzazepin-7-yl)-2-methyl-1*H*- benzimidazole-5-carboxamide trifluoroacetate (E178) | 2-Methyl-1*H*-benzimidazole-5- carboxylic acid | 375 |
| *N*-(3-Cyclobutyl-2,3,4,5-tetrahydro-1*H*- 3-benzazepin-7-yl)-2-quinoxalinecarboxamide trifluoroacetate (E179) | 2-Quinoxalinecarboxylic acid | 373 |
| *N*-(3-Cyclobutyl-2,3,4,5-tetrahydro-1*H*-3-benzazepin-7-yl)-2- (methyloxy)benzamide trifluoroacetate (E180) | 2-(Methyloxy)benzoic acid | 351 |
| *N*-(3-Cyclobutyl-2,3,4,5-tetrahydro-1*H*-3-benzazepin-7-yl)pyrazolo[1,5- a]pyridine-3-carboxamide trifluoroacetate (E181) | Pyrazolo[1,5-a]pyridine-3- carboxylic acid | 361 |
| *N*-(3-Cyclobutyl-2,3,4,5-tetrahydro-1*H*-3-benzazepin-7-yl)-5-methyl-1-[4- (methyloxy)phenyl]-1*H*-pyrazole-4- carboxamide trifluoroacetate (E182) | 5-Methyl-1-[4-(methyloxy)phenyl]-1*H*-pyrazole-4-carboxylic acid | 431 |
| *N*-(3-Cyclobutyl-2,3,4,5-tetrahydro-1*H*-3-benzazepin-7-yl)-3- (methyloxy)benzamide trifluoroacetate (E183) | 3-(Methyloxy)benzoic acid | 351 |
| *N*-(3-Cyclobutyl-2,3,4,5-tetrahydro-1*H*-3-benzazepin-7-yl)-1-benzothiophene-2-carboxamide trifluoroacetate (E184) | 1-Benzothiophene-2-carboxylic acid | 377 |
| *N*-(3-Cyclobutyl-2,3,4,5-tetrahydro-1*H*-3-benzazepin-7-yl)-2-naphthalenecarboxamide trifluoroacetate (E185) | 2-Naphthalenecarboxylic acid | 371 |
| 1-(4-Chlorophenyl)-*N*-(3-cyclobutyl- 2,3,4,5-tetrahydro-1*H*-3-benzazepin-7- yl)-5-(trifluoromethyl)-1*H*-pyrazole-4- carboxamide trifluoroacetate (E186) | 1-(4-Chlorophenyl)-5-(trifluoromethyl)-1*H*-pyrazole-4- carboxylic acid- | 489 |
| *N*-(3-Cyclobutyl-2,3,4,5-tetrahydro-1*H*-3-benzazepin-7-y)-1-(1-methylethyl)- 1*H*-1,2,3-benzotriazole-5-carboxamide trifluoroacetate (E187) | 1-(1-Methylethyl)-1*H*-1,2,3- benzotriazole-5-carboxylic acid | 404 |
| *N*-(3-Cyclobutyl-2,3,4,5-tetrahydro-1*H-* 3-benzazepin-7-yl)-1-(1-methylethyl)-2- (trifluoromethyl)-1*H*-benzimidazole-5- carboxamide trifluoroacetate (E188) | 1-(1-Methylethyl)-2-(trifluoromethyl)-1*H*- benzimidazole-5-carboxylic acid | 471 |
| *N*-(3-Cyclobutyl-2,3,4,5-tetrahydro-1*H*- 3-benzazepin-7-yl)-2,1-benzisoxazole-3- carboxamide trifluoroacetate (E189) | 2,1-Benzisoxazole-3-carboxylic acid | 362 |
| *N*-(3-Cyclobutyl-2,3,4,5-tetrahydro-1*H*- 3-benzazepin-7-yl)-1-methyl-3-propyl- 1*H*-pyrazole-5-carboxamide trifluoroacetate (E190) | 1-Methy)-3-propyl-1*H*-pyrazole-5-carboxylic acid | 367 |
| *N*-(3-Cyclobutyl-2,3,4,5-tetrahydro-1*H*- 3-benzazepin-7-yl)-1.5-dimethyl-3-oxo- 2-phenyl-2,3-dihydro-1*H*-pyrazole-4-carboxamide trifluoroacetate (E191) | 1,5-Dimethyl-3-oxo-2-phenyl-2,3-dihydro-1*H*-pyrazole-4-carboxylic acid | 431 |
| *N*-(3-Cyclobutyl-2,3,4,5-tetrahydro-1*H*-3-benzazepin-7-yl)-2-methyl-2*H*-1,2,3- triazole-4-carboxamide trifluoroacetate (E192) | 2-Methyl-2*H*-1,2,3-triazole-4- carboxylic acid | 326 |
| *N*-(3-Cyclobutyl-2,3,4,5-tetrahydro-1*H*- 3-benzazepin-7-yl)-1-benzothiophene-3- carboxamide trifluoroacetate (E193) | -Benzothiophene-3-carboxylic 1-Benzothiophene-3-carboxylic acid | 377 |
| *N*-(3-Cyclobutyl-2,3,4,5-tetrahydro-1*H-*3-benzazepin-7-yl)-1-phenyl-1*H*-1,2,3- triazole-5-carboxamide trifluoroacetate (E194) | 1-Phenyl-1*H*-1,2,3-triazole-5- carboxylic acid | 388 |
| *N*-(3-Cyclobutyl-2,3,4,5-tetrahydro-1*H*-3-benzazepin-7-yl)-3-methyl-5-[4-(methyloxy)phenyl]-1*H*-pyrazole-4- carboxamide trifluoroacetate (E195) | 3-Methyl-5-[4-(methyloxy)phenyl]- 1*H*-pyrazole-4-carboxylic acid | 431 |
| *N*-(3-Cyclobutyl-2,3,4,5-tetrahydro-1*H*-3-benzazepin-7-yl)-5-methyl-1-phenyl- 1*H*-pyrazole-3-carboxamide trifluoroacetate (E196) | 5-Methyl-1-phenyl-1*H*-pyrazole-3- carboxylic acid | 401 |
| *N*-(3-Cyclobutyl-2,3,4,5-tetrahydro-1*H*-3-benzazepin-7-yl)-3-[3- (methyloxy)phenyl]-1-phenyl-1*H*- pyrazole-4-carboxamide trifluoroacetate (E197) | 3-[3-(Methyloxy)phenyl]-1-phenyl-1*H*-pyrazole-4-carboxylic acid | 493 |
| *N*-(3-Cyclobutyl-2,3,4,5-tetrahydro-1*H*-3-benzazepin-7-yl)-3-(1,1- dimethylethyl)-1-methyl-1*H*-pyrazole-5- carboxamide trifluoroacetate (E198) | 3-(1,1-Dimethylethyl)-1-methyl- 1*H*-pyrazole-5-carboxylic acid | 381 |
| *N*-(3-Cyclobutyl-2,3,4,5-tetrahydro-1*H*-3-benzazepin-7-yl)-5,7- dimethylpyrazolo[1,5-a]pyrimidine-2- carboxamide trifluoroacetate (E199) | 5,7-Dimethylpyrazolo[1,5- a]pyrimidine-2-carboxylic acid | 390 |
| N-(3-Cyclobutyl-2,3,4,5-tetrahydro-1*H*- 3-benzazepin-7-yl)-3-methyl-1-phenyl- 1*H*-pyrazole-5-carboxamide trifluoroacetate (E200) | 3-Methyl-1-phenyl-1*H*-pyrazole-5-carboxylic acid | 401 |
| *N*-(3-Cyclobutyl-2,3,4,5-tetrahydro-1*H*-3-benzazepin-7-yl)-1-methyl-1*H*-1,2,3- benzotriazole-5-carboxamide trifluoroacetate (E201) | 1-Methyl-1*H*-1 ,2,3-benzotriazole- 5-carboxylic acid | 376 |
| *N*-(3-Cyclobutyl-2,3,4,5-tetrahydro-1*H*- 3-benzazepin-7-yl)-5-methyl-1-[(4- methylphenyl)methyl]-1*H*-pyrazofe-3-carboxamide trifluoroacetate (E202) | 5-Methyl-1-[(4-methylphenyl)methyl]-1*H*- pyrazole-3-carboxylic acid | 429 |
| *N*-(3-Cyclobutyl-2,3,4,5-tetrahydro-1*H*-3-benzazepin-7-yl)-1-ethyl-3-(2-thienyl)- 1*H*-pyrazole-5-carboxamide trifluoroacetate (E203) | 1-Ethyl-3-(2-thienyl)-1*H*-pyrazole- 5-carboxylic acid | 421 |
| *N*-(3-Cyclobutyl-2,3,4,5-tetrahydro-1*H*-3-benzazepin-7-yl)-1-ethyl-5-methyl-1*H*- pyrazole-3-carboxamide trifluoroacetate (E204) | 1-Ethyl-5-methyl-1*H*-pyrazole-3- carboxylic acid | 353 |
| *N*-(3-Cyclobutyl-2,3,4,5-tetrahydro-1*H*-3-benzazepin-7-yl)-4-[(4- fluorophenyl)oxy]-1*H*-1,2,3-triazole-5- carboxamide trifluoroacetate (E205) | 4-[(4-Fluorophenyl)oxy]-1*H*-1,2,3- triazole-5-carboxylic acid | 422 |
| *N*-(3-Cyclobutyl-2,3,4,5-tetrahydro-1*H-*3-benzazepin-7-yl)-4-ethyl-1-phenyl-1*H*- 1,2,3-triazole-5-carboxamide trifluoroacetate (E206) | 4-Ethyl-1-phenyl-1 *H*-1,2,3- triazole-5-carboxylic acid | 416 |
| *N*-(3-Cyclobutyl-2,3,4,5-tetrahydro-1*H*-3-benzazepin-7-yl)-5-ethyl-1-phenyl-1*H*- 1,2,3-triazole-4-carboxamide trifluoroacetate (E207) | 5-Ethyl-1-phenyl-1*H*-1,2,3- triazole-4-carboxylic acid | 416 |
| *N*-(3-Cyclobutyl-2,3,4,5-tetrahydro-1*H-*3-benzazepin-7-yl)-2-methyl-5-phenyl- 2*H*-1,2,3-triazole-4-carboxamide trifluoroacetate (E208) | 2-Methyl-5-phenyl-2*H*-1,2,3- triazole-4-carboxylic acid | 402 |
| *N*-(3-Cyclobutyl-2,3,4,5-tetrahydro-1*H*-3-benzazepin-7-yl)-5-methyl-1-phenyl- 1*H*-1,2,3-triazole-4-carboxamide trifluoroacetate (E209) | 5-Methyl-1-phenyl-1*H*-1,2,3- triazole-4-carboxylic acid | 402 |
| *N*-(3-Cyclobutyl-2,3,4,5-tetrahydro-1*H*-3-benzazepin-7-yl)-2*H*-chromene-3- carboxamide trifluoroacetate (E210) | 2H-Chromene-3-carboxylic acid | 375 |
| *N*-(3-Cyclobutyl-2,3,4,5-tetrahydro-1*H*- 3-benzazepin-7-yl)-2-methyl-4,5,6,7-tetrahydro-2*H*-indazole-3-carboxamide trifluoroacetate (E211) | 2-Methyl-4,5,6,7-tetrahydro-2*H*- indazole-3-carboxylic acid | 379 |
| 4-[3,4-bis(Methyloxy)phenyl]-*N*-(3-cyclobutyl-2,3,4,5-tetrahydro-1*H*-3- benzazepin-7-yl)-1*H*-1,2,3-triazole-5- carboxamide trifluoroacetate (E212) | 4-[3,4-bis(Methyloxy)phenyl]-1*H*- 1,2,3-triazole-5-carboxylic acid | 448 |
| *N*-(3-Cyclobutyl-2,3,4,5-tetrahydro-1*H*-3-benzazepin-7-yl)-1-(2,2,2- trifluoroethyl)-1*H*-1,2,3-triazole-4- carboxamide trifluoroacetate (E213) | 1-(2,2,2-Trifluoroethyl)-1*H*-1,2,3- triazole-4-carboxylic acid | 394 |
| *N*-(3-Cyclobutyl-2,3,4,5-tetrahydro-1*H*-3-benzazepin-7-yl)-3,5-dimethyl-1- phenyl-1*H*-pyrazole-4-carboxamide trifluoroacetate (E214) | 3,5-Dimethyl-1 -phenyl-1 *H*- pyrazole-4-carboxylic acid | 415 |
| *N*-(3-Cyclobutyl-2,3,4,5-tetrahydro-1*H*-3-benzazepin-7-yl)-1-methyl-1*H*- pyrazole-4-carboxamide trifluoroacetate (E215) | 1-Methyl-1*H*-pyrazol-4-carboxylic acid | 325 |
| *N*-(3-Cyclobutyl-2,3,4,5-tetrahydro-1*H*-3-benzazepin-7-yl)-1-methyl-3- (trifluoromethyl)-1*H*-pyrazole-4- carboxamide trifluoroacetate (E216) | 1-Methyl-3-(trifluoromethyl)-1*H*- pyrazole-4-carboxylic acid | 393 |
| 1-(2-Chlorophenyl)-*N*-(3-cyclobutyl-2,3,4,5-tetrahydro-1*H*-3-benzazepin-7- yl)-5-propyl-1*H*-pyrazole-4-carboxamide trifluoroacetate (E217) | 1-(2-Chlorophenyl)-5-propyl-1 *H*- pyrazole-4-carboxylic acid | 464 |
| 1-(4-Cyanophenyl)-*N*-(3-cyclobutyl- 2,3,4,5-tetrahydro-1*H*-3-benzazepin-7- yl)-3-(trifluoromethyl)-1*H*-pyrazole-4- carboxamide trifluoroacetate (E218) | 1-(4-Cyanophenyl)-3-(trifluoromethyl-1*H*-pyrazole-4- carboxylic acid | 480 |
| 1-(4-Chlorophenyl)-*N*-(3-cyclobutyl-2,3,4,5-tetrahydro-1*H*-3-benzazepin-7- yl)-5-propyl-1*H*-pyrazole-4-carboxamide trifluoroacetate (E219) | 1-(4-Chlorophenyl)-5-propyl-1*H*- pyrazole-4-carboxylic acid | 464 |
| *N*-(3-Cyclobutyl-2,3,4,5-tetrahydro-1*H*-3-benzazepin-7-yl)-3,4-dihydro-2*H*-chromene-3-carboxamide trifluoroacetate (E220) | 3,4-Dihydro-2*H*-chromene-3- carboxylic acid | 377 |
| *N*-(3-Cyclobutyl-2,3,4,5-tetrahydro-1*H-*3-benzazepin-7-yl)-1,3,5-trimethyl-1*H*- pyrazole-4-carboxamide trifluoroacetate (E221) | 1,3,5-Trimethyl-1*H*-pyrazole-4- carboxylic acid | 353 |
| *N*-(3-Cyclobutyl-2,3,4,5-tetrahydro-1*H*-3-benzazepin-7-yl)-1-(4-fluorophenyl)- 3,5-dimethyl-1*H*-pyrazole-4- carboxamide trifluoroacetate (E222) | 1-(4-Fluorophenyl)-3,5-dimethyl- 1*H*-pyrazole-4-carboxylic acid | 433 |
| *N*-(3-Cyclobutyl-2,3,4,5-tetrahydro-1*H-*3-benzazepin-7-yl)-1-benzofuran-5- carboxamide trifluoroacetate (E223) | 1-Benzofuran-5-carboxylic acid | 361 |
| *N*-(3-Cyclobutyl-2,3,4,5-tetrahydro-1*H*-3-benzazepin-7-yl)-1*H*-indole-3- carboxamide trifluoroacetate (E224) | 1*H*-Indole-3-carboxylic acid | 360 |
| *N*-(3-Cyclobutyl-2,3,4,5-tetrahydro-1*H*-3-benzazepin-7-yl)-1*H*-indazole-3- carboxamide trifluoroacetate (E225) | 1*H*-Indazole-3-carboxylic acid | 361 |
| *N*-(3-Cyclobutyl-2,3,4,5-tetrahydro-1*H*-3-benzazepin-7-yl)-2,3-dihydro-1- benzofuran-2-carboxamide trifluoroacetate (E226) | 2,3-Dihydro-1-benzofuran-2- carboxylic acid | 363 |
| *N*-(3-Cyclobutyl-2,3,4,5-tetrahydro-1*H*-3-benzazepin-7-yl)-1*H*-indole-5- carboxamide trifluoroacetate (E227) | 1*H*-Indole-5-carboxylic acid | 360 |
| *N*-(3-Cyclobutyl-2,3,4,5-tetrahydro-1*H*-3-benzazepin-7-yl)-2,3-dihydro-1- benzofuran-5-carboxamide trifluoroacetate (E228) | 2,3-Dihydro-1-benzofuran-5- carboxylic acid | 363 |
| *N*-(3-Cyclobutyl-2,3,4,5-tetrahydro-1*H*- 3-benzazepin-7-yl)-1,3-benzothiazole-6- carboxamide trifluoroacetate (E229) | 1,3-Benzothiazole-6-carboxylic acid | 378 |
| *N*-(3-Cyclobutyl-2,3,4,5-tetrahydro-1*H*-3-benzazepin-7-yl)-1 H-indole-6- carboxamide trifluoroacetate (E230) | 1*H*-Indole-6-carboxylic acid | 360 |
| *N*-(3-Cyclobutyl-2,3,4,5-tetrahydro-1*H*-3-benzazepin-7-yl)-1,2,3- benzothiadiazole-5-carboxamide trifluoroacetate (E231) | 1,2,3-Benzothiadiazole-5- carboxylic acid | 379 |
| *N*-(3-Cyclobutyl-2,3,4,5-tetrahydro-1*H*-3-benzazepin-7-yl)-2,7- dimethylpyrazolo[1,5-a]pyrimidine-6- carboxamide trifluoroacetate (E232) | 2,7-Dimethylpyrazolo[1,5- a]pyrimidine-6-carboxylic acid | 390 |
| *N*-(3-Cyclobutyl-2,3,4,5-tetrahydro-1*H*-3-benzazepin-7-yl)-1- naphthalenecarboxamide trifluoroacetate (E233) | 1-Naphthalenecarboxylic acid | 371 |
| *N*-(3-Cyclobutyl-2,3,4,5-tetrahydro-1*H*-3-benzazepin-7-yl)-3- quinolinecarboxamide trifluoroacetate (E234) | 3-Quinolinecarboxylic acid | 372 |
| *N*-(3-Cyclobutyl-2,3,4,5-tetrahydro-1*H-*3-benzazepin-7-yl)-4-(methyloxy)-2- quinolinecarboxamide trifluoroacetate (E235) | 4-(Methyloxy)-2- quinolinecarboxylic acid | 402 |
| *N*-(3-Cyclobutyl-2,3,4,5-tetrahydro-1*H*-3-benzazepin-7-yl)-2,1,3- benzothiadiazole-5-carboxamide trifluoroacetate (E236) | 2,1,3-Benzothiadiazole-5- carboxylic acid | 379 |
| *N*-(3-Cyclobutyl-2,3,4,5-tetrahydro-1*H*-3-benzazepin-7-yl)-2,3-dihydro-1,4- benzodioxin-2-carboxamide trifluoroacetate (E237) | 2,3-Dihydro-1,4-benzodioxin-2- carboxylic acid | 379 |
| *N*-(3-Cyclobutyl-2,3,4,5-tetrahydro-1*H*-3-benzazepin-7-yl)-6-(methyloxy)-2- naphthalenecarboxamide trifluoroacetate (E238) | 6-(Methyloxy)-2- naphthalenecarboxylic acid | 401 |
| *N*-(3-Cyclobutyl-2,3,4,5-tetrahydro-1*H*-3-benzazepin-7-yl)-3- isoquinolinecarboxamide trifluoroacetate (E239) | 3-Isoquinolinecarboxylic acid | 372 |
| *N*-(3-Cyclobutyl-2,3,4,5-tetrahydro-1*H*- 3-benzazepin-7-yl)-1-methyl-1*H*-indole- 2-carboxamide trifluoroacetate (E240) | 1-Methyl-1*H*-indole-2-carboxylic acid | 374 |
| *N*-(3-Cyclobutyl-2,3,4,5-tetrahydro-1*H*-3-benzazepin-7-yl)-1-benzofuran-2- carboxamide trifluoroacetate (E241) | 1-Benzofuran-2-carboxylic acid | 361 |
| *N*-(3-Cyclobutyl-2,3,4,5-tetrahydro-1*H*-3-benzazepin-7-yl)-4- (methyloxy)benzamide trifluoroacetate (E242) | 4-(Methyloxy)benzoic acid | 351 |
| *N*-(3-Cyclobutyl-2,3,4,5-tetrahydro-1*H*-3-benzazepin-7-yl)-1,3-diphenyl-1*H*- pyrazole-4-carboxamide trifluoroacetate (E243) | 1,3-Diphenyl-1*H*-pyrazole-4- carboxylic acid | 463 |
| *N*-(3-Cyclobutyl-2,3,4,5-tetrahydro-1*H*-3-benzazepin-7-yl)-2- quinolinecarboxamide trifluoroacetate (E244) | 2-Quinolinecarboxylic acid | 372 |
| *N*-(3-Cyclobutyl-2,3,4,5-tetrahydro-1*H*-3-benzazepin-7-yl)-2,3-dihydro-1,4- benzodioxin-6-carboxamide trifluoroacetate (E245) | 2,3-Dihydro-1,4-benzodioxin-6- carboxylic acid | 379 |
| N-(3-Cyclobutyl-2,3,4,5-tetrahydro-1*H-*3-benzazepin-7-yl)-4- (methylsulfonyl)benzamide trifluoroacetate (E246) | 4-(Methylsulfonyl)benzoic acid | 399 |
| *N*-(3-Cyclobutyl-2,3,4,5-tetrahydro-1*H*-3-benzazepin-7-yl)-2- (phenyloxy)benzamide trifluoroacetate (E247) | 2-(Phenyloxy)benzoic acid | 413 |
| *N*-(3-Cyclobutyl-2,3,4,5-tetrahydro-1*H-*3-benzazepin-7-yl)-3- (phenyloxy)benzamide trifluoroacetate (E248) | 3-(Phenyloxy)benzoic acid | 413 |
| *N*-(3-Cyclobutyl-2,3,4,5-tetrahydro-1*H*-3-benzazepin-7-yl)-4- (phenyloxy)benzamide trifluoroacetate (E249) | 4-(Phenyloxy)benzoic acid | 413 |
| *N*-(3-Cyclobutyl-2,3,4,5-tetrahydro-1*H*-3-benzazepin-7-yl)-2- (trifluoromethyl)benzamide trifluoroacetate (E250) | 2-(Trifluoromethyl)benzoic acid | 389 |
| *N*-(3-Cyclobutyl-2,3,4,5-tetrahydro-1*H*-3-benzazepin-7-yl)-3- (trifluoromethyl)benzamide trifluoroacetate (E251) | 3-(Trifluoromethyl)benzoic acid | 389 |
| *N*-(3-Cyclobutyl-2,3,4,5-tetrahydro-1*H*-3-benzazepin-7-yl)-4- (trifluoromethyl)benzamide trifluoroacetate (E252) | 4-(Trifluoromethyl)benzoic acid | 389 |
| *N*-(3-Cyclobutyl-2,3,4,5-tetrahydro-1*H*-3-benzazepin-7-yl)-2- biphenylcarboxamide trifluoroacetate (E253) | 2-Biphenylcarboxylic acid | 397 |
| (2*E*)-*N*-(3-Cyclobutyl-2,3,4,5-tetrahydro-1 H-3-benzazepin-7-yl)-3-phenyl-2- propenamide trifluoroacetate (E254) | (2E)-3-Phenyl-2-propenoic acid | 347 |
| *N*-(3-Cyclobutyl-2,3,4,5-tetrahydro-1*H*- 3-benzazepin-7-yl)-1*H*-benzimidazole-2- carboxamide trifluoroacetate (E255) | 1*H*-Benzimidazole-2-carboxylic acid | 361 |
| (2*E*)-*N*-(3-Cyclobutyl-2,3,4,5-tetrahydro-1*H*-3-benzazepin-7-yl)-3-[2- (methyloxy)phenyl]-2-propenamide trifluoroacetate (E256) | (2*E*)-3-[2-(Methyloxy)phenyl]-2- propenoic acid | 377 |
| (2*E*)-*N*-(3-Cyclobutyl-2,3,4,5-tetrahydro-1*H*-3-benzazepin-7-yl)-3-[3- (methyloxy)phenyl]-2-propenamide trifluoroacetate (E257) | (2*E* )-3-[3-(Methyloxy)phenyl]-2-propenoic acid | 377 |
| (2*E*)-*N*-(3-Cyclobutyl-2,3,4,5-tetrahydro-1*H*-3-benzazepin-7-yl)-3-[4- (methyloxy)phenyl]-2-propenamide trifluoroacetate (E258) | (2*E*)-3-[4-(Methyloxy)phenyl]-2- propenoic acid | 377 |
| (2*E*)-*N*-(3-Cyclobutyl-2,3,4,5-tetrahydro-1*H*-3-benzazepin-7-yl)-3-(2- fluorophenyl)-2-propenamide trifluoroacetate (E259) | (2*E*)-3-(2-Fluorophenyl)-2- propenoic acid | 365 |
| (2*E*)-*N*-(3-Cyclobutyl-2,3,4,5-tetrahydro-1*H*-3-benzazepin-7-yl)-3-(3- fluorophenyl)-2-propenamide trifluoroacetate (E260) | (2*E*)-3-(3-Fluorophenyl)-2- propenoic acid | 365 |
| (2*E*)-N-(3-Cyclobutyl-2,3,4,5-tetrahydro-1*H*-3-benzazepin-7-yl)-3-(4- fluorophenyl)-2-propenamide trifluoroacetate (E261) | (2*E*)-3-(4-Fluorophenyl)-2- propenoic acid | 365 |
| 3,5-Dichloro-*N*-(3-cyclobutyl-2,3,4,5-tetrahydro-1*H*-3-benzazepin-7- yl)benzamide trifluoroacetate (E262) | 3,5-Dichlorobenzoic acid | 390 |
| *N*-(3-Cyclobutyl-2,3,4,5-tetrahydro-1*H-*3-benzazepin-7-yl)-1-ethyl-3-methyl-1*H*- pyrazole-5-carboxamide trifluoroacetate (E263) | 1-Ethyl-3-methyl-1*H*-pyrazole-5- carboxylic acid | 353 |
| *N*-(3-Cyclobutyl-2,3,4,5-tetrahydro-1*H-*3-benzazepin-7-yl)-5-(2-furanyl)-1*H-* pyrazole-3-carboxamide trifluoroacetate (E264) | 5-(2-Furanyl)-1*H*-pyrazole-3- carboxylic acid | 377 |
| *N*-(3-Cyclobutyl-2,3,4,5-tetrahydro-1*H*-3-benzazepin-7-yl)-1-(3,4- dichlorophenyl)-3,5-dimethyl-1*H*- pyrazole-4-carboxamide trifluoroacetate (E265) | 1-(3,4-Dichlorophenyl)-3,5-dimethyl-1*H*-pyrazole-4- carboxylic acid | 484 |
| *N*-(3-Cyclobutyl-2,3,4,5-tetrahydro-1*H*- 3-benzazepin-7-yl)-3-(methyloxy)-2-naphthalenecarboxamide trifluoroacetate (E266) | 3-(Methyloxy)-2- naphthalenecarboxylic acid | 401 |

### Example 267

### N-(3-Cyclobutyl-2,3,4,5-tetrahydro-1H-3-benzazepin-7-yl)-1-(4-fluorophenyl)-5-phenyl-1H-pyrazole-3-carboxamide hydrochloride (E267)

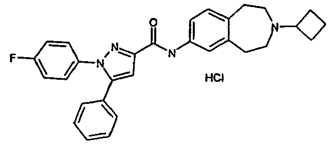

Example 267 was prepared from 3-cyclobutyl-2,3,4,5-tetrahydro-1*H*-3-benzazepin-7-amine (D2), using an analogous method to that described for Example 177 (E177) from 1-(4-fluorophenyl)-5-phenyl-1*H*-pyrazole-3-carboxylic acid (25mg, 0.09mmol) with additional steps: following MDAP, the trifluoroacetate salt was loaded onto an SCX ion exchange cartridge (Isolute-flash, 500mg), washing with dichloromethane followed by methanol, and eluted with a 1:4 mixture of 2M ammonia:methanol. The combined basic fractions were concentrated *in vacuo* and the residue stirred in 1 M hydrogen chloride solution in diethyl ether (0.015ml) in dichloromethane (1ml) for 1 hour. Concentration to dryness *in vacuo* afforded the title compound (E267) (26mg, 72%). MS(ES+ m/e 481[M+H]⁺.

### Examples 268-276

Examples 268-276 (E268-276) were prepared from 3-cyclobutyl-2,3,4,5-tetrahydro-1*H*-3-benzazepin-7-amine (D2), using an analogous method to that described for Example 267 (E267) from the appropriate acid indicated in the table:

| **Example** | **Acid** | **LC/MS** (**M+H⁺)** |
|---|---|---|
| *N*-(3-Cyclobutyl-2,3,4,5-tetrahydro-1*H*-3-benzazepin-7-yl)-1,5-diphenyl-1*H-* pyrazole-4-carboxamide hydrochloride (E268) | 1,5-Diphenyl-1*H*-pyrazole-4- carboxylic acid | 463 |
| *N*-(3-Cyclobutyl-2,3,4,5-tetrahydro-1*H-*3-benzazepin-7-yl)-5-methyl-2-(4- methylphenyl)-2*H*-1,2,3-triazole-4- carboxamide hydrochloride (E269) | 5-Methyl-2-(4-methylphenyl)-2*H*- 1,2,3-triazole-4-carboxylic acid | 416 |
| *N*-(3-Cyclobutyl-2,3,4,5-tetrahydro-1*H*-3-benzazepin-7-yl)-1-phenyl-5-(1*H*-pyrrol-1-yl)-1*H*-pyrazole-4-carboxamide hydrochloride (E270) | 1-Phenyl-5-(1*H*-pyrrol-1-yl)-1*H*- pyrazole-4-carboxylic acid | 452 |
| *N*-(3-Cyclobutyl-2,3,4,5-tetrahydro-1*H*- 3-benzazepin-7-yl)-1-(4-fluorophenyl)-5-methyl-1*H*-pyrazole-4-carboxamide hydrochloride (E271) | 1-(4-Fluorophenyl)-5-methyl-1 *H*- pyrazole-4-carboxylic acid | 419 |
| *N*-(3-Cyclobutyl-2,3,4,5-tetrahydro-1*H-*3-benzazepin-7-yl)-1-phenyl-5- (trifluoromethyl)-1*H*-pyrazole-4- carboxamide hydrochloride (E272) | 1-Phenyl-5-(trifluoromethyl)-1*H*- pyrazole-4-carboxylic acid | 455 |
| *N*-(3-Cyclobutyl-2,3,4,5-tetrahydro-1*H*-3-benzazepin-7-yl)-1-phenyl-5-propyl- 1*H*-pyrazole-4-carboxamide hydrochloride (E273) | 1-Phenyl-5-propyl-1*H*-pyrazole-4- carboxylic acid | 429 |
| *N*-(3-Cyclobutyl-2,3,4,5-tetrahydro-1*H*-3-benzazepin-7-yl)-5-methyl-1-(2- methylphenyl)-1*H*-pyrazole-4- carboxamide hydrochloride (E274) | 5-Methyl-1-(2-methylphenyl)-1*H*- pyrazole-4-carboxylic acid | 415 |
| *N*-(3-Cyclobutyl-2,3,4,5-tetrahydro-1*H*-3-benzazepin-7-yl)-1-(methyloxy)-2- naphthalenecarboxamide hydrochloride (E275) | 1-(Methyloxy)-2- naphthalenecarboxylic acid | 401 |
| 1-(4-Chlorophenyl)-N-(3-cyclobutyl-2,3,4,5-tetrahydro-1H-3-benzazepin-7- yl)-5-methyl-1H-pyrazole-4-carboxamide hydrochloride (E276) | 1-(4-Chlorophenyl)-5-methyl-1H- pyrazole-4-carboxylic acid | 435 |

### Example 277

### 5-Methyl-N-[3-(3-methylcyclopentyl)-2,3,4,5-tetrahydro-1H-3-benzazepin-7-yl]-2-phenyl-2H-1,2,3-triazole-4-carboxamide hydrochloride (E277)

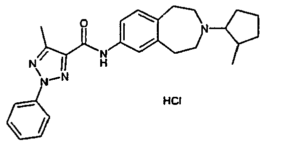

A mixture of 5-methyl-2-phenyl-*N*-(2,3,4,5-tetrahydro-1*H*-3-benzazepin-7-yl)-2*H*-1,2,3-triazole-4-carboxamide (D23, 28mg, 0.080mmol), 3-methylcyclopentanone (16mg, 0.16mmol), macroporous triacetoxyborohydride resin (2.25mmol/g, 144mg, 0.32mmol) and tetrahydrofuran (1ml) was shaken at room temperature for 5 days. 3-methylcyclopentanone (16mg, 0.16mmol) was added to the mixture which was shaken for 2 further days. Methylisocyanate polystyrene (2.86mmol/g, 86mg, 0.24mmol) was added to the reaction mixture which was shaken for a further 18 hours. The mixture was applied to a SCX ion exchange column (Isolute-flash, 500mg), washing with dichloromethane followed by methanol, and eluting with a 1:4 mixture of 2M ammonia:methanol. The combined basic fractions were concentrated *in vacuo* and the residue stirred in 1 M hydrogen chloride solution in diethyl ether (0.015ml) in dichloromethane (1ml) for 1 hour. Concentration to dryness *in vacuo* afforded the title compound (E277).
MS(ES+) m/e 430 [M+H]⁺.

### Example 278

Examples 278 (E278) was prepared using an analogous method to that described for Example 277 (E277), using 5-methyl-2-phenyl-*N*-(2,3,4,5-tetrahydro-1*H*-3-benzazepin-7-yl)-2H-1,2,3-triazole-4-carboxamide (D23) as the amine and the appropriate ketone indicated in the table:

| **Example** | **Ketone** | **LC/MS (M+H⁺)** |
|---|---|---|
| *N*-(3-Cyclohexyl-2,3,4,5-tetrahydro-1*H*-3-benzazepin-7-yl)-5-methyl-2-phenyl-2*H*-1,2,3-triazole-4-carboxamide hydrochloride (E278) | Cyclohexanone | 430 |

### Examples 279-280

Examples 279-280 (E279-E280) were prepared using an analogous method to that described for Example 277 (E277), using 5-methyl-2-phenyl-*N*-(2,3,4,5-tetrahydro-1*H*-3-benzazepin-7-yl)-2*H*-1,2,3-triazole-4-carboxamide (D23) as the amine and the appropriate ketone indicated in the table:

| **Example** | **Ketone** | **LC/MS (M+H⁺)** |
|---|---|---|
| 5-Methyl-*N*-[3-(2-methylcyclopentyl)-2,3,4,5-tetrahydro-1*H*-3-benzazepin-7- yl]-2-phenyl-2*H*-1,2,3-triazole-4-carboxamide trifluoroacetate (E279) | 2-Methylcyclopentanone | 430 |
| *N*-(3-Cyclopentyl-2,3,4,5-tetrahydro-1*H*-3-benzazepin-7-yl)-5-methyl-2-phenyl- 2*H*-1,2,3-triazole-4-carboxamide trifluoroacetate (E280) | Cyclopentanone | 416 |

### Biological Data

A membrane preparation containing histamine H3 receptors may be prepared in accordance with the following procedures:

### (i) Generation of histamine H3 cell line

DNA encoding the human histamine H3 gene (Huvar, A. et al. (1999) Mol. Pharmacol. 55(6), 1101-1107) was cloned into a holding vector, pCDNA3.1 TOPO (InVitrogen) and its cDNA was isolated from this vector by restriction digestion of plasmid DNA with the enzymes BamH1 and Not-1 and ligated into the inducible expression vector pGene (InVitrogen) digested with the same enzymes. The GeneSwitch^{™} system (a system where in transgene expression is switched off in the absence of an inducer and switched on in the presence of an inducer) was performed as described in US Patent nos: 5,364,791; 5,874,534; and 5,935,934. Ligated DNA was transformed into competent DH5α E. coli host bacterial cells and plated onto Luria Broth (LB) agar containing Zeocin^{™} (an antibiotic which allows the selection of cells expressing the sh ble gene which is present on pGene and pSwitch) at 50µg ml⁻¹. Colonies containing the re-ligated plasmid were identified by restriction analysis. DNA for transfection into mammalian cells was prepared from 250ml cultures of the host bacterium containing the pGeneH3 plasmid and isolated using a DNA preparation kit (Qiagen Midi-Prep) as per manufacturers guidelines (Qiagen).
CHO K1 cells previously transfected with the pSwitch regulatory plasmid (InVitrogen) were seeded at 2x10e6 cells per T75 flask in Complete Medium, containing Hams F12 (GIBCOBRL, Life Technologies) medium supplemented with 10% v/v dialysed foetal bovine serum, L-glutamine, and hygromycin (100µg ml⁻¹), 24 hours prior to use. Plasmid DNA was transfected into the cells using Lipofectamine plus according to the manufacturers guidelines (InVitrogen). 48 hours post transfection cells were placed into complete medium supplemented with 500µg ml⁻¹ Zeocin^{™}.
10-14 days post selection 10nM Mifepristone (InVitrogen), was added to the culture medium to induce the expression of the receptor. 18 hours post induction cells were detached from the flask using ethylenediamine tetra-acetic acid (EDTA; 1:5000; InVitrogen), following several washes with phosphate buffered saline pH 7.4 and resuspended in Sorting Medium containing Minimum Essential Medium (MEM), without phenol red, and supplemented with Earles salts and 3% Foetal Clone II (Hyclone). Approximately 1 x 10e7 cells were examined for receptor expression by staining with a rabbit polyclonal antibody, 4a, raised against the N-terminal domain of the histamine H3 receptor, incubated on ice for 60 minutes, followed by two washes in sorting medium. Receptor bound antibody was detected by incubation of the cells for 60 minutes on ice with a goat anti rabbit antibody, conjugated with Alexa 488 fluorescence marker (Molecular Probes). Following two further washes with Sorting Medium, cells were filtered through a 50µm Filcon^{™} (BD Biosciences) and then analysed on a FACS Vantage SE Flow Cytometer fitted with an Automatic Cell Deposition Unit. Control cells were non-induced cells treated in a similar manner. Positively stained cells were sorted as single cells into 96-well plates, containing Complete Medium containing 500µg ml⁻¹ Zeocin^{™} and allowed to expand before reanalysis for receptor expression via antibody and ligand binding studies. One clone, 3H3, was selected for membrane preparation.

### (ii) Membrane preparation from cultured cells

All steps of the protocol are carried out at 4°C and with pre-cooled reagents. The cell pellet is resuspended in 10 volumes of buffer A2 containing 50mM N-2-hydroxyethylpiperazine-N'-2-ethanesulfonic acid (HEPES) (pH 7.40) supplemented with 10e-4M leupeptin (acetyl-leucyl-leucyl-arginal; Sigma L2884), 25µg/ml bacitracin (Sigma B0125), 1mM ethylenediamine tetra-acetic acid (EDTA), 1 mM phenylmethylsulfonyl fluoride (PMSF) and 2x10e-6M pepstain A (Sigma). The cells are then homogenised by 2 x 15 second bursts in a 1 litre glass Waring blender, followed by centrifugation at 500g for 20 minutes. The supernatant is then spun at 48,000g for 30 minutes. The pellet is resuspended in 4 volumes of buffer A2 by vortexing for 5 seconds, followed by homogenisation in a Dounce homogeniser (10-15 strokes). At this point the preparation is aliquoted into polypropylene tubes and stored at -70°C.

Compounds of the invention may be tested for in vitro biological activity in accordance with the following assays:

### (I) Histamine H3 binding assay

For each compound being assayed, in a white walled clear bottom 96 well plate, is added:-
(a) 10µl of test compound (or 10µl of iodophenpropit (a known histamine H3 antagonist) at a final concentration of 10mM) diluted to the required concentration in 10% DMSO;
(b) 10µl ¹²⁵I 4-[3-(4-iodophenylmethoxy)propyl]-1H-imidazolium (iodoproxyfan) (Amersham; 1.85MBq/µl or 50µCi/ml; Specific Activity ~2000Ci/mmol) diluted to 200pM in assay buffer (50mM Tris(hydroxymethyl)aminomethane buffer (TRIS) pH 7.4, 0.5mM ethylenediamine tetra-acetic acid (EDTA)) to give 20pM final concentration; and
(c) 80µl bead/membrane mix prepared by suspending Scintillation Proximity Assay (SPA) bead type WGA-PVT at 100mg/ml in assay buffer followed by mixing with membrane (prepared in accordance with the methodology described above) and diluting in assay buffer to give a final volume of 80µl which contains 7.5µg protein and 0.25mg bead per well - mixture was pre-mixed at room temperature for 60 minutes on a roller.
   The plate is shaken for 5 minutes and then allowed to stand at room temperature for 3-4 hours prior to reading in a Wallac Microbeta counter on a 1 minute normalised tritium count protocol. Data was analysed using a 4-parameter logistic equation.

### (II) Histamine H3 functional antagonist assay

For each compound being assayed, in a white walled clear bottom 96 well plate, is added:-
(a) 10µl of test compound (or 10µl of guanosine 5'- triphosphate (GTP) (Sigma) as nonspecific binding control) diluted to required concentration in assay buffer (20mM N-2-Hydroxyethylpiperazine-N'-2-ethanesulfonic acid (HEPES) + 100mM NaCl + 10mM MgCl₂, pH7.4 NaOH);
(b) 60µl bead/membrane/GDP mix prepared by suspending wheat germ agglutinin-polyvinyltoluene (WGA-PVT) scintillation proximity assay (SPA) beads at 100mg/ml in assay buffer followed by mixing with membrane (prepared in accordance with the methodology described above) and diluting in assay buffer to give a final volume of 60µl which contains 10µg protein and 0.5mg bead per well - mixture is pre-mixed at 4°C for 30 minutes on a roller and just prior to addition to the plate, 10µM final concentration of guanosine 5' diphosphate (GDP) (Sigma; diluted in assay buffer) is added;
   The plate is incubated at room temperature to equilibrate antagonist with receptor/beads by shaking for 30 minutes followed by addition of:
(c) 10µl histamine (Tocris) at a final concentration of 0.3µM; and
(d) 20µl guanosine 5'[γ35-S] thiotriphosphate, triethylamine salt (Amersham; radioactivity concentration = 37kBq/µl or 1mCi/ml; Specific Activity 1160Ci/mmol) diluted to 1.9nM in assay buffer to give 0.38nM final.
   The plate is then incubated on a shaker at room temperature for 30 minutes followed by centrifugation for 5 minutes at 1500 rpm. The plate is read between 3 and 6 hours after completion of centrifuge run in a Wallac Microbeta counter on a 1 minute normalised tritium count protocol. Data is analysed using a 4-parameter logistic equation. Basal activity used as minimum i.e. histamine not added to well.

### Results

The compounds of Examples E1-10, E12-122, E124-280 were tested in the histamine H3 functional antagonist assay and exhibited antagonism > 6.5 pK_{b}. More particularly, the compounds of Examples 2, 8, 29, 33-34, 37, 44, 88, 98, 113 and 148 exhibited antagonism > 9.5 pK_{b}.

## Claims

1. A compound of formula (I) or a pharmaceutically acceptable salt thereof: 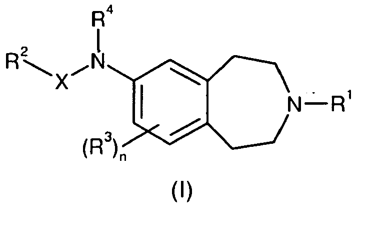 wherein:
R¹ represents -C₃₋₇ cycloalkyl optionally substituted by C₁₋₃ alkyl;
R² represents hydrogen, -C₁₋₆ alkyl, -C₃₋₈ cycloalkyl, -C₁₋₆ alkyl-C₃₋₈ cycloalkyl, -aryl, -heterocyclyl, -heteroaryl, -C₃₋₈ cycloalkyl-Y-C₃₋₈ cycloalkyl, -C₃₋₈ cycloalkyl-Y-aryl, -C₃₋₈ cycloalkyl-Y-heteroaryl, -C₃₋₈ cycloalkyl-Y-heterocyclyl, -aryl-Y-C₃₋₈ cycloalkyl, -aryl-Y-aryl, -aryl-Y-heteroaryl, -aryl-Y-heterocyclyl, -heteroaryl-Y-C₃₋₈ cycloalkyl, -heteroaryl-Y-aryl, -heteroaryl-Y-heteroaryl, -heteroaryl-Y-heterocyclyl, -heterocyclyl-Y-C₃₋₈ cycloalkyl, -heterocyclyl-Y-aryl, -heterocyclyl-Y-heteroaryl, -heterocyclyl-Y-heterocyclyl;
X represents a bond, CO, SO₂, CONR⁵, COO or COC₂₋₆ alkenyl;
Y represents a bond, C₁₋₆ alkyl, CO, CONH, NHCO, O, SO₂, SO₂NH or NHSO₂;
R³ represents halogen, C₁₋₆ alkyl, C₁₋₆ alkoxy, cyano, amino or trifluoromethyl;
R⁴ and R⁵ independently represent hydrogen, -C₁₋₆ alkyl, -C₃₋₈ cycloalkyl, -aryl, -heterocyclyl or -heteroaryl;
n is 0, 1 or 2;
wherein said alkyl, cycloalkyl, aryl, heteroaryl and heterocyclyl groups of R², R³ and R⁴ may be optionally substituted by one or more substituents (e.g. 1, 2 or 3) which may be the same or different, and which are selected from the group consisting of halogen, hydroxy, cyano, nitro, =O, haloC₁₋₆ alkyl, haloC₁₋₆ alkoxy, C₁₋₆ alkyl, C₁₋₆ alkoxy, arylC₁₋₆ alkoxy, C₁₋₆ alkylthio, C₁₋₆ alkoxyC₁₋₆ alkyl, C₃₋₇ cycloalkylC₁₋₆ alkoxy, C₁₋₆ alkanoyl, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, sulfonyl, arylsulfonyl, arylsulfonyloxy, arylsulfonylC₁₋₆ alkyl, aryloxy, C₁₋₆ alkylsulfonamido, C₁₋₆ alkylamino, C₁₋₆ alkylamido, -R⁸, -CO₂R⁸, -COR⁸, C₁₋₆ alkylsulfonamidoC₁₋₆ alkyl, C₁₋₆ alkylamidoC₁₋₆ alkyl, arylsulfonamido, arylcarboxamido, arylsulfonamidoC₁₋₆ alkyl, arylcarboxamidoC₁₋₆ alkyl, aryl, aroyl, aroylC₁₋₆ alkyl, arylC₁₋₆ alkanoyl, or a group -NR⁶R⁷, -C₁₋₆ alkyl-NR⁶R⁷, -C₃₋₈ cycloalkyl-NR⁶R⁷, -CONR⁶R⁷, -NR⁶COR⁷, -NR⁶SO₂R⁷, -OCONR⁶R⁷ , -NR⁶CO₂R⁷, -NR⁸CONR⁶R⁷ or -SO₂NR⁶R⁷ (wherein R⁶, R⁷ and R⁸ independently represent hydrogen, C₁₋₆ alkyl, -C₃₋₈ cycloalkyl, -C₁₋₆ alkyl-C₃₋₈ cycloalkyl, aryl, heterocyclyl or heteroaryl or -NR⁶R⁷ may represent a nitrogen containing heterocyclyl group, wherein said R⁵, R⁶ and R⁷ groups may be optionally substituted by one or more substituents (e.g. 1, 2 or 3) which may be the same or different, and which are selected from the group consisting of halogen, hydroxy, C₁₋₆ alkyl, C₁₋₆ alkoxy, cyano, amino, =O or trifluoromethyl);
or solvates thereof;
wherein said compound is not 7-amino-3-cyclopropyl-2,3,4,5-tetrahydro-1 H-3-benzazepine.

2. A compound according to claim 1 which is:
*N*-(3-Cyclobutyl-2,3,4,5-tetrahydro-1*H*-3-benzazepin-7-yl)-4-morpholinecarboxamide;
4-Cyano-*N*-(3-cyclobutyl-2,3,4,5-tetrahydro-1*H*-3-benzazepin-7-yl)benzamide;
*N*-(3-Cyclobutyl-2,3,4,5-tetrahydro-1*H*-3-benzazepin-7-yl)tetrahydro-2*H*-pyran-4-carboxamide;
*N*-(3-Cyclobutyl-2,3,4,5-tetrahydro-1*H-*3-benzazepin-7-yl)acetamide;
*N*-(3-Cyclobutyl-2,3,4,5-tetrahydro-1*H*-3-benzazepin-7-yl)cyclopropanecarboxamide;
*N*-(3-Cyclobutyl-2,3,4,5-tetrahydro-1*H*-3-benzazepin-7-yl)methanesulfonamide;
6-[(3-Cyclobutyl-2,3,4,5-tetrahydro-1*H*-3-benzazepin-7-yl)amino]-*N*-methyl-3-pyridinecarboxamide;
*N*-(3-Cyclobutyl-2,3,4,5-tetrahydro-1*H*-3-benzazepin-7-yl)-4-(2-pyridinyl)benzamide;
*N*-(3-Cyclobutyl-2,3,4,5-tetrahydro-1*H*-3-benzazepin-7-yl)-5-hydroxy-2-pyrazinecarboxamide;
*N*-(3-Cyclobutyl-2,3,4,5-tetrahydro-1*H*-3-benzazepin-7-yl)-4-(1,3-oxazol-4-yl)benzamide;
*N*-(3-Cyclobutyl-2,3,4,5-tetrahydro-1*H*-3-benzazepin-7-yl)-4-iodobenzamide;
*N*-(3-Cyclobutyl-2,3,4,5-tetrahydro-1*H*-3-benzazepin-7-yl)-4-(3-pyridinyl)benzamide;
*N*-(3-Cyclohexyl-2,3,4,5-tetrahydro-1*H*-3-benzazepin-7-yl)-5-methyl-1-phenyl-1*H*-pyrazole-4-carboxamide trifluoroacetate;
*N*-(3-Cyclobutyl-2,3,4,5-tetrahydro-1*H*-3-benzazepin-7-yl)-4-(1,3-oxazol-5-yl) benzamide;
*N*-(3-Cyclobutyl-2,3,4,5-tetrahydro-1*H*-3-benzazepin-7-yl)-4-(3,5-dimethyl-4-isoxazolyl)benzamide;
*N*-(3-Cyclobutyl-2,3,4,5-tetrahydro-1*H*-3-benzazepin-7-yl)-4-imidazo[1,2-a]pyridin-2-ylbenzamide;
*N*-(3-Cyclobutyl-2,3,4,5-tetrahydro-1*H*-3-benzazepin-7-yl)-1,2,5-oxadiazole-3-carboxamide;
*N*-(3-Cyclobutyl-2,3,4,5-tetrahydro-1*H*-3-benzazepin-7-yl)-3-isoxazolecarboxamide;
*N*-(3-Cyclobutyl-2,3,4,5-tetrahydro-1*H*-3-benzazepin-7-yl) cyclohexane carboxamide ;
*N*-(3-Cyclobutyl-2,3,4,5-tetrahydro-1*H*-3-benzazepin-7-yl)-2-cyclopentyl acetamide ;
*N*-(3-Cyclobutyl-2,3,4,5-tetrahydro-1*H*-3-benzazepin-7-yl)-3-methylbenzamide;
*N*-(3-Cyclobutyl-2,3,4,5-tetrahydro-1*H*-3-benzazepin-7-yl)-4-fluorobenzamide;
*N*-(3-Cyclobutyl-2,3,4,5-tetrahydro-1*H*-3-benzazepin-7-yl)-3-methyl-2-thiophenecarboxamide;
*N*-(3-Cyclobutyl-2,3,4,5-tetrahydro-1*H*-3-benzazepin-7-yl)bicyclo[4.2.0]octa-1,3,5-triene-7-carboxamide;
*N*-(3-Cyclobutyl-2,3,4,5-tetrahydro-1*H*-3- benzazepin-7-yl)-2-methylbenzamide;
*N*-(3-Cyclobutyl-2,3,4,5-tetrahydro-1*H*-3-benzazepin-7-yl)-4-methylbenzamide;
*N*-(3-Cyclobutyl-2,3,4,5-tetrahydro-1*H*-3-benzazepin-7-yl)-2-ethylbutanamide;
*N*-(3-Cyclobutyl-2,3,4,5-tetrahydro-1*H*-3-benzazepin-7-yl)-3-fluorobenzamide;
*N*-(3-Cyclobutyl-2,3,4,5-tetrahydro-1*H*-3-benzazepin-7-yl)-4-(5-pyrimidinyl)benzamide;
*N*-(3-Cyclobutyl-2,3,4,5-tetrahydro-1*H*-3-benzazepin-7-yl)-4-(methylsulfonyl)benzamide;
*N*-(3-Cyclobutyl-2,3,4,5-tetrahydro-1*H*-3-benzazepin-7-yl)-6-quinoxalinecarboxamide;
*N*-(3-Cyclobutyl-2,3,4,5-tetrahydro-1*H*-3-benzazepin-7-yl)-2-methyl-1,6-naphthyridine-3-carboxamide;
*N*-(3-Cyclobutyl-2,3,4,5-tetrahydro-1*H*-3-benzazepin-7-yl)-4-(1*H*-tetrazol-1-yl)benzamide; 4-(6-Cyano-3-pyridinyl)-*N*-(3-cyclobutyl-2,3,4,5-tetrahydro-1*H*-3-benzazepin-7-yl)benzamide;
*N*-(3-Cyclobutyl-2,3,4,5-tetrahydro-1*H*-3-benzazepin-7-yl)-2-(trifluoromethyl)-1,8-naphthyridine-3-carboxamide;
*N*-(3-Cyclobutyl-2,3,4,5-tetrahydro-1*H*-3-benzazepin-7-yl)-4-methylnicotinamide;
*N*-(3-Cyclobutyl-2,3,4,5-tetrahydro-1*H*-3-benzazepin-7-yl)-6-methylnicotinamide;
*N*-(3-Cyclobutyl-2,3,4,5-tetrahydro-1*H*-3-benzazepin-7-yl)nicotinamide;
*N*-(3-Cyclobutyl-2,3,4,5-tetrahydro-1*H*-3-benzazepin-7-yl)-4-(trifluoromethyl)nicotinamide;
*N*-(3-Cyclobutyl-2,3,4,5-tetrahydro-1*H*-3-benzazepin-7-yl)-6-(1*H*-pyrazol-1-yl)nicotinamide;
*N*-(3-Cyclobutyl-2,3,4,5-tetrahydro-1*H*-3-benzazepin-7-yl)-6-(trifluoromethyl)nicotinamide;
*N*-(3-Cyclobutyl-2,3,4,5-tetrahydro-1*H*-3-benzazepin-7-yl)-4-(5-methyl-1*H*-tetrazol-1 - yl)benzamide;
*N*-(3-Cyclobutyl-2,3,4,5-tetrahydro-1*H*-3-benzazepin-7-yl)-5-(3-pyridinyl)-1*H*-pyrazole-3-carboxamide;
*N*-(3-Cyclobutyl-2,3,4,5-tetrahydro-1*H*-3-benzazepin-7-yl)-6-(4-morpholinyl)-3-pyridinecarboxamide;
*N*-(3-Cyclobutyl-2,3,4,5-tetrahydro-1*H*-3-benzazepin-7-yl)-1*H*-1,2,3-benzotriazole-5-carboxamide;
*N*-(3-Cyclobutyl-2,3,4,5-tetrahydro-1*H-*3-benzazepin-7-yl)-4-(2-pyrazinyl)benzamide;
*N-*(3-Cyclobutyl-2,3,4,5-tetrahydro-1*H*-3-benzazepin-7-yl)-2-methyl-1,8-naphthyridine-3-carboxamide;
*N*-(3-Cyclobutyl-2,3,4,5-tetrahydro-1*H*-3-benzazepin-7-yl)-5-quinoxaline carboxamide;
*N*-(3-Cyclobutyl-2,3,4,5-tetrahydro-1*H*-3-benzazepin-7-yl)pyrazolo[1,5-a]pyrimidine-3-carboxamide;
*N*-(3-Cyclobutyl-2,3,4,5-tetrahydro-1*H*-3-benzazepin-7-yl)-5-pyrimidinecarboxamide;
*N*-(3-Cyclobutyl-2,3,4,5-tetrahydro-1*H*-3-benzazepin-7-yl)-4-pyridazinecarboxamide;
*N*-(3-Cyclobutyl-2,3,4,5-tetrahydro-1*H*-3-benzazepin-7-yl)-3-(5-pyrimidinyl) benzamide;
*N*-(3-Cyclobutyl-2,3,4,5-tetrahydro-1*H*-3-benzazepin-7-yl)-3-(2-pyrazinyl)benzamide;
*N*-(3-Cyclobutyl-2,3,4,5-tetrahydro-1*H*-3-benzazepin-7-yl)-3-(4-pyrimidinyl) benzamide;
*N*-(3-Cyclobutyl-2,3,4,5-tetrahydro-1*H*-3-benzazepin-7-yl)-4-(4-pyrimidinyl)benzamide;
N-(3-Cyclobutyl-2,3,4,5-tetrahydro-1H-3-benzazepin-7-yl)-5-methyl-3-isoxazolecarboxamide;
6-Cyano-N-(3-cyclobutyl-2,3,4,5-tetrahydro-1H-3-benzazepin-7-yl)-3-pyridinecarboxamide;
N-(3-Cyclobutyl-2,3,4,5-tetrahydro-1H-3-benzazepin-7-yl)-4-(1H-imidazol-1-yl)benzamide;
N-(3-Cyclobutyl-2,3,4,5-tetrahydro-1H-3-benzazepin-7-yl)-2-pyrazinecarboxamide;
N-(3-Cyclobutyl-2,3,4,5-tetrahydro-1H-3-benzazepin-7-yl)-2-methyl-3-pyridinecarboxamide;
N-(3-Cyclobutyl-2,3,4,5-tetrahydro-1H-3-benzazepin-7-yl)-3-methyl-2-pyridinecarboxamide;
N-(3-Cyclobutyl-2,3,4,5-tetrahydro-1H-3-benzazepin-7-yl)-3-thiophenecarboxamide;
N-(3-Cyclobutyl-2,3,4,5-tetrahydro-1H-3-benzazepin-7-yl)-3-furancarboxamide;
N-(3-Cyclobutyl-2,3,4,5-tetrahydro-1H-3-benzazepin-7-yl)-2-methyl-3-furancarboxamide;
N-(3-Cyclobutyl-2,3,4,5-tetrahydro-1H-3-benzazepin-7-yl)-2,5-dimethyl-3-furancarboxamide;
N-(3-Cyclobutyl-2,3,4,5-tetrahydro-1H-3-benzazepin-7-yl)-2-methyl-5-phenyl-3-furancarboxamide;
N-(3-Cyclobutyl-2,3,4,5-tetrahydro-1H-3-benzazepin-7-yl)-1,2,3-thiadiazole-4-carboxamide;
N-(3-Cyclobutyl-2,3,4,5-tetrahydro-1H-3-benzazepin-7-yl)-1,3-thiazole-4-carboxamide;
N-(3-Cyclobutyl-2,3,4,5-tetrahydro-1H-3-benzazepin-7-yl)-5-methyl-2-phenyl-2H-1,2,3-triazole-4-carboxamide;
N-(3-Cyclobutyl-2,3,4,5-tetrahydro-1H-3-benzazepin-7-yl)-1-methyl-1H-imidazole-4-carboxamide;
N-(3-Cyclobutyl-2,3,4,5-tetrahydro-1H-3-benzazepin-7-yl)-1-methyl-1H-1,2,3-triazole-4-carboxamide;
N-(3-Cyclobutyl-2,3,4,5-tetrahydro-1H-3-benzazepin-7-yl)-5-phenyl-1,3-oxazole-4-carboxamide;
N-(3-Cyclobutyl-2,3,4,5-tetrahydro-1H-3-benzazepin-7-yl)-2-phenyl-2H-1,2,3-triazole-4-carboxamide;
2-(2,1,3-Benzoxadiazol-5-yl)-N-(3-cyclobutyl-2,3,4,5-tetrahydro-1H-3-benzazepin-7-yl)-1,3-thiazole-4-carboxamide;
N-(3-Cyclobutyl-2,3,4,5-tetrahydro-1H-3-benzazepin-7-yl)-1,5-dimethyl-1H-pyrazole-3-carboxamide;
*N*-(3-Cyclobutyl-2,3,4,5-tetrahydro-1*H*-3-benzazepin-7-yl)-1,2,5-thiadiazole-3-carboxamide;
N-(3-Cyclobutyl-2,3,4,5-tetrahydro-1H-3-benzazepin-7-yl)-6-(1H-1,2,4-triazo)-1-yl)-3-pyridinecarboxamide;
N-(3-Cyclobutyl-2,3,4,5-tetrahydro-1H-3-benzazepin-7-yl)-6-phenyl-3-pyridinecarboxamide;
N-(3-Cyclobutyl-2,3,4,5-tetrahydro-1H-3-benzazepin-7-yl)-3,3'-bipyridine-5-carboxamide;
N-(3-Cyclobutyl-2,3,4,5-tetrahydro-1H-3-benzazepin-7-yl)-6-oxo-1,6-dihydro-3-pyridinecarboxamide;
N-(3-Cyclobutyl-2,3,4,5-tetrahydro-1H-3-benzazepin-7-yl)-3-methyl-5-isoxazolecarboxamide;
N-(3-Cyclobutyl-2,3,4,5-tetrahydro-1H-3-benzazepin-7-yl)-5-methyl-2-pyrazinecarboxamide;
N-(3-Cyclobutyl-2,3,4,5-tetrahydro-1H-3-benzazepin-7-yl)-6-oxo-1,6-dihydro-2-pyridinecarboxamide;
N-(3-Cyclobutyl-2,3,4,5-tetrahydro-1H-3-benzazepin-7-yl)-2,4-dimethyl-1,3-thiazole-5-carboxamide;
N-(3-Cyclobutyl-2,3,4,5-tetrahydro-1H-3-benzazepin-7-yl)-2-phenyl-1,3-thiazole-4-carboxamide;
*N*-(3-Cyclobutyl-2,3,4,5-tetrahydro-1*H*-3-benzazepin-7-yl)-4-(1,1-dioxido-2-isothiazolidinyl)benzamide;
*N*-(3-Cyclobutyl-2,3,4,5-tetrahydro-1*H*-3-benzazepin-7-yl)-4-(2-oxo-1-pyrrolidinyl)benzamide;
*N*-(3-Cyclobutyl-2,3,4,5-tetrahydro-1*H*-3-benzazepin-7-yl)-4-(2-oxo-1-imidazolidinyl)benzamide;
*N*-(3-Cyclobutyl-2,3,4,5-tetrahydro-1*H*-3-benzazepin-7-yl)-4-(1*H*-pyrazol-1-yl)benzamide;
*N*-(3-Cyclobutyl-2,3,4,5-tetrahydro-1*H*-3-benzazepin-7-yl)-4-(1*H*-1,2,4-triazol-1-yl)benzamide;
*N*-(3-Cyclobutyl-2,3,4,5-tetrahydro-1*H*-3-benzazepin-7-yl)-3-(2-oxo-1-pyrrolidinyl)benzamide;
*N*-(3-Cyclobutyl-2,3,4,5-tetrahydro-1*H*-3-benzazepin-7-yl)-3-(1,1-dioxido-2-isothiazolidinyl)benzamide;
*N*-(3-Cyclobutyl-2,3,4,5-tetrahydro-1*H*-3-benzazepin-7-yl)-3-(2-oxo-1-imidazolidinyl)benzamide;
*N*-(3-Cyclobutyl-2,3,4,5-tetrahydro-1*H*-3-benzazepin-7-yl)-3-(1*H*-pyrazol-1-yl)benzamide;
*N*-(3-Cyclobutyl-2,3,4,5-tetrahydro-1*H*-3-benzazepin-7-yl)-3-(1*H-*1,2,4-triazol-1-yl)benzamide;
*N*-(3-Cyclobutyl-2,3,4,5-tetrahydro-1*H*-3-benzazepin-7-yl)-*N*'-methyl-2,5-pyrazinedicarboxamide;
*N*-(3-Cyclobutyl-2,3,4,5-tetrahydro-1*H*-3-benzazepin-7-yl)-4-(4-pyridinyl)benzamide;
6-(4-Cyanophenyl)-*N*-(3-cyclobutyl-2,3,4,5-tetrahydro-1*H*-3-benzazepin-7-yl)-3-pyridinecarboxamide;
*N*-(3-Cyclobutyl-2,3,4,5-tetrahydro-1*H*-3-benzazepin-7-yl)-2,3'-bipyridine-5-carboxamide;
*N*-(3-Cyclobutyl-2,3,4,5-tetrahydro-1*H*-3-benzazepin-7-yl)-6-(5-pyrimidinyl)-3-pyridinecarboxamide;
6-(3-Cyanophenyl)-*N*-(3-cyclobutyl-2,3,4,5-tetrahydro-1*H*-3-benzazepin-7-yl)-3-pyridinecarboxamide;
N-(3-Cyclobutyl-2,3,4,5-tetrahydro-1H-3-benzazepin-7-yl)-5-(4-fluorophenyl)-2-pyridinecarboxamide;
5-(4-Cyanophenyl)-*N*-(3-cyclobutyl-2,3,4,5-tetrahydro-1*H*-3-benzazepin-7-yl)-2-pyridinecarboxamide;
6'-Cyano-*N*-(3-cyclobutyl-2,3,4,5-tetrahydro-1*H*-3-benzazepin-7-yl)-3,3'-bipyridine-6-carboxamide;
N-(3-Cyclobutyl-2,3,4,5-tetrahydro-1H-3-benzazepin-7-yl)-5-(5-pyrimidinyl)-2-pyridinecarboxamide;
*N*-(3-Cyclobutyl-2,3,4,5-tetrahydro-1*H*-3-benzazepin-7-yl)-5-(2-pyrazinyl)-2-pyridinecarboxamide;
*N*-(3-Cyclobutyl-2,3,4,5-tetrahydro-1*H*-3-benzazepin-7-yl)-2,3'-bipyridine-6'-carboxamide;
*N*-(3-Cyclobutyl-2,3,4,5-tetrahydro-1*H*-3-benzazepin-7-yl)-5-(1*H*-pyrazol-1-yl)-2-pyridinecarboxamide;
*N*-(3-Cyclobutyl-2,3,4,5-tetrahydro-1*H*-3-benzazepin-7-yl)-5-(1*H*-imidazol-1 -yl)-2-pyridinecarboxamide;
*N*-(3-Cyclobutyl-2,3,4,5-tetrahydro-1*H*-3-benzazepin-7-yl)-5-(1*H*-1,2,4-triazol-1-yl)-2-pyridinecarboxamide;
*N*-(3-Cyclobutyl-2,3,4,5-tetrahydro-1*H*-3-benzazepin-7-yl)-5-(4-morpholinyl)-2-pyrazinecarboxamide;
*N*-(3-Cyclobutyl-2,3,4,5-tetrahydro-1*H*-3-benzazepin-7-yl)-5-(1-pyrrolidinyl)-2-pyrazinecarboxamide;
*N*-(3-Cyclobutyl-2,3,4,5-tetrahydro-1*H*-3-benzazepin-7-yl)-5-(1-piperidinyl)-2-pyrazinecarboxamide;
*N-*(3-Cyclobutyl-2,3,4,5-tetrahydro-1*H*-3-benzazepin-7-yl)-5-(tetrahydro-2*H*-pyran-4-yloxy)-2-pyrazinecarboxamide;
*N*-(3-Cyclobutyl-2,3,4,5-tetrahydro-1*H*-3-benzazepin-7-yl)-5-(phenyloxy)-2-pyrazinecarboxamide;
5-(4-Cyanophenyl)-*N*-(3-cyclobutyl-2,3,4,5-tetrahydro-1*H*-3-benzazepin-7-yl)-2-pyrazinecarboxamide;
5-(3-Cyanophenyl)-*N*-(3-cyclobutyl-2,3,4,5-tetrahydro-1*H*-3-benzazepin-7-yl)-2-pyrazinecarboxamide;
N-(3-Cyclobutyl-2,3,4,5-tetrahydro-1H-3-benzazepin-7-yl)-4-pyridinecarboxamide;
N-(3-Cyclobutyl-2,3,4,5-tetrahydro-1H-3-benzazepin-7-yl)-4-biphenylcarboxamide;
3-Cyano-N-(3-cyclobutyl-2,3,4,5-tetrahydro-1H-3-benzazepin-7-yl)benzamide;
N-(3-Cyclobutyl-2,3,4,5-tetrahydro-1H-3-benzazepin-7-yl)-1,3-dimethyl-1H-pyrazole-5-carboxamide;
N-(3-Cyclobutyl-2,3,4,5-tetrahydro-1H-3-benzazepin-7-yl)-3,5-dimethyl-4-isoxazolecarboxamide;
5-Bromo-N-(3-cyclobutyl-2,3,4,5-tetrahydro-1H-3-benzazepin-7-yl)-3-pyridinecarboxamide;
N-(3-Cyclobutyl-2,3,4,5-tetrahydro-1H-3-benzazepin-7-yl)-5-(1H-imidazol-1-yl)-3. pyridinecarboxamide;
*N*-(3-Cyclobutyl-2,3,4,5-tetrahydro-1*H*-3-benzazepin-7-yl)-*N*-methyl-4-(2-pyrazinyl)benzamide;
N-(3-Cyclobutyl-2,3,4,5-tetrahydro-1H-3-benzazepin-7-yl)-N-methyl-4-(2-pyridinyl)benzamide;
*N*-(3-Cyclobutyl-2,3,4,5-tetrahydro-1*H*-3-benzazepin-7-yl)-*N*-methyl-4-(5-pyrimidinyl)benzamide;
*N*-(3-Cyclobutyl-2,3,4,5-tetrahydro-1*H*-3-benzazepin-7-yl)-*N*,6-dimethyl-3-pyridinecarboxamide;
*N*-(3-Cyclobutyl-2,3,4,5-tetrahydro-1*H*-3-benzazepin-7-yl)-*N*'-phenylurea;
*N*-(4-Cyanophenyl)-*N*'-(3-cyclobutyl-2,3,4,5-tetrahydro-1*H*-3-benzazepin-7-yl)urea;
*N*-1,3-Benzodioxol-5-yl-*N*'-(3-cyclobutyl-2,3,4,5-tetrahydro-1*H*-3-benzazepin-7-yl)urea;
*N*-(3-Cyclobutyl-2,3,4,5-tetrahydro-1*H*-3-benzazepin-7-yl)-*N*'-cyclohexylurea;
*N*-(3-Cyclobutyl-2,3,4,5-tetrahydro-1*H*-3-benzazepin-7-yl)-*N*'-(1-methylethyl)urea;
*N*-(3-Cyclobutyl-2,3,4,5-tetrahydro-1*H*-3-benzazepin-7-yl)-*N*'-[2-(methyloxy)phenyl]urea;
*N*-(3-Cyclobutyl-2,3,4,5-tetrahydro-1*H*-3-benzazepin-7-yl)-*N*-8-quinolinylurea;
*N*-(3-Cyclobutyl-2,3,4,5-tetrahydro-1*H*-3-benzazepin-7-yl)-*N*-(2,2-difluoro-1,3-benzodioxol-4-yl)urea;
*N*-(3-Cyclobutyl-2,3,4,5-tetrahydro-1*H*-3-benzazepin-7-yl)-*N*-3-pyridinylurea;
*N*-(3-Cyclobutyl-2,3,4,5-tetrahydro-1*H*-3-benzazepin-7-yl)-*N*'-4-pyridinylurea;
*N*-(3-Cyclobutyl-2,3,4,5-tetrahydro-1*H*-3-benzazepin-7-yl)-*N*-[2-(ethyloxy) phenyl]urea;
*N*-(3-Cyclobutyl-2,3,4,5-tetrahydro-1*H*-3-benzazepin-7-yl)-*N*'-{2-[(trifluoromethyl)oxy]phenyl}urea;
*N*-(3-Cyclobutyl-2,3,4,5-tetrahydro-1*H*-3-benzazepin-7-yl)-*N*'-[2-methyl-6-(methyloxy)phenyl]urea;
*N*-(3-Cyanophenyl)-*N*'-(3-cyclobutyl-2,3,4,5-tetrahydro-1*H*-3-benzazepin-7-yl)urea;
*N*-[5-Chloro-2-(methyloxy)phenyl]-*N*'-(3-cyclobutyl-2,3,4,5-tetrahydro-1*H*-3-benzazepin-7-yl)urea;
*N*-(3-Cyclobutyl-2,3,4,5-tetrahydro-1*H*-3-benzazepin-7-yl)-4-pyrazin-2-ylpiperazine-1-carboxamide;
4-(5-Cyanopyridin-2-yl)-*N*-(3-cyclobutyl-2,3,4,5-tetrahydro-1*H*-3-benzazepin-7-y))piperazine-1-carboxamide;
5-Chloro-*N*-(3-cyclobutyl-2,3,4,5-tetrahydro-1*H*-3-benzazepin-7-yl)indoline-1-carboxamide;
*N*-(3-Cyclobutyl-2,3,4,5-tetrahydro-1*H*-3-benzazepin-7-yl)-2,3-dihydro-1*H*-indole-1-carboxamide;
*N*-(3-Cyclobutyl-2,3,4,5-tetrahydro-1*H*-3-benzazepin-7-yl)-3,4-dihydro-1(2*H*)-quinolinecarboxamide;
*N*-(3-Cyclobutyl-2,3,4,5-tetrahydro-1*H*-3-benzazepin-7-yl)-1,3-dihydro-2*H*-isoindole-2-carboxamide;
*N*-(3-Cyclobutyl-2,3,4,5-tetrahydro-1*H*-3-benzazepin-7-yl)-3,4-dihydro-2(1*H*)-isoquinolinecarboxamide;
*N*-(3-Cyclobutyl-2,3,4,5-tetrahydro-1*H*-3-benzazepin-7-yl)-4-phenyl-1-piperidinecarboxamide;
4-[(4-Cyanophenyl)oxy]-*N*-(3-cyclobutyl-2,3,4,5-tetrahydro-1*H*-3-benzazepin-7-yl)-1-piperidinecarboxamide;
*N*-(3-Cyclobutyl-2,3,4,5-tetrahydro-1*H*-3-benzazepin-7-yl)-4-(2-pyridinyl)-1-piperidinecarboxamide;
*N*-(3-Cyclobutyl-2,3,4,5-tetrahydro-1*H*-3-benzazepin-7-yl)-4-(4-pyridinyl)-1-piperidinecarboxamide;
*N*-(3-Cyclobutyl-2,3,4,5-tetrahydro-1*H*-3-benzazepin-7-yl)-4-phenyl-1-piperazinecarboxamide;
4-(4-Cyanophenyl)-*N*-(3-cyclobutyl-2,3,4,5-tetrahydro-1*H*-3-benzazepin-7-yl)-1-piperazinecarboxamide;
*N*-(3-Cyclobutyl-2,3,4,5-tetrahydro-1*H*-3-benzazepin-7-yl)-1-piperidinecarboxamide;
*N*-(3-Cyclobutyl-2,3,4,5-tetrahydro-1*H*-3-benzazepin-7-yl)-1-pyrrolidinecarboxamide;
*N*-(3-Cyclobutyl-2,3,4,5-tetrahydro-1*H*-3-benzazepin-7-yl)-5-fluoro-2,3-dihydro-1*H*-indole-1-carboxamide;
*N*-(3-Cyclobutyl-2,3,4,5-tetrahydro-1*H*-3-benzazepin-7-yl)-4-(4-fluorophenyl)-1-piperidinecarboxamide;
*N*-(3-Cyclobutyl-2,3,4,5-tetrahydro-1*H*-3-benzazepin-7-yl)-2-phenyl-1-pyrrolidinecarboxamide;
5-Cyano-*N*-(3-cyclobutyl-2,3,4,5-tetrahydro-1*H*-3-benzazepin-7-yl)-2,3-dihydro-1*H*-indole-1-carboxamide;
4-(4-Cyanophenyl)-*N*-(3-cyclobutyl-2,3,4,5-tetrahydro-1*H*-3-benzazepin-7-yl)-1-piperidinecarboxamide;
*N*-(3-Cyclobutyl-2,3,4,5-tetrahydro-1*H*-3-benzazepin-7-yl)-1,2,4,5-tetrahydro-3*H*-3-benzazepine-3-carboxamide;
4-(3-Cyano-2-pyrazinyl)-*N*-(3-cyclobutyl-2,3,4,5-tetrahydro-1*H*-3-benzazepin-7-yl)-1-piperazinecarboxamide;
*N*-(3-Cyclobutyl-2,3,4,5-tetrahydro-1*H*-3-benzazepin-7-yl)-2,3-dihydro-4*H*-1,4-benzoxazine-4-carboxamide;
*N*-(3-Cyclobutyl-2,3,4,5-tetrahydro-1*H*-3-benzazepin-7-yl)-4-{[4-(methyloxy)phenyl]oxy}-1-piperidinecarboxamide;
*N*-(3-Cyclobutyl-2,3,4,5-tetrahydro-1*H*-3-benzazepin-7-yl)-4-[(4-fluorophenyl)oxy]-1-piperidinecarboxamide;
*N*-(3-Cyclobutyl-2,3,4,5-tetrahydro-1*H*-3-benzazepin-7-yl)-4-(phenyloxy)-1-piperidinecarboxamide;
*N*-(3-Cyclobutyl-2,3,4,5-tetrahydro-1*H*-3-benzazepin-7-yl)-4-(2-pyridinyloxy)-1-piperidinecarboxamide;
*N*-(3-Cyclobutyl-2,3,4,5-tetrahydro-1*H*-3-benzazepin-7-yl)-4-(3-pyridinyloxy)-1-piperidinecarboxamide;
7-Cyano-*N*-(3-cyclobutyl-2,3,4,5-tetrahydro-1*H*-3-benzazepin-7-yl)-2,3-dihydro-4*H*-1,4-benzoxazine-4-carboxamide;
*N*-(4-Cyanophenyl)-*N'*-(3-cyclobutyl-2,3,4,5-tetrahydro-1*H*-3-benzazepin-7-yl)-*N-*methylurea;
*N*-(3-Cyclobutyl-2,3,4,5-tetrahydro-1*H*-3-benzazepin-7-yl)-2,3-dihydro-1*H*-pyrrolo[2,3-*b*]pyridine-1-carboxamide;
5-Cyano-*N*-(3-cyclobutyl-2,3,4,5-tetrahydro-1*H*-3-benzazepin-7-yl)-1,3-dihydro-2*H-*isoindole-2-carboxamide;
1-{6-[(3-Cyclobutyl-2,3,4,5-tetrahydro-1*H*-3-benzazepin-7-yl)amino]-3-pyridinyl}-2-pyrrolidinone;
*N*-(3-Cyclobutyl-2,3,4,5-tetrahydro-1*H*-3-benzazepin-7-yl)-1,3-benzodioxole-5-carboxamide trifluoroacetate;
*N*-(3-Cyclobutyl-2,3,4,5-tetrahydro-1*H*-3-benzazepin-7-yl)-2-methyl-1*H*-benzimidazole-5-carboxamide trifluoroacetate;
*N*-(3-Cyclobutyl-2,3,4,5-tetrahydro-1*H*-3-benzazepin-7-yl)-2-quinoxalinecarboxamide trifluoroacetate;
*N*-(3-Cyclobutyl-2,3,4,5-tetrahydro-1*H*-3-benzazepin-7-yl)-2-(methyloxy)benzamide trifluoroacetate;
*N*-(3-Cyclobutyl-2,3,4,5-tetrahydro-1*H*-3-benzazepin-7-yl)pyrazolo[1,5-a]pyridine-3-carboxamide trifluoroacetate;
*N*-(3-Cyclobutyl-2,3,4,5-tetrahydro-1*H*-3-benzazepin-7-yl)-5-methyl-1-[4-(methyloxy)phenyl]-1*H*-pyrazole-4-carboxamide trifluoroacetate;
*N*-(3-Cyclobutyl-2,3,4,5-tetrahydro-1*H*-3-benzazepin-7-yl)-3-(methyloxy)benzamide trifluoroacetate;
*N*-(3-Cyclobutyl-2,3,4,5-tetrahydro-1*H*-3-benzazepin-7-yl)-1-benzothiophene-2-carboxamide trifluoroacetate;
*N*-(3-Cyclobutyl-2,3,4,5-tetrahydro-1*H*-3-benzazepin-7-yl)-2-naphthalenecarboxamide trifluoroacetate;
1-(4-Chlorophenyl)-*N*-(3-cyclobutyl-2,3,4,5-tetrahydro-1*H*-3-benzazepin-7-yl)-5-(trifluoromethyl)-1 *H*-pyrazole-4-carboxamide trifluoroacetate;
*N*-(3-Cyclobutyl-2,3,4,5-tetrahydro-1*H*-3-benzazepin-7-yl)-1-(1-methylethyl)-1*H*-1,2,3-benzotriazole-5-carboxamide trifluoroacetate;
*N*-(3-Cyclobutyl-2,3,4,5-tetrahydro-1*H*-3-benzazepin-7-yl)-1-(1-methylethyl)-2-(trifluoromethyl)-1*H*-benzimidazole-5-carboxamide trifluoroacetate;
*N*-(3-Cyclobutyl-2,3,4,5-tetrahydro-1*H*-3-benzazepin-7-yl)-2,1-benzisoxazole-3-carboxamide trifluoroacetate;
*N*-(3-Cyclobutyl-2,3,4,5-tetrahydro-1*H*-3-benzazepin-7-yl)-1-methyl-3-propyl,1*H*-pyrazole-5-carboxamide trifluoroacetate;
*N*-(3-Cyclobutyl-2,3,4,5-tetrahydro-1*H*-3-benzazepin-7-yl)-1,5-dimethyl-3-oxo-2-phenyl-2,3-dihydro-1*H*-pyrazole-4-carboxamide trifluoroacetate;
*N*-(3-Cyclobutyl-2,3,4,5-tetrahydro-1*H*-3-benzazepin-7-yl)-2-methyl-2*H*-1,2,3-triazole-4-carboxamide trifluoroacetate;
*N*-(3-Cyclobutyl-2,3,4,5-tetrahydro-1*H*-3-benzazepin-7-yl)-1-benzothiophene-3-carboxamide trifluoroacetate;
*N*-(3-Cyclobutyl-2,3,4,5-tetrahydro-1*H*-3-benzazepin-7-yl)-1-phenyl-1*H*-1,2,3-triazole-5-carboxamide trifluoroacetate;
*N*-(3-Cyclobutyl-2,3,4,5-tetrahydro-1*H*-3-benzazepin-7-yl)-3-methyl-5-[4-(methyloxy)phenyl]-1*H*-pyrazole-4-carboxamide trifluoroacetate;
*N*-(3-Cyclobutyl-2,3,4,5-tetrahydro-1*H*-3-benzazepin-7-yl)-5-methyl-1-phenyl-1*H*-pyrazole-3-carboxamide trifluoroacetate;
*N*-(3-Cyclobutyl-2,3,4,5-tetrahydro-1*H*-3-benzazepin-7-yl)-3-[3-(methyloxy)phenyl]-1-phenyl-1*H*-pyrazole-4-carboxamide trifluoroacetate;
*N*-(3-Cyclobutyl-2,3,4,5-tetrahydro-1*H*-3-benzazepin-7-yl)-3-(1,1-dimethylethyl)-1-methyl-1*H*-pyrazole-5-carboxamide trifluoroacetate;
*N*-(3-Cyclobutyl-2,3,4,5-tetrahydro-1*H*-3-benzazepin-7-yl)-5,7-dimethylpyrazolo[1,5-a]pyrimidine-2-carboxamide trifluoroacetate;
*N*-(3-Cyclobutyl-2,3,4,5-tetrahydro-1*H*-3-benzazepin-7-yl)-3-methyl-1-phenyl-1*H*-pyrazole-5-carboxamide trifluoroacetate;
*N*-(3-Cyclobutyl-2,3,4,5-tetrahydro-1*H*-3-benzazepin-7-yl)-1-methyl-1*H*-1,2,3-benzotriazole-5-carboxamide trifluoroacetate;
*N-*(3-Cyclobutyl-2,3,4,5-tetrahydro-1*H*-3-benzazepin-7-yl)-5-methyl-1-[(4-methylphenyl)methyl]-1*H*-pyrazole-3-carboxamide trifluoroacetate;
*N*-(3-Cyclobutyl-2,3,4,5-tetrahydro-1*H*-3-benzazepin-7-yl)-1-ethyl-3-(2-thienyl)-1*H*-pyrazole-5-carboxamide trifluoroacetate;
*N*-(3-Cyclobutyl-2,3,4,5-tetrahydro-1*H*-3-benzazepin-7-yl)-1-ethyl-5-methyl-1*H*-pyrazole-3-carboxamide trifluoroacetate;
*N*-(3-Cyclobutyl-2,3,4,5-tetrahydro-1*H*-3-benzazepin-7-yl)-4-[(4-fluorophenyl)oxy]-1*H*-1,2,3-triazole-5-carboxamide trifluoroacetate;
*N*-(3-Cyclobutyl-2,3,4,5-tetrahydro-1*H*-3-benzazepin-7-yl)-4-ethyl-1-phenyl-1*H*-1,2,3-triazole-5-carboxamide trifluoroacetate;
*N*-(3-Cyclobutyl-2,3,4,5-tetrahydro-1*H*-3-benzazepin-7-yl)-5-ethyl-1-phenyl-1*H*-1,2,3-triazole-4-carboxamide trifluoroacetate;
*N*-(3-Cyclobutyl-2,3,4,5-tetrahydro-1*H*-3-benzazepin-7-yl)-2-methyl-5-phenyl-2*H*-1,2,3-triazole-4-carboxamide trifluoroacetate;
*N*-(3-Cyclobutyl-2,3,4,5-tetrahydro-1*H*-3-benzazepin-7-yl)-5-methyl-1-phenyl-1*H*-1,2,3-triazole-4-carboxamide trifluoroacetate;
*N*-(3-Cyclobutyl-2,3,4,5-tetrahydro-1*H*-3-benzazepin-7-yl)-2*H*-chromene-3-carboxamide trifluoroacetate;
*N*-(3-Cyclobutyl-2,3,4,5-tetrahydro-1*H*-3-benzazepin-7-yl)-2-methyl-4,5,6,7-tetrahydro-2*H-*indazole-3-carboxamide trifluoroacetate;
4-[3,4-bis(Methyloxy)phenyl]-*N*-(3-cyclobutyl-2,3,4,5-tetrahydro-1*H*-3-benzazepin-7-yl)-1*H-*1,2,3-triazole-5-carboxamide trifluoroacetate;
*N*-(3-Cyclobutyl-2,3,4,5-tetrahydro-1*H*-3-benzazepin-7-yl)-1-(2,2,2-trifluoroethyl)-1*H*-1,2,3-triazole-4-carboxamide trifluoroacetate;
*N*-(3-Cyclobutyl-2,3,4,5-tetrahydro-1*H*-3-benzazepin-7-yl)-3,5-dimethyl-1-phenyl-1*H-*pyrazole-4-carboxamide trifluoroacetate;
*N*-(3-Cyclobutyl-2,3,4,5-tetrahydro-1*H*-3-benzazepin-7-yl)-1-methyl-1*H*-pyrazole-4-carboxamide trifluoroacetate;
*N*-(3-Cyclobutyl-2,3,4,5-tetrahydro-1*H*-3-benzazepin-7-yl)-1-methyl-3-(trifluoromethyl)-1*H-*pyrazole-4-carboxamide trifluoroacetate;
1-(2-Chlorophenyl)-*N*-(3-cyclobutyl-2,3,4,5-tetrahydro-1*H*-3-benzazepin-7-yl)-5-propyl-1*H-*pyrazole-4-carboxamide trifluoroacetate;
1-(4-Cyanophenyl)-*N*-(3-cyclobutyl-2,3,4,5-tetrahydro-1*H*-3-benzazepin-7-yl)-3-(trifluoromethyl)-1*H*-pyrazole-4-carboxamide trifluoroacetate;
1-(4-Chlorophenyl)-*N*-(3-cyclobutyl-2,3,4,5-tetrahydro-1*H*-3-benzazepin-7-yl)-5-propyl-1*H-*pyrazole-4-carboxamide trifluoroacetate;
*N*-(3-Cyclobutyl-2,3,4,5-tetrahydro-1*H*-3-benzazepin-7-yl)-3,4-dihydro-2*H*-chromene-3-carboxamide trifluoroacetate;
*N*-(3-Cyclobutyl-2,3,4,5-tetrahydro-1*H*-3-benzazepin-7-yl)-1,3,5-trimethyl-1*H*-pyrazole-4-carboxamide trifluoroacetate;
*N*-(3-Cyclobutyl-2,3,4,5-tetrahydro-1*H*-3-benzazepin-7-yl)-1-(4-fluorophenyl)-3,5-dimethyl-1*H*-pyrazole-4-carboxamide trifluoroacetate;
*N*-(3-Cyclobutyl-2,3,4,5-tetrahydro-1*H*-3-benzazepin-7-yl)-1-benzofuran-5-carboxamide trifluoroacetate;
*N*-(3-Cyclobutyl-2,3,4,5-tetrahydro-1*H*-3-benzazepin-7-yl)-1*H*-indole-3-carboxamide trifluoroacetate;
*N*-(3-Cyclobutyl-2,3,4,5-tetrahydro-1*H*-3-benzazepin-7-yl)-1*H*-indazole-3-carboxamide trifluoroacetate;
*N*-(3-Cyclobutyl-2,3,4,5-tetrahydro-1*H*-3-benzazepin-7-yl)-2,3-dihydro-1-benzofuran-2-carboxamide trifluoroacetate;
*N*-(3-Cyclobutyl-2,3,4,5-tetrahydro-1*H*-3-benzazepin-7-yl)-1*H*-indole-5-carboxamide trifluoroacetate;
*N*-(3-Cyclobutyl-2,3,4,5-tetrahydro-1*H*-3-benzazepin-7-yl)-2,3-dihydro-1-benzofuran-5-carboxamide trifluoroacetate;
*N*-(3-Cyclobutyl-2,3,4,5-tetrahydro-1*H*-3-benzazepin-7-yl)-1,3-benzothiazole-6-carboxamide trifluoroacetate;
*N*-(3-Cyclobutyl-2,3,4,5-tetrahydro-1*H*-3-benzazepin-7-yl)-1*H*-indole-6-carboxamide trifluoroacetate;
*N*-(3-Cyclobutyl-2,3,4,5-tetrahydro-1*H*-3-benzazepin-7-yl)-1,2,3-benzothiadiazole-5-carboxamide trifluoroacetate;
*N*-(3-Cyclobutyl-2,3,4,5-tetrahydro-1*H*-3-benzazepin-7-yl)-2,7-dimethylpyrazolo[1,5-a]pyrimidine-6-carboxamide trifluoroacetate;
*N*-(3-Cyclobutyl-2,3,4,5-tetrahydro-1*H*-3-benzazepin-7-yl)-1-naphthalenecarboxamide trifluoroacetate;
*N*-(3-Cyclobutyl-2,3,4,5-tetrahydro-1*H*-3-benzazepin-7-yl)-3-quinolinecarboxamide trifluoroacetate;
*N*-(3-Cyclobutyl-2,3,4,5-tetrahydro-1*H*-3-benzazepin-7-yl)-4-(methyloxy)-2-quinolinecarboxamide trifluoroacetate;
*N*-(3-Cyclobutyl-2,3,4,5-tetrahydro-1*H*-3-benzazepin-7-yl)-2,1,3-benzothiadiazole-5-carboxamide trifluoroacetate;
*N*-(3-Cyclobutyl-2,3,4,5-tetrahydro-1*H*-3-benzazepin-7-yl)-2,3-dihydro-1,4-benzodioxin-2-carboxamide trifluoroacetate;
*N*-(3-Cyclobutyl-2,3,4,5-tetrahydro-1*H*-3-benzazepin-7-yl)-6-(methyloxy)-2-naphthalenecarboxamide trifluoroacetate;
*N*-(3-Cyclobutyl-2,3,4,5-tetrahydro-1*H*-3-benzazepin-7-yl)-3-isoquinolinecarboxamide trifluoroacetate;
*N*-(3-Cyclobutyl-2,3,4,5-tetrahydro-1*H*-3-benzazepin-7-yl)-1-methyl-1*H*-indole-2-carboxamide trifluoroacetate;
*N*-(3-Cyclobutyl-2,3,4,5-tetrahydro-1*H*-3-benzazepin-7-yl)-1-benzofuran-2-carboxamide trifluoroacetate;
*N*-(3-Cyclobutyl-2,3,4,5-tetrahydro-1*H*-3-benzazepin-7-yl)-4-(methyloxy)benzamide trifluoroacetate;
*N*-(3-Cyclobutyl-2,3,4,5-tetrahydro-1*H*-3-benzazepin-7-yl)-1,3-diphenyl-1*H*-pyrazole-4-carboxamide trifluoroacetate;
*N*-(3-Cyclobutyl-2,3,4,5-tetrahydro-1*H*-3-benzazepin-7-yl)-2-quinolinecarboxamide trifluoroacetate;
*N*-(3-Cyclobutyl-2,3,4,5-tetrahydro-1*H*-3-benzazepin-7-yl)-2,3-dihydro-1,4-benzodioxin-6-carboxamide trifluoroacetate;
*N*-(3-Cyclobutyl-2,3,4,5-tetrahydro-1*H*+3-benzazepin-7-yl)-4-(methylsulfonyl)benzamide trifluoroacetate;
*N*-(3-Cyclobutyl-2,3,4,5-tetrahydro-1*H*-3-benzazepin-7-yl)-2-(phenyloxy)benzamide trifluoroacetate;
*N*-(3-Cyclobutyl-2,3,4,5-tetrahydro-1*H*-3-benzazepin-7-yl)-3-(phenyloxy)benzamide trifluoroacetate;
*N*-(3-Cyclobutyl-2,3,4,5-tetrahydro-1*H*-3-benzazepin-7-yl)-4-(phenyloxy)benzamide trifluoroacetate;
*N*-(3-Cyclobutyl-2,3,4,5-tetrahydro-1*H*-3-benzazepin-7-yl)-2-(trifluoromethyl)benzamide trifluoroacetate;
*N*-(3-Cyclobutyl-2,3,4,5-tetrahydro-1*H*-3-benzazepin-7-yl)-3-(trifluoromethyl)benzamide trifluoroacetate;
*N*-(3-Cyclobutyl-2,3,4,5-tetrahydro-1*H*-3-benzazepin-7-yl)-4-(trifluoromethyl)benzamide trifluoroacetate;
*N*-(3-Cyclobutyl-2,3,4,5-tetrahydro-1*H*-3-benzazepin-7-yl)-2-biphenylcarboxamide trifluoroacetate;
(2*E*)-*N*-(3-Cyclobutyl-2,3,4,5-tetrahydro-1*H*-3-benzazepin-7-yl)-3-phenyl-2-propenamide trifluoroacetate;
*N-*(3-Cyclobutyl-2,3,4,5-tetrahydro-1*H*-3-benzazepin-7-yl)-1*H*-benzimidazole-2-carboxamide trifluoroacetate;
(2*E*)-*N*-(3-Cyclobutyl-2,3,4,5-tetrahydro-1*H*-3-benzazepin-7-yl)-3-[2-(methyloxy)phenyl]-2-propenamide trifluoroacetate;
(2*E*)-*N*-(3-Cyclobutyl-2,3,4,5-tetrahydro-1*H*-3-benzazepin-7-yl)-3-[3-(methyloxy)phenyl]-2-propenamide trifluoroacetate;
(2*E*)-*N*-(3-Cyclobutyl-2,3,4,5-tetrahydro-1*H*-3-benzazepin-7-yl)-3-[4-(methyloxy)phenyl]-2-propenamide trifluoroacetate;
(2*E*)-*N*-(3-Cyclobutyl-2,3,4,5-tetrahydro-1*H*-3-benzazepin-7-yl)-3-(2-fluorophenyl)-2-propenamide trifluoroacetate;
(2*E*)-*N*-(3-Cyclobutyl-2,3,4,5-tetrahydro-1*H*-3-benzazepin-7-yl)-3-(3-fluorophenyl)-2-propenamide trifluoroacetate;
(2*E*)-N-(3-Cyclobutyl-2,3,4,5-tetrahydro-1*H*-3-benzazepin-7-yl)-3-(4-fluorophenyl)-2-propenamide trifluoroacetate;
3,5-Dichloro-*N*-(3-cyclobutyl-2,3,4,5-tetrahydro-1*H*-3-benzazepin-7-yl)benzamide trifluoroacetate;
*N*-(3-Cyclobutyl-2,3,4,5-tetrahydro-1*H*-3-benzazepin-7-yl)-1-ethyl-3-methyl-1*H*-pyrazole-5-carboxamide trifluoroacetate;
*N*-(3-Cyclobutyl-2,3,4,5-tetrahydro-1*H*-3-benzazepin-7-yl)-5-(2-furanyl)-1*H*-pyrazole-3-carboxamide trifluoroacetate;
*N*-(3-Cyclobutyl-2,3,4,5-tetrahydro-1*H*-3-benzazepin-7-yl)-1-(3,4-dichlorophenyl)-3,5-dimethyl-1 *H*-pyrazole-4-carboxamide trifluoroacetate;
*N*-(3-Cyclobutyl-2,3,4,5-tetrahydro-1*H*-3-benzazepin-7-yl)-3-(methyloxy)-2-naphthalenecarboxamide trifluoroacetate;
*N*-(3-Cyclobutyl-2,3,4,5-tetrahydro-1*H*-3-benzazepin-7-yl)-1-(4-fluorophenyl)-5-phenyl-1*H-*pyrazole-3-carboxamide;
*N*-(3-Cyclobutyl-2,3,4,5-tetrahydro-1*H*-3-benzazepin-7-yl)-1,5-diphenyl-1*H*-pyrazole-4-carboxamide;
*N*-(3-Cyclobutyl-2,3,4,5-tetrahydro-1*H-*3-benzazepin-7-yl)-5-methyl-2-(4-methylphenyl)-2*H-*1,2,3-triazole-4-carboxamide;
*N*-(3-Cyclobutyl-2,3,4,5-tetrahydro-1*H*-3-benzazepin-7-yl)-1-phenyl-5-(1*H*-pyrrol-1-yl)-1*H-*pyrazole-4-carboxamide;
*N*-(3-Cyclobutyl-2,3,4,5-tetrahydro-1*H*-3-benzazepin-7-yl)-1-(4-fluorophenyl)-5-methyl-1*H-*pyrazole-4-carboxamide;
*N*-(3-Cyclobutyl-2,3,4,5-tetrahydro-1*H*-3-benzazepin-7-yl)-1-phenyl-5-(trifluoromethyl)-1*H-*pyrazole-4-carboxamide;
*N*-(3-Cyclobutyl-2,3,4,5-tetrahydro-1*H*-3-benzazepin-7-yl)-1-phenyl-5-propyl-1*H*-pyrazole-4-carboxamide;
*N*-(3-Cyclobutyl-2,3,4,5-tetrahydro-1*H*-3-benzazepin-7-yl)-5-methyl-1-(2-methylphenyl)-1*H-*pyrazole-4-carboxamide;
*N*-(3-Cyclobutyl-2,3,4,5-tetrahydro-1*H*-3-benzazepin-7-yl)-1-(methyloxy)-2-naphthalenecarboxamide;
1-(4-Chlorophenyl)-N-(3-cyclobutyl-2,3,4,5-tetrahydro-1H-3-benzazepin-7-yl)-5-methyl-1H-pyrazole-4-carboxamide;
5-Methyl-*N*-[3-(3-methylcyclopentyl)-2,3,4,5-tetrahydro-1*H*-3-benzazepin-7-yl]-2-phenyl-2*H-*1,2,3-triazole-4-carboxamide;
*N*-(3-Cyclohexyl-2,3,4,5-tetrahydro-1*H*-3-benzazepin-7-yl)-5-methyl-2-phenyl-2*H*-1,2,3-triazole-4-carboxamide;
5-Methyl-*N*-[3-(2-methylcyclopentyl)-2,3,4,5-tetrahydro-1*H*-3-benzazepin-7-yl]-2-phenyl-2*H-*1,2,3-triazole-4-carboxamide trifluoroacetate; or
*N*-(3-Cyclopentyl-2,3,4,5-tetrahydro-1*H*-3-benzazepin-7-yl)-5-methyl-2-phenyl-2*H*-1,2,3-triazole-4-carboxamide trifluoroacetate;
or a pharmaceutically acceptable salt or solvate thereof.

3. A pharmaceutical composition which comprises the compound of formula (I) as defined in claim 1 or claim 2 or a pharmaceutically acceptable salt or solvate thereof and a pharmaceutically acceptable carrier or excipient.

4. A compound as defined in claim 1 or claim 2 or a pharmaceutically acceptable salt or solvate thereof for use in therapy.

5. Use of a compound as defined in claim 1 or claim 2 or a pharmaceutically acceptable salt or solvate thereof in the manufacture of a medicament for the treatment of neurological diseases.

## Patentansprüche

1. Verbindung der Formel (I) oder ein pharmazeutisch verträgliches Salz davon: 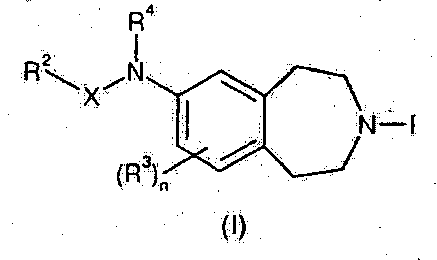 wobei:
R¹ für -C₃₋₇-Cyloalkyl steht, das gegebenenfalls mit C₁₋₃-Alkyl substituiert ist;
R² Wasserstoff, -C₁₋₆-Alkyl, -C₃₋₈-Cycloalkyl, -C₁₋₆-Alkyl-C₃₋₈-cycloalkyl, -Aryl, -Heterocyclyl, -Heteroaryl, -C₃₋₈-Cycloalkyl-Y-C₃₋₈-cycloalkyl, -C₃₋₈-Cycloalkyl-Y-aryl, -C₃₋₈-Cycloalkyl-Y-heteroaryl, -C₃₋₈-Cycloalkyl-Y-heterocyclyl, -Aryl-Y-C₃₋₈-cycloalkyl, -Aryl-Y-aryl, -Aryl-Y-heteroaryl, -Aryl-Y-heterocyclyl, -Heteroaryl-Y-C₃₋₈-cycloalkyl, -Heteroaryl-Y-aryl, -Heteroaryl-Y-heteroaryl, -Heteroaryl-Y-heterocyclyl, -Heterocyclyl-Y-C₃₋₈-cycloalkyl, -Heterocyclyl-Y-aryl, -Heterocyclyl-Y-heteroaryl, -Heterocyclyl-Y-heterocyclyl bedeutet;
X eine Bindung, CO, SO₂, CONR⁵, COO oder COC₂₋₆-Alkenyl bedeutet;
Y eine Bindung, C₁₋₆-Alkyl, CO, CONH, NHCO, O, SO₂, SO₂NH oder NHSO₂ bedeutet;
R³ Halogen, C₁₋₆-Alkyl, C₁₋₆-Alkoxy, Cyano, Amino oder Trifluormethyl bedeutet;
R⁴ und R⁵ unabhängig Wasserstoff, -C₁₋₆-Alkyl, -C₃₋₈-Cycloalkyl, -Aryl, -Heterocyclyl oder -Heteroaryl bedeuten;
n 0,1 oder 2 ist;
wobei die Alkyl-, Cycloalkyl-, Aryl-, Heteroaryl- und Heterocyclylreste von R², R³ und R⁴ gegebenenfalls mit einem oder mehreren (z.B. 1, 2 oder 3) Substituenten substituiert sein können, die gleich oder verschieden sein können und die ausgewählt sind aus Halogen, Hydroxy, Cyano, Nitro, =O, Halo-C₁₋₆-alkyl, Halo-C₁₋₆-alköxy, C₁₋₆-Alkyl, C₁₋₆-Alkoxy, Aryl-C₁₋₆-alkoxy, C₁₋₆-Alkylthio, C₁₋₆-Alkoxy-C₁₋₆-alkyl, C₃₋₇-Cycloalkyl-C₁₋₆-alkoxy, C₁₋₆-Alkanoyl, C₁₋₆-Alkoxycarbonyl, C₁₋₆-Alkylsulfonyl, C₁₋₆-Alkylsulfinyl, C₁₋₆-Alkylsulfonyloxy, C₁₋₆-Alkylsulfonyl-C₁₋₆-alkyl, Sulfonyl, Arylsulfonyl, Arylsulfonyloxy, Arylsulfonyl-C₁₋₆-alkyl, Aryloxy, C₁₋₆-Alkylsulfonamido, C₁₋₆-Alkylamino, C₁₋₆-Alkylamido, -R⁸, -CO₂R⁸, -COR⁸, C₁₋₆-Alkylsulfonamido-C₁₋₆-alkyl, C₁₋₆-Alkylamido-C₁₋₆-alkyl, Arylsulfonamido, Arylcarboxamido, Arylsulfonamido-C₁₋₆-alkyl, Arylcarboxamido-C₁₋₆-alkyl, Aryl, Aroyl, Aroyl-C₁₋₆-alkyl, Aryl-C₁₋₆-alkanoyl oder einem Rest -NR⁶R⁷, -C₁₋₆-Alkyl-NR⁶R⁷, -C₃₋₈-Cycloalkyl-NR⁶R⁷ -CONR⁶R⁷ -NR⁶COR⁷, -NR⁶SO₂R⁷, -OCONR⁶R⁷, -NR⁶CO₂R⁷,
- NR⁸CONR⁶R⁷ oder -SO₂NR⁶R⁷ (wobei R⁶, R⁷ und R⁸ unabhängig Wasserstoff, C₁₋₆-Alkyl, -C₃₋₈-Cycloalkyl, -C₁₋₆- Alkyl-C₃₋₈-cycloalkyl; Aryl, Heterocyclyl oder Heteroaryl bedeuten oder -NR⁶R⁷ einen Stickstoff-haltigen Heterocyclylrest bedeuten kann, wobei die Reste R⁵, R⁶ und R⁷ gegebenenfalls mit einem oder mehreren (z.B. 1, 2 oder 3) Substituenten substituiert sein können, die gleich oder verschieden sein können und die ausgewählt sind aus Halogen, Hydroxy, C₁₋₆-Alkyl, C₁₋₆-Alkoxy, Cyano, Amino, =O oder Trifluormethyl); oder Solvate davon;
wobei die Verbindung nicht 7-Amino-3-cyclopropyl-2,3,4,5-tetrahydro-1H-3-benzazepin ist.

2. Verbindung nach Anspruch 1, bei welcher es sich um folgende handelt:
N-(3-Cyclobutyl-2,3,4,5-tetrahydro-1H-3-benzazepin-7-yl)-4-morpholincarboxamid;
4-Cyano-N-(3-cyclobutyl-2,3,4,5-tetrahydro-1H-3-benzazepin-7-yl)benzamid;
N-(3-Cyclobutyl-2,3,4,5-tetrahydro-1H-3-benzazepin-7-yl)tetrahydro-2H-pyran-4-carboxamid;
N-(3-Cyclobutyl-2,3,4,5-tetrahydro-1H-3-benzazepin-7-yl)acetamid;
N-(3-Cyclobutyl-2,3,4,5-tetrahydro-1H-3-benzazepin-7-yl)cyclopropancarboxamid;
N-(3-Cyclobutyl-2,3,4,5-tetrahydro-1H-3-benzazepin-7-yl)methansulfonamid;
6-[(3-Cyclobutyl-2,3,4,5-tetrahydro-1H-3-benzazepin-7-yl)amino]-N-methyl-3-pyridincarboxamid;
N-(3-Cyclobutyl-2,3,4,5-tetrahydro-1H-3-benzazepin-7-yl)-4-(2-pyridinyl)benzamid;
N-(3-Cyclobutyl-2,3,4,5-tetrahydro-1H-3-benzazepin-7-yl)-5-hydroxy-2-pyrazin-carboxamid;
N-(3-Cyclobutyl-2,3,4,5-tetrahydro-1H-3-benzazepin-7-yl)-4-(1,3-oxazol-4-yl)benzamid;
N-(3-Cyclobutyl-2,3,4,5-tetrahydro-1H-3-benzazepin-7-yl)-4-iodbenzamid;
*N*-(3-Cyclobutyl-2,3,4,5-tetrahydro-1H-3-benzazepin-7-yl)-4-(3-pyridinyl)benzamid;
N-(3-Cyclohexyl-2,3,4,5-tetrahydro-1H-3-benzazepin-7-yl)-5-methyl-1-phenyl-1H-pyrazol-4-carböxamidtrifluoracetat;
N-(3-Cyclobutyl-2,3,4,5-tetrahydro-1H-3-benzazepin-7-yl)-4-(1,3-oxazol-5-yl)benzamid;
N-(3-Cyclobutyl-2,3,4,5-tetrahydro-1H-3-benzazepin-7-yl)-4-(3,5-dimethyl-4-isoxazolyl)benzamid;
N-(3-Cyclobutyl-2,3,4,5-tetrahydro-1H-3-benzazepin-7-yl)-4-imidazo[1,2-a]pyridin-2-yl-benzamid;
N-(3-Cyclobutyl-2,3,4,5-tetrahydro-1H-3-benzazepin-7-yl)-1,2,5-oxadiazol-3-carboxamid; N-(3-Cyclobutyl-2,3,4,5-tetrahydro-1H-3-benzazepin-7-yl)-3-isoxazolcarboxamid;
N-(3-Cyclobutyl-2,3,4,5-tetrahydro-1H-3-benzazepin-7-yl)cyclohexancarboxamid;
N-(3-Cyclobutyl-2,3,4,5-tetrahydro-1H-3-benzazepin-7-yl)-2-cyclopentylacetamid;
N-(3-Cyclobutyl-2,3,4,5-tetrahydro-1H-3-benzazepin-7-yl)-3-methylbenzamid;
N-(3-Cyclobutyl-2,3,4,5-tetrahydro-1H-3-benzazepin-7-yl)-4-fluorbenzamid;
N-(3-Cyclobutyl-2,3,4,5-tetrahydro-1H-3-benzazepin-7-yl)-3-methyl-2-thiophen-carboxamid;
N-(3-Cyclobutyl-2,3,4,5-tetrahydro-1H-3-benzazepin-7-yl)bicyclo[4.2.0]octa-1,3,5-trien-7-carboxamid;
N-(3-Cyclobutyl-2,3,4,5-tetrahydro-1H-3-benzazepin-7-yl)-2-methylbenzamid;
N-(3-Cyclobutyl-2,3,4,5-tetrahydro-1H-3-benzazepin-7-yl)-4-methylbenzamid;
N-(3-Cyclobutyl-2,3,4,5-tetrahydro-1H-3-benzazepin-7-yl)-2-ethylbutanamid;
N-(3-Cyclobutyl-2,3,4,5-tetrahydro-1H-3-benzazepin-7-yl)-3-fluorbenzamid;
N-(3-Cyclobutyl-2,3,4,5-tetrahydro-1H-3-benzazepin-7-yl)-4-(5-pyrimidinyl)benzamid;
N-(3-Cyclobutyl-2,3,4,5-tetrahydro-1H-3-benzazepin-7-yl)-4-(methylsulfonyl)benzamid;
N-(3-Cyclobutyl-2,3,4,5-tetrahydro-1H-3-benzazepin-7-yl)-6-chinoxalincarboxamid;
N-(3-Cyclobutyl-2,3,4,5-tetrahydro-1H-3-benzazepin-7-yl)-2-methyl-1,6-naphthyridin-3-carboxamid;
N-(3-Cyclobutyl-2,3,4,5-tetrahydro-1H-3-benzazepin-7-yl)-4-(1H-tetrazol-1-yl)benzamid;
4-(6-Cyano-3-pxidinyl)-N-(3-cyclobutyl-2,3,4,5-tetrahydro-1H-3-benzazepin-7-yl)-benzamid;
N-(3-Cyclobutyl-2,3,4,5-tetrahydro-1H-3-benzazepin-7-yl)-2-(trifluoromethyl)-1,8-naphthyridin-3-carboxamid;
N-(3-Cyclobutyl-2,3,4,5-tetrahydro-1H-3-benzazepin-7-yl)-4-methylnicotinamid;
N-(3-Cyclobutyl-2,3,4,5-tetrahydro-1H-3-benzazepin-7-yl)-6-methylnicotinamid;
N-(3-Cyclobutyl-2,3,4,5-tetrahydro-1H-3-benzazepin-7-yl)nicotinamid;
N-(3-Cyclobutyl-2,3,4,5-tetrahydro-1H-3-benzazepin-7-yl)-4-(trifluormethyl)nicotinamid;
N-(3-Cyclobutyl-2,3,4,5-tetrahydro-1H-3-benzazepin-7-yl)-6-(1H-pyrazol-1-yl)nicotin-amid;
N-(3-Cyclobutyl-2,3,4,5-tetrahydro-1H-3-benzazepin-7-yl)-6-(trifluormethyl)nicotin-amid;
N-(3-Cyclobutyl-2,3,4,5-tetrahydro-1H-3-benzazepin-7-yl)-4-(5-methyl-1H-tetrazol-1-yl)-benzamid;
N-(3-Cyclobutyl-2,3,4,5-tetrahydro-1H-3-benzazepin-7-yl)-5-(3-pyridinyl)-1H-pyrazol-3-carboxamid;
N-(3-Cyclobutyl-2,3,4,5-tetrahydro-1H-3-benzazepin-7-yl)-6-(4-morpholinyl)-3-pyridincarboxamid;
N-(3-Cyclobutyl-2,3,4,5-tetrahydro-1H-3-benzazepin-7-yl)-1H-1,2,3-benzotriazol-5-carboxamid;
N-(3-Cyclobutyl-2,3,4,5-tetrahydro-1H-3-benzazepin-7-yl)-4-(2-pyrazinyl)benzamid;
N-(3-Cyclobutyl-2,3,4,5-tetrahydro-1H-3-benzazepin-7-yl)-2-methyl-1,8-naphthyridin-3-carboxamid;
N-(3-Cyclobutyl-2,3,4,5-tetrahydro-1H-3-benzazepin-7-yl)-5-chinoxalincarboxamid;
N-(3-Cyclobutyl-2,3,4,5-tetrahydro-1H-3-benzazepin-7-yl)pyrazolo[1,5-a]pyrimidin-3-carboxamid;
N-(3-Cyclobutyl-2,3,4,5-tetrahydro-1H-3-benzazepin-7-yl)-5-pyrimidincarboxamid;
N-(3-Cydobutyl-2,3,4,5-tetrahydro-1H-3-benzazepin-7-yl)-4-pyridazincarboxamid;
N-(3-Cyclobutyl-2,3,4,5-tetrahydro-1H-3-benzazepin-7-yl)-3-(5-pyrimidinyl)benzamid;
N-(3-Cyclobutyl-2,3,4,5-tetrahydro-1H-3-benzazepin-7-yl)-3-(2-pyrazinyl)benzamid;
N-(3-Cyclobutyl-2,3,4,5-tetrahydro-1H-3-benzazepin-7-yl)-3-(4-pyrimidinyl)benzamid;
N-(3-Cyclobutyl-2,3,4,5-tetrahydro-1H-3-benzazepin-7-yl)-4-(4-pyrimidinyl)benzamid;
N-(3-Cyclobutyl-2,3,4,5-tetrahydro-1H-3-benzazepin-7-yl)-5-methyl-3-isoxazol-carboxamid;
6-Cyano-N-(3-cyclobutyl-2,3,4,5-tetrahydro-1H-3-benzazepin-7-yl)-3-pyridincarboxamid;
N-(3-Cyclobutyl-2,3,4,5-tetrahydro-1H-3-benzazepin-7-yl)-4-(1H-imidazol-1-yl)benzamid; N-(3-Cyclobutyl-2,3,4,5-tetrahydro-1H-3-benzazepin-7-yl)-2-pyrazincarboxamid;
N-(3-Cyclobutyl-2,3,4,5-tetrahydro-1H-3-benzazepin-7-yl)-2-methyl-3-pyridincarboxamid ; N-(3-Cyclobutyl-2,3,4,5-tetrahydro-1H-3-benzazepin-7-yl)-3-methyl-2-pyridincarboxamid; N-(3-Cyclobutyl-2,3,4,5-tetrahydro-1H-3-benzazepin-7-yl)-3-thiophencarboxamid;
N-(3-Cyclobutyl-2,3,4,5-tetrahydro-1H-3-benzazepin-7-yl)-3-furancarboxamid;
N-(3-Cyclobutyl-2,3,4,5-tetrahydro-1H-3-benzazepin-7-yl)-2-methyl-3-furancarboxamid;
N-(3-Cyclobutyl-2,3,4,5-tetrahydro-1H-3-benzazepin-7-yl)-2,5-dimethyl-3-furan-carboxamid;
N-(3-Cyclobutyl-2,3,4,5-tetrahydro-1H-3-benzazepin-7-yl)-2-methyl-5-phenyl-3-furan-carboxamid;
N-(3-Cyclobutyl-2,3,4,5-tetrahydro-1H-3-benzazepin-7-yl)-1,2,3-thiadiazol-4-carboxamid;
N-(3-Cyclobutyl-2,3,4,5-tetrahydro-1H-3-benzazepin-7-yl)-1,3-thiazol-4-carboxamid;
N-(3-Cyclobutyl-2,3,4,5-tetrahydro-1H-3-benzazepin-7-yl)-5-methyl-2-phenyl-2H-1,2,3-triazol-4-carboxamid;
N-(3-Cyclobutyl-2,3,4,5-tetrahydro-1H-3-benzazepin-7-yl)-1-methyl-1H-imidazol-4-carboxamid;
N-(3-Cyclobutyl-2,3,4,5-tetrahydro-1H-3-benzazepin-7-yl)-1 -methyl-1H-1,2,3-triazol-4-carboxamid;
N-(3-Cyclobutyl-2,3,4,5-tetrahydro-1H-3-benzazepin-7-yl)-5-phenyl-1,3-oxazol-4-carboxamid;
N-(3-Cyclobutyl-2,3,4,5-tetrahydro-1H-3-benzazepin-7-yl)-2-phenyl-2H-1,2,3-triazol-4-carboxamid;
2-(2,1,3-Benzoxadiazol-5-yl)-N-(3-cyclobutyl-2,3,4,5-tetrahydro-1H-3-benzazepin-7-yl)-1,3-thiazol-4-carboxamid;
N-(3-Cyclobutyl-2,3,4,5-tetrahydro-1H-3-benzazepin-7-yl)-1,5-dimethyl-1H-pyrazol-3-carboxamid;
N-(3-Cyclobutyl-2,3,4,5-tetrahydro-1H-3-benzazepin-7-yl)-1,2,5-thiadiazol-3-carboxamid;
N-(3-Cyclobutyl-2,3,4,5-tetrahydro-1H-3-benzazepin-7-yl)-6-(1H-1,2,4-triazol-1-yl)-3-pyridincarboxamid;
N-(3-Cyclobutyl-2,3,4,5-tetrahydro-1H-3-benzazepin-7-yl)-6-phenyl-3-pyridincarboxamid; N-(3-Cyclobutyl-2,3,4,5-tetrahydro-1H-3-benzazepin-7-yl)-3,3'-bipyridin-5-carboxamid;
N-(3-Cyclobutyl-2,3,4,5-tetrahydro-1H-3-benzazepin-7-yl)-6-oxo-1,6-dihydro-3-pyridincarboxamid;
N-(3-Cyclobutyl-2,3,4,5-tetrahydro-1H-3-benzazepin-7-yl)-3-methyl-5-isoxazol-carboxamid;
N-(3-Cyclobutyl-2,3,4,5-tetrahydro-1H-3-benzazepin-7-yl)-5-methyl-2-pyrazin-carboxamid;
N-(3-Cyclobutyl-2,3,4,5-tetrahydro-1H-3-benzazepin-7-yl)-6-oxo-1,6-dihydro-2-pyridincarboxamid;
N-(3-Cyclobutyl-2,3,4,5-tetrahydro-1H-3-benzazepin-7-yl)-2,4-dimethyl-1,3-thiazol-carboxamid;
N-(3-Cyclobutyl-2,3,4,5-tetrahydro-1H-3-benzazepin-7-yl)-2-phenyl-1,3-thiazol-4-carboxamid;
N-(3-Cyclobutyl-2,3,4,5-tetrahydro-1H-3-benzazepin-7-yl)-4-(1,1-dioxido-2-isothiazolidinyl)benzamid;
N-(3-Cyclobutyl-2,3,4,5-tetrahydro-1H-3-benzazepin-7-yl)-4-(2-oxo-1-pyrrolidinyl)-benzamid;
N-(3-Cyclobutyl-2,3,4,5-tetrahydro-1H-3-benzazepin-7-yl)-4-(2-oxo-1-imidazolidinyl)-benzamid;
N-(3-Cyclobutyl-2,3,4,5-tetrahydro-1H-3-benzazepin-7-yl)-4-(1H-pyrazol-1-yl)-benzamid;
N-(3-Cyclobutyl-2,3,4,5-tetrahydro-1H-3-benzazepin-7-yl)-4-(1H-1,2,4-triazol-1-yl)-benzamid;
N-(3-Cyclobutyl-2,3,4,5-tetrahydro-1H-3-benzazepin-7-yl)-3-(2-oxo-1-pyrrolidinyl)-benzamid;
N-(3-Cyclobutyl-2,3,4,5-tetrahydro-1H-3-benzazepin-7-yl)-3-(1,1-dioxido-2-isothiazolidinyl)benzamid;
N-(3-Cyclobutyl-2,3,4,5-tetrahydro-1H-3-benzazepin-7-yl)-3-(2-oxo-1-imidazolidinyl)-benzamid;
N-(3-Cyclobutyl-2,3,4,5-tetrahydro-1H-3-benzazepin-7-yl)-3-(1H-pyrazol-1-yl)benzamid; N-(3-Cyclobutyl-2,3,4,5-tetrahydro-1H-3-benzazepin-7-yl)-3-(1H-1,2,4-triazol-1-yl)benzamid;
N-(3-Cyclobutyl-2,3,4,5-tetrahydro-1H-3-benzazepin-7-yl)-N-methyl-2,5-pyrazin-dicarboxamid;
N-(3-Cyclobutyl-2,3,4,5-tetrahydro-1H-3-benzazepin-7-yl)-4-(4-pyridinil)-benzamid;
6-(4-Cyanophenyl)-N-(3-cyclobutyl-2,3,4,5-tetrahydro-1H-3-benzazepin-7-yl)-3-pyridincarboxamid;
N-(3-Cyclobutyl-2,3,4,5-tetrahydro-1H-3-benzazepin-7-yl)-2,3'-bipyridin-5-carboxamid;
N-(3-Cyclobutyl-2,3,4,5-tetrahydro-1H-3-benzazepin-7-yl)-6-(5-pyrimidinyl)-3-pyridincarboxamid;
6-(3-Cyanophenyl)-N-(3-cyclobutyl-2,3,4,5-tetrahydro-1H-3-benzazepin-7-yl)-3-pyridincarboxamid;
N-(3-Cyclobutyl-2,3,4,5-tetrahydro-1H-3-benzazepin-7-yl)-5-(4-fluorphenyl)-2-pyridincarboxamid;
5-(4-Cyanophenyl)-N-(3-cyclobutyl-2,3,4,5-tetrahydro-1H-3-benzazepin-7-yl)-2-pyridincarboxamid;
6'-Cyano-N-(3-cyclobutyl-2,3,4,5-tetrahydro-1H-3-benzazepin-7-yl)-3,3'-bipyridin-6-carboxamid;
N-(3-Cyclobutyl-2,3,4,5-tetrahydro-1H-3-benzazepin-7-yl)-5-(5-pyrimidinyl)-2-pyridincarboxamid;
N-(3-Cyclobutyl-2,3,4,5-tetrahydro-1H-3-benzazepin-7-yl)-5-(2-pyrazinyl)-2-pyridincarboxamid;
N-(3-Cyclobutyl-2,3,4,5-tetrahydro-1H-3-benzazepin-7-yl)-2,3'-bipyridin-6'-carboxamid;
N-(3-Cyclobutyl-2,3,4,5-tetrahydro-1H-3-benzazepin-7-yl)-5-(1H-pyrazol-1-yl)-2-pyridincarboxamid;
N-(3-Cydobutyl-2,3,4,5-tetrahydro-1H-3-benzazepin-7-yl)-5-(1H-imidazol-1-yl)-2-pyridincarboxamid;
N-(3-Cyclobutyl-2,3,4,5-tetrahydro-1H-3-benzazepin-7-yl)-5-(1H-1,2,4-triazol-1-yl)-2-pyridincarboxamid;
N-(3-Cyclobutyl-2,3,4,5-tetrahydro-1H-3-benzazepin-7-yl)-5-(4-morpholinyl)-2-pyrazin-carboxamid;
N-(3-Cyclobutyl-2,3,4,5-tetrahydro-1H-3-benzazepin-7-yl)-5-(1-pyrrolidinyl)-2-pyrazin-carboxamid;
N-(3-Cyclobutyl-2,3,4,5-tetrahydro-1H-3-benzazepin-7-yl)-5-(1-piperidinyl)-2-pyrazin-carboxamid;
N-(3-Cyclobutyl-2,3,4,5-tetrahydro-1H-3-benzazepin-7-yl)-5-(tetrahydro-2H-pyran-4-yl-oxy)-2-pyrazincarboxamid;
N-(3-Cyclobutyl-2,3,4,5-tetrahydro-1H-3-benzazepin-7-yl)-5-(phenyloxy)-2-pyrazin-carboxamid;
5-(4-Cyanophenyl)-N-(3-cyclobutyl-2,3,4,5-tetrahydro-1H-3-benzazepin-7-yl)-2-pyrazin-carboxamid;
5-(3-Cyanophenyl)-N-(3-cyclobutyl-2,3,4,5-tetrahydro-1H-3-benzazepin-7-yl)-2-pyrazincarboxamid;
N-(3-Cyclobutyl-2,3,4,5-tetrahydro-1H-3-benzazepin-7-yl)-4-pyridincarboxamid;
N-(3-Cyclobutyl-2,3,4,5-tetrahydro-1H-3-benzazepin-7-yl)-4-biphenylcarboxamid;
3-Cyano-N-(3-cyclobutyl-2,3,4,5-tetrahydro-1H-3-benzazepin-7-yl)benzamid;
N-(3-Cyclobutyl-2,3,4,5-tetrahydro-1H-3-benzazepin-7-yl)-1,3-dimethyl-1H-pyrazol-5-carboxamid;
N-(3-Cyclobutyl-2,3,4,5-tetrahydro-1H-3-benzazepin-7-yl)-3,5-dimethyl-4-isoxazol-carboxamid;
5-Brom-N-(3-cyclobutyl-2,3,4,5-tetrahydro-1H-3-benzazepin-7-yl)-3-pyridincarboxamid;
N-(3-Cyclobutyl-2,3,4,5-tetrahydro-1H-3-benzazepin-7-yl)-5-(1H-imidazol-1-yl)-3-pyridincarboxamid;
N-(3-Cyclobutyl-2,3,4,5-tetrahydro-1H-3-benzazepin-7-yl)-N-methyl-4-(2-pyrazinyl)-benzamid;
N-(3-Cyclobutyl-2,3,4,5-tetrahydro-1H-3-benzazepin-7-yl)-N-methyl-4-(2-pyridinyl)-benzamid;
N-(3-Cyclobutyl-2,3,4,5-tetrahydro-1H-3-benzazepin-7-yl)-N-methyl-4-(5-pyrimidinyl)-benzamid;
N-(3-Cyclobutyl-2,3,4,5-tetrahydro-1H-3-benzazepin-7-yl)-N-6-dimethyl-3-pyridincarboxamid;
N-(3-Cyclobutyl-2,3,4,5-tetrahydro-1H-3-benzazepin-7-yl)-N-phenylharnstoff;
N-(4-Cyanophenyl)-N-(3-cyclobutyl-2,3,4,5-tetrahydro-1H-3-benzazepin-7-yl)harnstoff;
N-1,3-Benzodioxol-5-yl-N-(3-cyclobutyl-2,3,4,5-tetrahydro-1H-3-benzazepin-7-yl)-harnstoff;
N-(3-Cyclobutyl-2,3,4,5-tetrahydro-1H-3-benzazepin-7-yl)-N-cyclohexylharnstoff;
N-(3-Cyclobutyl-2,3,4,5-tetrahydro-1H-3-benzazepin-7-yl)-N-(1-methylethyl)harnstoff;
N-(3-Cyclobutyl-2,3,4,5-tetrahydro-1H-3-benzazepin-7-yl)-N-[2-(methyloxy)-phenyl]-harnstoff;
N-(3-Cyclobutyl-2,3,4,5-tetrahydro-1H-3-benzazepin-7-yl)-N-8-chinolinylharnstoff;
N-(3-Cyclobutyl-2,3,4,5-tetrahydro-1H-3-benzazepin-7-yl)-N-(2,2-difluor-1,3-benzo-dioxol-4-yl)harnstoff;
N-(3-Cyclobutyl-2,3,4,5-tetrahydro-1H-3-benzazepin-7-yl)-N-3-pyridinylharnstoff;
N-(3-Cyclobutyl-2,3,4,5-tetrahydro-1H-3-benzazepin-7-yl)-N-4-pyridinylharnstoff;
N-(3-Cyclobutyl-2,3,4,5-tetrahydro-1H-3-benzazepin-7-yl)-N-[2-(ethyloxy)phenyl]-harnstoff;
N-(3-Cyclobutyl-2,3,4,5-tetrahydro,1H-3-benzazepin-7-yl)-N-{2, [(trifluormethyl)oxy]-phenyl}harnstoff;
N-(3-Cyclobutyl-2,3,4,5-tetrahydro-1H-3-benzazepin-7-yl)-N-[2-methyl-6-(methyloxy)-phenyl]harnstoff;
N-(3-Cyanophenyl)-N-(3-cyclobutyl-2,3,4,5-tetrahydro-1H-3-benzazepin-7-yl)harnstoff;
N-[5-Chlor-2-(methyloxy)phenyl]-N-(3-cyclobutyl-2,3,4,5-tetrahydro-1H-3-benzazepin-7-yl)harnstoff;
N-(3-Cyclobutyl-2,3,4,5-tetrahydro-1H-3-benzazepin-7-yl)-4-pyrazin-2-ylpiperazin-1-carboxamid;
4-(5-Cyanopyridin-2-yl)-N-(3-cyclobutyl-2,3,4,5-tetrahydro-1H-3-benzazepin-7-yl)-piperazin-1-carboxamid;
5-Chlor-N-(3-cyclobutyl-2,3,4,5-tetrahydro-1H-3-benzazepin-7-yl)indolin-1-carboxamid;
N-(3-Cyclobutyl-2,3,4,5-tetrahydro-1H-3-benzazepin-7-yl)-2,3-dihydro-1H-indol-1-carboxamid;
N-(3-Cyclobutyl-2,3,4,5-tetrahydro-1H-3-benzazepin-7-yl)-3,4-dihydro-1(2H)-chinolincarboxamid;
N-(3-Cyclobutyl-2,3,4,5-tetrahydro-1H-3-benzazepin-7-yl)-1,3-dihydro-2H-isoindol-2-carboxamid;
N-(3-Cyclobutyl-2,3,4,5-tetrahydro-1H-3-benzazepin-7-yl)-3,4-dihydro-2(1H)-isochinolin-carboxamid;
N-(3-Cyclobutyl-2,3,4,5-tetrahydro-1H-3-benzazepin-7-yl)-4-phenyl-1-piperidin-carboxamid;
4-[(4-Cyanophenyl)oxy]-N-(3-cyclobutyl-2,3,4,5-tetrahydro-1H-3-benzäzepin-7-yl)-1-piperidincarboxamid;
N-(3-Cyclobutyl-2,3,4,5-tetrahydro-1H-3-benzazepin-7-yl)-4-(2-pyridinyl)-1-piperidin-carboxamid;
N-(3-Cyclobutyl-2,3,4,5-tetrahydro-1H-3-benzazepin-7-yl)-4-(4-pyridinyl)-1-piperidin-carboxamid;
N-(3-Cyclobutyl-2,3,4,5-tetrahydro-1H-3-benzazepin-7-yl)-4-phenyl-1-piperazin-carboxamid;
4-(4-Cyanophenyl)-N-(3-cyclobutyl-2,3,4,5-tetrahydro-1H-3-benzazepin-7-yl)-1-piperazin-carboxamid;
N-(3-Cyclobutyl-2,3,4,5-tetrahydro-1H-3-benzazepin-7-yl)-1-piperidincarboxamid;
N-(3-Cyclobutyl-2,3,4,5-tetrahydro-1H-3-benzazepin-7-yl)-1-pyrrolidincarboxamid;
N-(3-Cyclobutyl-2,3,4,5-tetrahydro-1H-3-benzazepin-7-yl)-5-fluor-2,3-dihydro-1H-indol-1-carboxamid;
N-(3-Cyclobutyl-2,3,4,5-tetrahydro-1H-3-benzazepin-7-yl)-4-(4-fluorphenyl)-1-piperidincarboxamid;
N-(3-Cyclobutyl-2,3,4,5-tetrahydro-1H-3-benzazepin-7-yl)-2-phenyl-1-pyrrolidin-carboxamid;
5-Cyano-N-(3-cyclobutyl-2,3,4,5-tetrahydro-1H-3-benzazepin-7-yl)-2,3-dihydro-1H-indol-1-carboxamid;
4-(4-Cyanophenyl)-N-(3-cyclobutyl-2,3,4,5-tetrahydro-1H-3-benzazepin-7-yl)-1-piperidincarboxamid;
N-(3-Cyclobutyl-2,3,4,5-tetrahydro-1H-3-benzazepin-7-yl)-1,2,4,5-tetrahydro-3H-3-benzazepin-3-carboxamid;
4-(3-Cyano-2-pyrazinyl)-N-(3-cyclobutyl-2,3,4,5-tetrahydro-1H-3-benzazepin-7-yl)-1-piperazincarboxamid;
N-(3-Cyclobutyl-2,3,4,5-tetrahydro-1H-3-benzazepin-7-yl)-2;3-dihydro-4H-1,4-benzoxazin-4-carboxamid;
N-(3-Cyclobutyl-2,3,4,5-tetrahydro-1H-3-benzazepin-7-yl)-4{[4-(methyloxy)phenyl]oxy}-1-piperidincarboxamid;
N-(3-Cyclobutyl-2,3,4,5-tetrahydro-1H-3-benzazepin-7-yl)-4-[(4-fluorphenyl)oxy]-1-piperidincarboxamid;
N-(3-Cyclobutyl-2,3,4,5-tetrahydro-1H-3-benzazepin-7-yl)-4-(phenyloxy)-1-piperidincarboxamid;
N-(3-Cyclobutyl-2,3,4,5-tetrahydro-1H-3-benzazepin-7-yl)-4-(2-pyridinyloxy)-1-piperidincarboxamid;
N-(3-Cyclobutyl-2,3,4,5-tetrahydro-1H-3-benzazepin-7-yl)-4-(3-pyridinyloxy)-1-piperidincarboxamid;
7-Cyano-N-(3-cyclobutyl-2,3,4,5-tetrahydro-1H-3-benzazepin-7-yl)-2,3-dihydro-4H-1,4-benzoxazin-4-carboxamid;
N-(4-Cyanophenyl)-N-(3-cyclobutyl-2,3,4,5-tetrahydro-1H-3-benzazepin-7-yl)-N-methylharnstoff;
N-(3-Cyclobutyl-2,3,4,5-tetrahydro-1H-3-benzazepim-7-yl)-2,3-dihydro-1H-pyrrolo[2,3-b]pyridin-1-carboxamid;
5-Cyano-N-(3-cyclobutyl-2,3,4,5-tetrahydro-1H-3-benzazepin-7-yl)-1,3-dihydro-2H-isoindol-2-carboxamid;
1-{6-[(3-Cyclobutyl-2,3,4,5-tetrahydro-1H-3-benzazepin-7-yl)amino]-3-pyridinyl}-2-pyrrolidinon;
N-(3-Cyclobutyl-2,3,4,5-tetrahydro-1H-3-benzazepm-7-yl)-1,3-benzodioxol-5-carboxamidtrifluoracetat;
N-(3-Cyclobutyl-2,3,4,5-tetrahydro-1H-3-benzazepin-7-yl)-2-methyl-1H-benzimidazol-5-carboxamidtrifluoracetat;
N-(3-Cyclobutyl-2,3,4,5-tetrahydro-1H-3-benzazepin-7-yl)-2-chinoxalincarboxamidtrifluoracetat;
N-(3-Cyclobutyl-2,3,4,5-tetrahydro-1H-3-benzazepin-7-yl)-2-(methyloxy)benzamidtrifluoracetat;
N-(3-Cyclobutyl-2,3,4,5-tetrahydro-1H-3-benzazepin-7-yl)pyrazolo[1,5-a]pyridin-3-carboxamidtrifluoracetat;
N-(3-Cyclobutyl-2,3,4,5-tetrahydro-1H-3-benzazepin-7-yl)-5-methyl-1-[4-(methyloxy)-phenyl]-1H-pyrazol-4-carboxamidtrifluoracetat;
N-(3-Cyclobutyl-2,3,4,5-tetrahydro-1H-3-benzazepin-7-yl)-3-(methyloxy)benzamid-trifluoracetat;
N-(3-Cyclobutyl-2,3,4,5-tetrahydro-1H-3-benzazepin-7-yl)-1-benzothiophen-2-carboxamidtrifluoracetat ;
N-(3-Cyclobutyl-2,3,4,5-tetrahydro-1H-3-benzazepin-7-yl)-2-naphthalincarboxamidtrifluoracetat;
1-(4-Chlorphenyl)-N-(3-cyclobutyl-2,3,4,5-tetrahydro-1H-3-benzazepin-7-yl)-5-(trifluormethyl)-1H-pyrazol-4-carboxamidtrifluoracetat ;
N-(3-Cyclobutyl-2,3,4,5-tetrahydro-1H-3-benzazepin-7-yl)-1-(1-methylethyl)-1H-1,2,3-benzotriazol-5-carboxamidtrifluoracetat;
N-(3-Cyclobutyl-2,3,4,5-tetrahydro-1H-3-benzazepin-7-yl)-1-(1-methylethyl)-2-(trifluormethyl)-1H-benzimidazol-5-carboxamidtrifluoracetat;
N-(3-Cyclobutyl-2,3,4,5-tetrahydro-1H-3-benzazepin-7-yl)-2,1-benzisoxazol-3-carboxamidtrifluoracetat;
N-(3-Cyclobutyl-2,3,4,5-tetrahydro-1H-3-benzazepin-7-yl)-1-methyl-3-propyl-1H-pyrazol-5-carboxamidtrifluoracetat;
N-(3-Cyclobutyl-2,3,4,5-tetrahydro-1H-3-benzazepin-7-yl)-1,5-dimethyl-3-oxo-2-phenyl-2,3-dihydro-1H-pyrazol-4-carboxamidtrifluoracetat;
N-(3-Cyclobutyl-2,3,4,5-tetrahydro-1H-3-benzazepin-7-yl)-2-methyl-2H-1,2,3-triazol-4-carboxamidtrifluoracetat;
N-(3-Cyclobutyl-2,3,4,5-tetrahydro-1H-3-benzazepin-7-yl)-1-benzothiophen-3-carboxamidtrifluoracetat;
N-(3-Cyclobutyl-2,3,4,5-tetrahydro-1H-3-benzazepin-7-yl)-1-phenyl-1H-1,2,3-triazol-5-carboxamidtrifluoracetat;
N-(3-Cyclobutyl-2,3,4,5-tetrahydro-1H-3-benzazepin-7-yl)-3-methyl-5-[4-(methyloxy)-phenyl]-1H-pyrazol-4-carboxamidtrifluoracetat;
N-(3-Cyclobutyl-2,3,4,5-tetrahydro-1H-3-benzazepin-7-yl)-5-methyl-1-phenyl-1H-pyrazol-3-carboxamidtrifluoracetat;
N-(3-Cyclobutyl-2,3,4,5-tetrahydro-1H-3-benzazepin-7-yl)-3-[3-(methyloxy)phenyl]-1-phenyl-1 H-pyrazol-4-carboxamidtrifluoracetät;
N-(3-Cyclobutyl-2,3,4,5-tetrahydro-1H-3-benzazepin-7-yl)-3-(1,1-dimethylethyl)-1-methyl-1 H-pyrazol-5-carboxamidtrifluoracetat;
N-(3-Cyclobutyl-2,3,4,5-tetrahydro-1H-3-benzazepin-7-yl)-5,7-dimethyl-pyrazolo[1,5-a]pyrimidin-2-carboxamidtrifluoracetat;
N-(3-Cyclobutyl-2,3,4,5-tetrahydro-1H-3-benzazepin-7-yl)-3-methyl-1-phenyl-1H-pyrazol-5-carboxamidtrifluoracetat;
N-(3-Cyclobutyl-2,3,4,5-tetrahydro-1H-3-benzazepin-7-yl)-1-methyl-1H-1,2,3-benzotriazol-5-carboxamidtrifluoracetat;
N-(3-Cyclobutyl-2,3,4,5-tetrahydro-1H-3-benzazepin-7-yl)-5-methyl-1-[(4-methylphenyl)-methyl]-1H-pyrazol-3-carboxamidtrifluoracetat;
N-(3-Cyclobutyl-2,3,4,5-tetrahydro-1H-3-benzazepin-7-yl)-1-ethyl-3-(2-thienyl)-1H-pyrazol-5-carboxamidtrifluoracetat;
N-(3-Cyclobutyl-2,3,4,5-tetrahydro-1H-3-benzazepin-7-yl)-1-ethyl-5-methyl-1H-pyrazol-3-carboxamidtrifluoracetat;
N-(3-Cydobutyl-2,3,4,5-tetrahydro-1H-3-benzazepin-7-yl)-4-[(4-fluorphenyl)oxy]-1H-1,2,3-triazol-5-carboxamidtrifluoracetat;
N-(3-Cyclobutyl-2,3,4,5-tetrahydro-1H-3-benzazepin-7-yl)-4-ethyl-1-phenyl-1H-1,2,3-triazol-5-carboxamidtrifluoracetat;
N-(3-Cyclobutyl-2,3,4,5-tetrahydro-1H-3-benzazepin-7-yl)-5-ethyl-1-phenyl-1H-1,2,3-triazol-4-carboxamidtrifluoracetat;
N-(3-Cyclobutyl-2,3,4,5-tetrahydro-1H-3-benzazepin-7-yl)-2-methyl-5-phenyl-2H-1,2,3-triazol-4-carboxamidtrifluoracetat;
N-(3-Cyclobutyl-2,3,4,5-tetrahydro-1H-3-benzazepin-7-yl)-5-methyl-1-phenyl-1H-1,2,3-triazol-4-carboxanüdtrifluoracetat;
N-(3-Cyclobutyl-2,3,4,5-tetrahydro-1H-3-benzazepin-7-yl)-2H-chromen-3-carboxamidtrifluoracetat;
N-(3-Cyclobutyl-2,3,4,5-tetrahydro-1H-3-benzazepin-7-yl)-2-methyl-4,5,6,7-tetrahydro-2H-indazol-3-carboxamidtrifluoracetat;
4-[3,4-Bis(methyloxy)phenyl]-N-(3-cyclobutyl-2,3,4,5-tetrahydro-1H-3-benzazepin-7-yl)-1H-1,2,3-triazol-5-carboxamidtrifluoracetat;
N-(3-Cyclobutyl-2,3,4,5-tetrahydro-1H-3-benzazepin-7-yl)-1-(2,2,2-trifluorethyl)-1H-1,2,3-triazol-4-carboxamidtrifluoracetat;
N-(3-Cyclobutyl-2,3,4,5-tetrahydro-1H-3-benzazepin-7-yl)-3,5-dimethyl-1-phenyl-1H-pyrazol-4-carboxamidtrifluoracetat;
N-(3-Cyclobutyl-2,3,4,5-tetrahydro-1H-3-benzazepin-7-yl)-1-methyl-1H-pyrazol-4-carboxamidtrifluoracetat;
N-(3-Cyclobutyl-2,3,4,5-tetrahydro-1H-3-benzazepin-7-yl)-1-methyl-3-(trifluormethyl)-1H-pyrazol-4-carboxamidtrifluoracetat;
1-(2-Chlorphenyl)-N-(3-cyclobutyl-2,3,4,5-tetrahydro-1H-3-benzazepin-7-yl)-5-propyl-1H-pyrazol-4-carboxamidtrifluoracetat;
1-(4-Cyanophenyl)-N-(3-cyclobutyl-2,3,4,5-tetrahydro-1H-3-benzazepin-7-yl)-3-(trifluormethyl)-1H-pyrazol-4-carboxamidtrifluoracetat;
1-(4-Chlorphenyl)-N-(3-cyclobutyl-2,3,4,5-tetrahydro-1H-3-benzazepin-7-yl)-5-propyl-1H-pyrazol-4-carboxamidtrifluoracetat;
N-(3-Cyclobutyl-2,3,4,5-tetrahydro-1H-3-benzazepin-7-yl)-3,4-dihydro-2H-chromen-3-carboxamidtrifluoracetat;
N-(3-Cyclobutyl-2,3,4,5-tetrahydro-1H-3-benzazepin-7-yl)-1,3,5-trimethyl-1H-pyrazol-4-carboxamidtrifluoracetat;
N-(3-Cyclobutyl-2,3,4,5-tetrahydro-1H-3-benzazepin-7-yl)-1-(4-fluorphenyl)-3,5-dimethyl-1H-pyrazol-4-carboxamidtrifluoracetat;
N-(3-Cyclobutyl-2,3,4,5-tetrahydro-1H-3-benzazepin-7-yl)-1-benzofuran-5-carboxamidtrifluoracetat;
N-(3-Cyclobutyl-2,3,4,5-tetrahydro-1H-3-benzazepin-7-yl)-1H-indol-3-carboxamidtrifluoracetat;
N-(3-Cyclobutyl-2,3,4,5-tetrahydro-1 H-3-benzazepin-7-yl)-1 H-indazol-3-carboxamidtrifluoracetat;
N-(3-Cyclobutyl-2,3,4,5-tetrahydro-1H-3-benzazepin-7-yl)-2,3-dihydro-1-benzofuran-2-carboxamidtrifluoracetat;
N-(3-Cyclobutyl-2,3,4,5-tetrahydro-1H-3-benzazepin-7-yl)-1H-indol-5-carboxamidtrifluoracetat;
N-(3-Cyclobutyl-2,3,4,5-tetrahydro-1H-3-benzazepin-7-yl)-2,3-dihydro-1-benzofuran-5-carboxamidtrifluoracetat;
N-(3-Cyclobutyl-2,3,4,5-tetrahydro-1H-3-benzazepin-7-yl)-1,3-benzothiazol-6-carboxamid-trifluoracetat;
N-(3-Cyclobutyl-2,3,4,5-tetrahydro-1H-3-benzazepin-7-yl)-1H-indol-6-carboxamidtrifluoracetat;
N-(3-Cyclobutyl-2,3,4,5-tetrahydro-1H-3-benzazepin-7-yl)-1,2,3-benzothiadiazol-5-carboxamidtrifluoracetat;
N-(3-Cyclobutyl-2,3,4,5-tetrahydro-1H-3-benzazepin-7-yl)-2,7-dimethylpyrazolo[1,5-a]-pyrimidin-6-carboxamidtrifluoracetat;
N-(3-Cyclobutyl-2,3,4,5-tetrahydro-1H-3-benzazepin-7-yl)-1-naphthalincarboxamidtrifluoracetat;
N-(3-Cyclobutyl-2,3,4,5-tetrahydro-1H-3-benzazepin-7-yl)-3-chinolincarboxamid-trifluoracetat;
N-(3-Cyclobutyl-2,3,4,5-tetrahydro-1H-3-benzazepin-7-yl)-4-(methyloxy)-2-chinolin-carboxamidtrifluoracetat;
N-(3-Cyclobutyl-2,3,4,5-tetrahydro-1H-3-benzazepin-7-yl)-2,1,3-benzothiadiazol-5-carböxamidtrifluoracetat;
N-(3-Cyclobutyl-2,3,4,5-tetrahydro-1H-3-benzazepin-7-yl)-2,3-dihydro-1,4-benzodioxin-2- carboxamidtrifluoracetat;
N-(3-Cyclobutyl-2,3,4,5-tetrahydro-1H-3-benzazepin-7-yl)-6-(methyloxy)-2-naphthalin-carboxamidtrifluoracetat ;
N-(3-Cyclobutyl-2,3,4,5-tetrahydro-1H-3-benzazepin-7-yl)-3-isochinolin-carboxamidtrifluoracetat;
N-(3-Cyclobutyl-2,3,4,5-tetrahydro-1H-3-benzazepm-7-yl)-1-methyl-1H-indol-2-carboxamidtrifluoracetat;
N-(3-Cyclobutyl-2,3,4,5-tetrahydro-1H-3-benzazepin-7-yl)-1-benzofuran-2-carboxamidtrifluoracetat;
N-(3-Cyclobutyl-2,3,4,5-tetrahydro-1H-3-benzazepin-7-yl)-4-(methyloxy)benzamid-trifluoracetat;
N-(3-Cyclobutyl-2,3,4,5-tetrahydro-1H-3-benzazepin-7-yl)-1,3-diphenyl-1H-pyrazol-4-carboxamidtrifluoracetat;
N-(3-Cyclobutyl-2,3,4,5-tetrahydro-1H-3-benzazepin-7-yl)-2-chinolincarboxamid-trifluoracetat;
N-(3-Cyclobutyl-2,3,4,5-tetrahydro-1H-3-benzazepin-7-yl)-2,3-dihydro-1,4-benzodioxin-6-carboxamidtrifluoracetat;
N-(3-Cyclobutyl-2,3,4,5-tetrahydro-1H-3-benzazepin-7-yl)-4-(methylsulfonyl)benzamid-trifluoracetat;
N-(3-Cyclobutyl-2,3,4,5-tetrahydro-1H-3-benzazepin-7-yl)-2-(phenyloxy)benzamid-trifluoracetat;
N-(3-Cyclobutyl-2,3,4,5-tetrahydro-1H-3-benzazepin-7-yl)-3-(phenyloxy)benzamid-trifluoracetat;
N-(3-Cyclobutyl-2,3,4,5-tetrahydro-1H-3-benzazepin-7-yl)-4-(phenyloxy)benzamid-trifluoracetat;
N-(3-Cyclobutyl-2,3,4,5-tetrahydro-1H-3-benzazepin-7-yl)-2-(trifluormethyl)benzamid-trifluoracetat;
N-(3-Cydobutyl-2,3,4,5-tetrahydro-1H-3-benzazepin-7-yl)-3-(trifluormethyl)benzamid-trifluoracetat;
N-(3-Cyclobutyl-2,3,4,5-tetrahydro-1H-3-benzazepih-7-yl)-4-(trifluormethyl)benzamid-trifluoracetat;
N-(3-Cyclobutyl-2,3,4,5-tetrahydro-1H-3-benzazepin-7-yl)-2-biphenylcarboxamid-trifluoracetat;
(2E)-N-(3-Cydobutyl-2,3,4,5-tetrahydro-1H-3-benzazepin-7-yl)-3-phenyl-2-propenamid-trifluoracetat ;
N-(3-Cyclobutyl-2,3,4,5-tetrahydro-1H-3-benzazepin-7-yl)-1H-benzimidazol-2-carboxamidtrifluoracetat;
(2E)-N-(3-Cyclobutyl-2,3,4,5-tetrahydro-1H-3-benzazepin-7-yl)-3-[2-(methyloxy)phenyl]-2-propenamidtrifluoracetat;
(2E)-N-(3-Cyclobutyl-2,3,4,5-tetrahydro-1H-3-benzazepin-7-yl)-3-[3-(methyloxy)phenyl]-2-propenamidtrifluoracetat;
(2E)-N-(3-Cyclobutyl-2,3,4,5-tetrahydro-1H-3-benzazepin-7-yl)-3-[4-(methyloxy)phenyl]-2-propenamidtrifluoracetat;
(2E)-N-(3-Cyclobutyl-2,3,4,5-tetrahydro-1H-3-benzazepin-7-yl)-3-(2-fluorphenyl)-2-propenamidtrifluoracetat;
(2E)-N-(3-Cyclobutyl-2,3,4,5-tetrahydro-1H-3-benzazepin-7-yl)-3-(3-fluorphenyl)-2-propenamidtrifluoracetat;
(2E)-N-(3-Cyclobutyl-2,3,4,5-tetrahydro-1H-3-benzazepin-7-yl)-3-(4-fluorphenyl)-2-propenamidtrifluoracetat;
3,5-Dichlor-N-(3-cyclobutyl-2,3,4,5-tetrahydro-1H-3-benzazepin-7=yl)benzamid-trifluoracetat;
N-(3-Cyclobutyl-2,3,4,5-tetrahydro-1H-3-benzazepin-7-yl)-1-ethyl-3-methyl-1H-pyrazol-5-carboxamidtrifluoracetat;
N-(3-Cyclobutyl-2,3,4,5-tetrahydro-1H-3-benzazepin-7-yl)-5-(2-furanyl)-1H-pyrazol-3-carboxamidtrifluoracetat;
N-(3-Cyclobutyl-2,3,4,5-tetrahydro-1H-3-benzazepin-7-yl)-1-(3,4-dichlorphenyl)-3,5-dimethyl-1 H-pyrazol-4-carboxamidtrifluoracetat;
N-(3-Cyclobutyl-2,3,4,5-tetrahydro-1H-3-benzazepin-7-yl)-3-(methyloxy)-2-naphthalin-carboxamidtrifluoracetat;
N-(3-Cyclobutyl-2,3,4,5-tetrahydro-1H-3-benzazepin-7-yl)-1-(4-fluorphenyl)-5-phenyl-1H-pyrazol-3-carboxamid;
N-(3-Cyclobutyl-2,3,4,5-tetrahydro-1H-3-benzazepin-7-yl)-1,5-diphenyl-1H-pyrazol-4-carboxamid;
N-(3-Cyclobutyl-2,3,4,5-tetrahydro-1H-3-benzazepin-7-yl)-5-methyl-2-(4-methylphenyl)-2H-1,2,3-triazol-4-carboxamid;
N-(3-Cyclobutyl-2,3,4,5-tetrahydro-1H-3-benzazepin-7-yl)-1-phenyl-5-(1H-pyrrol-1-yl)-1H-pyrazol-4-carboxamid;
N-(3-Cyclobutyl-2,3,4,5-tetrahydro-1H-3-benzazepin-7-yl)-1-(4-fluorphenyl)-5-methyl-1H-pyrazol-4-carboxamid;
N-(3-Cyclobutyl-2,3,4,5-tetrahydro-1H-3-benzazepin-7-yl)-1-phenyl-5-(trifluormethyl)-1H-pyrazol-4-carboxamid;
N-(3-Cyclobutyl-2,3,4,5-tetrahydro-1H-3-benzazepin-7-yl)-1-phenyl-5-propyl-1H-pyrazol-4-carboxamid;
N-(3-Cyclobutyl-2,3,4,5-tetrahydro-1H-3-benzazepin-7-yl)-5-methyl-1-(2-methylphenyl)-1H-pyrazol-4-carboxamid;
N-(3-Cyclobutyl-2,3,4,5-tetrahydro-1H-3-benzazepin-7-yl)-1-(methyloxy)-2-naphthalin-carboxamid;
1-(4-Chlorphenyl)-N-(3-cyclobutyl-2,3,4,5-tetrahydro-1H-3-benzazepin-7-yl)-5-methyl-1H-pyrazol-4-carboxamid;
5-Methyl-N-[3-(3-methylcyclopentyl)-2,3,4,5-tetrahydro-1H-3-benzazepin-7-yl]-2-phenyl-2H-1,2,3-triazol-4-carboxamid;
N-(3-Cyclohexyl-2,3,4,5-tetrahydro-1H-3-benzazepin-7-yl)-5-methyl-2-phenyl-2H-1,2,3-triazol-4-carboxamid;
5-Methyl-N-[3-(2-methylcyclopentyl)-2,3,4,5-tetrahydro-1H-3-benzazepin-7-yl]-2-phenyl-2H-1,2,3-triazol-4-carboxamidtrifluoracetat; oder
N-(3-Cyclopentyl-2,3,4,5-tetrahydro-1H-3-benzazepin-7-yl)-5-methyl-2-phenyl-2H-1,2,3-triazol-4-carboxamidtrifluoracetat;
oder ein pharmazeutisch verträgliches Salz oder Solvat davon.

3. Arzneimittel, umfassend die Verbindung der Formel (I) nach Anspruch 1 oder Anspruch 2 oder ein pharmazeutisch verträgliches Salz oder Solvat davon und einen pharmazeutisch verträglichen Träger oder Exzipienten.

4. Verbindung nach Anspruch 1 oder Anspruch 2 oder ein pharmazeutisch verträgliches Salz oder Solvat davon zur Verwendung bei der Therapie.

5. Verwendung einer Verbindung nach Anspruch 1 oder Anspruch 2 oder eines pharmazeutisch verträglichen Salzes oder Solvats davon bei der Herstellung eines Medikaments zur Behandlung neurologischer Erkrankungen.

## Revendications

1. Composé de formule (I) ou un de ses sels pharmaceutiquement acceptables: 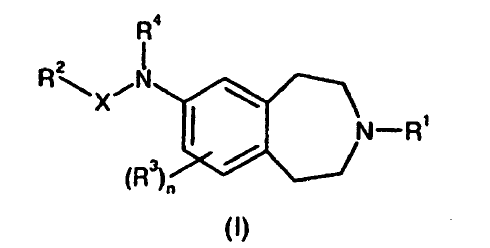 formule dans laquelle :
R¹ représente un groupe -cycloalkyle en C₃ à C₇ facultativement substitué avec un substituant alkyle en C₁ à C₃ ;
R² représente un atome d'hydrogène, un groupe -alkyle en C₁ à C₆, -cycloalkyle en C₃ à C₈, -(alkyle en C₁ à C₆)-(cycloalkyle en C₃ à C₈), -aryle, -hétérocyclyle, -hétéroaryle, -(cycloalkyle en C₃ à C₈)-Y-(cycloalkyle en C₃ à C₈), - (cycloalkyle en C₃ à C₈) -Y-aryle, - (cycloalkyle en C₃ à C₈)-Y-hétéroaryle, -(cycloalkyle en C₃ à C₈)-Y-hétérocyclyle, -aryl-Y-cycloalkyle en C₃ à C₈, -aryl-Y-aryle, -aryl-Y-hétéroaryle, -aryl-Y-hétérocyclyle, -hétéroaryl-Y-(cycloalkyle en C₃ à C₈), -hétéroaryl-Y-aryle, -hétéroaryl-Y-hétéroaryle, -hétéroaryl-Y-hétérocyclyle, -hétérocyclyl-Y-(cycloalkyle en C₃ à C₈), -hétérocyclyl-Y-aryle, -hétérocyclyl-Y-hétéroaryle, -hétérocyclyl-Y-hétérocyclyle ;
X représente une liaison, un groupe CO, SO₂, CONR⁵, COO ou CO(alcényle en C₂ à C₆) ;
Y représente une liaison, un groupe alkyle en C₁ à C₆, CO, CONH, NHCO, O, SO₂, SO₂NH ou NHSO₂ ;
R³ représente un atome d'halogène, un groupe alkyle en C₁ à C₆, alkoxy en C₁ à C₆, cyano, amino ou trifluorométhyle ;
R⁴ et R⁵ représentent indépendamment un atome d'hydrogène, un groupe -alkyle en C₁ à C₆, -cycloalkyle en C₃ à C₈, -aryle, -hétérocyclyle ou -hétéroaryle ;
n est égal à 0, 1 ou 2 ;
lesdits groupes alkyle, cycloalkyle, aryle, hétéroaryle, hétérocyclyle de R², R³ et R⁴ pouvant être facultativement substitués avec un ou plusieurs substituants (par exemple 1, 2 ou 3 substituants) qui peuvent être identiques ou différents et qui sont choisis dans le groupe consistant en des substituants halogéno, hydroxy, cyano, nitro, =O, halogénalkyle en C₁ à C₆, halogénalkoxy en C₁ à C₆, alkyle en C₁ à C₆, alkoxy en C₁ à C₆, aryl(alkoxy en C₁ à C₆), alkylthio en C₁ à C₆, (alkoxy en C₁ à C₆) (alkyle en C₁ à C₆), (cycloalkyle en C₃ à C₇)alkoxy en C₁ à C₆, alcanoyle en C₁ à C₆, (alkoxy en C₁ à C₆)carbonyle, alkylsulfonyle en C₁ à C₆, alkylsulfinyle en C₁ à C₆, alkylsulfonyloxy en C₁ à C₆, (alkyle en C₁ à C₆) sulfonyl (alkyle en C₁ à C₆), sulfonyle, arylsulfonyle, arylsulfonyloxy, arylsulfonyl(alkyle en C₁ à C₆), aryloxy, (alkyle en C₁ à C₆)sulfonamido, alkylamino en C₁ à C₆, (alkyle en C₁ à C₆)amido, -R⁸, -CO₂R⁸, -COR⁸, (alkyle en C₁ à C₆) sulfonamido (alkyle en C₁ à C₆), (alkyle en C₁ à C₆)amido(alkyle en C₁ à C₆), arylsulfonamido, arylcarboxamido, arylsulfonamido(alkyle en C₁ à C₆), arylcarboxamido(alkyle en C₁ à C₆), aryle, aroyle, aroyl (alkyle en C₁ à C₆), aryl (alcanoyle en C₁ à C₆) ou un groupe -NR⁶R⁷, -(alkyle en C₁ à C₆)-NR⁶R⁷, -(cycloalkyle en C₃ à C₈)-NR⁶R⁷, -CONR⁶R⁷, -NR⁶COR⁷, -NR⁶SO₂R⁷, -OCONR⁶R⁷, -NR⁶CO₂R⁷, -NR⁸CONR⁶R⁷ ou -SO₂NR⁶R⁷ (où R⁶, R⁷ et R⁸ représentent indépendamment un atome d'hydrogène, un groupe alkyle en C₁ à C₆, -cycloalkyle en C₃ à C₈, -(alkyle en C₁ à C₆)-(cycloalkyle en C₃ à C₈), aryle, hétérocyclyle ou hétéroaryle, ou bien le groupe -NR⁶R⁷ peut représenter un groupe hétérocyclyle contenant de l'azote, dans lequel lesdits groupes R⁵, R⁶ et R⁷ peuvent être facultativement substitués avec un ou plusieurs substituants (par exemple 1, 2 ou 3 substituants) qui peuvent être identiques ou différents et qui sont choisis dans le groupe consistant en des substituants halogéno, hydroxy, alkyle en C₁ à C₆, alkoxy en C₁ à C₆, cyano, amino, =O et trifluorométhyle) ;
ou leurs produits de solvatation ;
ledit composé n'étant pas la 7-amino-3-cyclopropyl-2,3,4,5-tétrahydro-1H-3-benzazépine.

2. Composé suivant la revendication 1, qui est :
le *N*-(3-cyclobutyl-2,3,4,5-tétrahydro-1*H*-3-benzazépine-7-yl)-4-morpholinecarboxamide ;
le 4-cyano-*N*-(3-cyclobutyl-2,3,4,5-tétrahydro-1*H*-3-benzazépine-7-yl)benzamide ;
le *N*-(3-cyclobutyl-2,3,4,5-tétrahydro-1*H*-3-benzazépine-7-yl)tétrahydro-2*H*-pyranne-4-carboxamide ;
le *N*-(3-cyclobutyl-2,3,4,5-tétrahydro-1*H*-3-benzazépine-7-yl)acétamide ;
le *N*-(3-cyclobutyl-2,3,4,5-tétrahydro-1*H*-3-benzazépine-7-yl)cyclopropanecarboxamide ;
le *N*-(3-cyclobutyl-2,3,4,5-tétrahydro-1*H*-3-benzazépine-7-yl)méthanesulfonamide ;
le 6-[(3-cyclobutyl-2,3,4,5-tétrahydro-1*H*-3-benzazépine-7-yl)amino]-N-méthyl-3-pyridinecarboxamide ;
le *N*-(3-cyclobutyl-2,3,4,5-tétrahydro-1*H*-3-benzazépine-7-yl) -4- (2-pyridinyl)benzamide ;
le *N*-(3-cyclobutyl-2,3,4,5-tétrahydro-1*H*-3-benzazépine-7-yl)-5-hydroxy-2-pyrazinecarboxamide ;
le *N*-(3-cyclobutyl-2,3,4,5-tétrahydro-1*H*-3-benzazépine-7-yl)-4-(1,3-oxazole-4-yl)benzamide ;
le *N*-(3-cyclobutyl-2,3,4,5-tétrahydro-1*H*-3-benzazépine-7-yl)-4-iodobenzamide ;
le *N*-(3-cyclobutyl-2,3,4,5-tétrahydro-1*H*-3-benzazépine-7-yl)-4-(3-pyridinyl)benzamide ;
le trifluoracétate de *N*-(3-cyclobutyl-2,3,4,5-tétrahydro-1*H*-3-benzazépine-7-yl)-5-méthyl-1-phényl-1*H*-pyrazole-4-carboxamide ;
le *N*-(3-cyclobutyl-2,3,4,5-tétrahydro-1*H*-3-benzazépine-7-yl)-4-(1,3-oxazole-5-yl)benzamide ;
le *N*-(3-cyclobutyl-2,3,4,5-tétrahydro-1*H*-3-benzazépine-7-yl)-4-(3,5-diméthyl-4-isoxazolyl)benzamide ;
le *N*-(3-cyclobutyl-2,3,4,5-tétrahydro-1*H*-3-benzazépine-7-yl)-4-imidazo[1,2-a]pyridine-2-ylbenzamide ;
le *N*-(3-cyclobutyl-2,3,4,5-tétrahydro-1*H*-3-benzazépine-7-yl)-1,2,5-oxadiazole-3-carboxamide ;
le *N*-(3-cyclobutyl-2,3,4,5-tétrahydro-1*H*-3-benzazépine-7-yl)-3-isoxazolecarboxamide ;
le *N*-(3-cyclobutyl-2,3,4,5-tétrahydro-1*H*-3-benzazépine-7-yl)cyclohexanecarboxamide ;
le *N*-(3-cyclobutyl-2,3,4,5-tétrahydro-1*H*-3-benzazépine-7-yl)-2-cyclopentylacétamide ;
le *N*-(3-cyclobutyl-2,3,4,5-tétrahydro-1*H*-3-benzazépine-7-yl)-3-méthylbenzamide ;
le *N*-(3-cyclobutyl-2,3,4,5-tétrahydro-1*H*-3-benzazépine-7-yl)-4-fluorobenzamide ;
le *N*-(3-cyclobutyl-2,3,4,5-tétrahydro-1*H*-3-benzazépine-7-yl)-3-méthyl-2-thiophènecarboxamide ;
le *N*-(3-cyclobutyl-2,3,4,5-tétrahydro-1*H*-3-benzazépine-7-yl)bicyclo[4.2.0]octa-1,3,5-triène-7-carboxamide ;
le *N*-(3-cyclobutyl-2,3,4,5-tétrahydro-1*H*-3-benzazépine-7-yl)-2-méthylbenzamide ;
le *N*-(3-cyclobutyl-2,3,4,5-tétrahydro-1*H*-3-benzazépine-7-yl)-4-méthylbenzamide ;
le *N*-(3-cyclobutyl-2,3,4,5-tétrahydro-1*H*-3-benzazépine-7-yl)-2-éthylbutanamide ;
le *N*-(3-cyclobutyl-2,3,4,5-tétrahydro-1*H*-3-benzazépine-7-yl)-3-fluorobenzamide ;
le *N*-(3-cyclobutyl-2,3,4,5-tétrahydro-1*H*-3-benzazépine-7-yl)-4-(5-pyrimidinyl)benzamide ;
le *N*-(3-cyclobutyl-2,3,4,5-tétrahydro-1*H*-3-benzazépine-7-yl)-4-(méthylsulfonyl)benzamide ;
le *N*-(3-cyclobutyl-2,3,4,5-tétrahydro-1*H*-3-benzazépine-7-yl)-6-quinoxalinecarboxamide ;
le *N*-(3-cyclobutyl-2, 3, 4, 5-tétrahydro-1*H*-3-benzazépine-7-yl)-2-méthyl-1,6-naphtyridine-3-carboxamide ;
le *N*-(3-cyclobutyl-2, 3, 4, 5-tétrahydro-1*H*-3-benzazépine-7-yl)-4-(1*H*-tétrazole-1-yl)benzamide ;
le 4-(6-cyano-3-pyridinyl)-N-(3-cyclobutyl-2,3,4,5-tétrahydro-1*H*-3-benzazépine-7-yl)-benzamide ;
le *N*-(3-cyclobutyl-2,3,4,5-tétrahydro-1*H*-3-benzazépine-7-yl)-2-(trifluorométhyl)-1,8-naphtyridine-3-carboxamide ;
le *N*-(3-cyclobutyl-2,3,4,5-tétrahydro-1*H*-3-benzazépine-7-yl)-4-méthylnicotinamide ;
le *N*-(3-cyclobutyl-2,3,4,5-tétrahydro-1*H*-3-benzazépine-7-yl)-6-méthylnicotinamide ;
le *N*-(3-cyclobutyl-2,3,4,5-tétrahydro-1*H*-3-benzazépine-7-yl)nicotinamide ;
le *N*-(3-cyclobutyl-2,3,4,5-tétrahydro-1*H*-3-benzazépine-7-yl)-4-(trifluorométhyl)nicotinamide ;
le *N*-(3-cyclobutyl-2,3,4,5-tétrahydro-1*H*-3-benzazépine-7-yl)-6-(1*H*-pyrazole-1-yl)nicotinamide ;
le *N*-(3-cyclobutyl-2,3,4,5-tétrahydro-1*H*-3-benzazépine-7-yl)-6-(trifluorométhyl)nicotinamide ;
le N-(3-cyclobutyl-2,3,4,5-tétrahydro-1H-3-benzazépine-7-yl)-4-(5-méthyl-1*H*-tétrazole-1-yl)benzamide ;
le *N*-(3-cyclobutyl-2,3,4,5-tétrahydro-1*H*-3-benzazépine-7-yl)-5-(3-pyridinyl)-1*H*-pyrazole-3-carboxamide ;
le *N*-(3-cyclobutyl-2,3,4,5-tétrahydro-1*H*-3-benzazépine-7-yl)-6-(4-morpholinyl)-3-pyridinecarboxamide ;
le *N*-(3-cyclobutyl-2,3,4,5-tétrahydro-1*H*-3-benzazépine-7-yl)-1*H*-1,2,3-benzotriazole-5-carboxamide ;
le *N*-(3-cyclobutyl-2,3,4,5-tétrahydro-1*H*-3-benzazépine-7-yl)-4-(2-pyrazinyl)benzamide ;
le *N*-(3-cyclobutyl-2,3,4,5-tétrahydro-1*H*-3-benzazépine-7-yl)-2-méthyl-1,8-naphtyridine-3-carboxamide ;
le *N*-(3-cyclobutyl-2,3,4,5-tétrahydro-1*H*-3-benzazépine-7-yl)-5-quinoxalinecarboxamide ;
le *N*-(3-cyclobutyl-2,3,4,5-tétrahydro-1*H*-3-benzazépine-7-yl)pyrazolo[1,5-a]pyrimidine-3-carboxamide ;
le *N*-(3-cyclobutyl-2,3,4,5-tétrahydro-1*H*-3-benzazépine-7-yl)-5-pyrimidinecarboxamide ;
le *N*-(3-cyclobutyl-2,3,4,5-tétrahydro-1*H*-3-benzazépine-7-yl)-4-pyridazinecarboxamide ;
le *N*-(3-cyclobutyl-2,3,4,5-tétrahydro-1*H*-3-benzazépine-7-yl)-3-(5-pyrimidinyl)benzamide ;
le *N*-(3-cyclobutyl-2,3,4,5-tétrahydro-1*H*-3-benzazépine-7-yl)-3-(2-pyrazinyl)benzamide ;
le *N*-(3-cyclobutyl-2,3,4,5-tétrahydro-1*H*-3-benzazépine-7-yl)-3-(4-pyrimidinyl)benzamide ;
le *N*-(3-cyclobutyl-2,3,4,5-tétrahydro-1*H*-3-benzazépine-7-yl)-4-(4-pyrimidinyl)benzamide ;
le *N*-(3-cyclobutyl-2,3,4,5-tétrahydro-1*H*-3-benzazépine-7-yl)-5-méthyl-3-isoxazolecarboxamide ;
le 6-cyano-N-(3-cyclobutyl-2,3,4,5-tétrahydro-1H-3-benzazépine-7-yl)-3-pyridinecarboxamide ;
le N-(3-cyclobutyl-2,3,4,5-tétrahydro-1H-3-benzazépine-7-yl)-4-(1H-imidazole-1-yl)benzamide ;
le N-(3-cyclobutyl-2,3,4,5-tétrahydro-1H-3-benzazépine-7-yl)-2-pyrazinecarboxamide ;
le N-(3-cyclobutyl-2,3,4,5-tétrahydro-1H-3-benzazépine-7-yl)-2-méthyl-3-pyridinecarboxamide ;
le N-(3-cyclobutyl-2,3,4,5-tétrahydro-1H-3-benzazépine-7-yl)-3-méthyl-2-pyridinecarboxamide ;
le N-(3-cyclobutyl-2,3,4,5-tétrahydro-1H-3-benzazépine-7-yl)-3-thiophènecarboxamide ;
le N-(3-cyclobutyl-2,3,4,5-tétrahydro-1H-3-benzazépine-7-yl)-3-furannecarboxamide ;
le N-(3-cyclobutyl-2,3,4,5-tétrahydro-1H-3-benzazépine-7-yl)-2-méthyl-3-furannecarboxamide ;
le N-(3-cyclobutyl-2,3,4,5-tétrahydro-1H-3-benzazépine-7-yl)-2,5-diméthyl-3-furannecarboxamide ;
le N-(3-cyclobutyl-2,3,4,5-tétrahydro-1H-3-benzazépine-7-yl)-2-méthyl-5-phényl-3-furannecarboxamide ;
le N-(3-cyclobutyl-2,3,4,5-tétrahydro-1H-3-benzazépine-7-yl)-1,2,3-thiadiazole-4-carboxamide ;
le N-(3-cyclobutyl-2,3,4,5-tétrahydro-1H-3-benzazépine-7-yl)-1,3-thiazole-4-carboxamide ;
le N-(3-cyclobutyl-2,3,4,5-tétrahydro-1H-3-benzazépine-7-yl)-5-méthyl-2-phényl-2H-1,2,3-triazole-4-carboxamide ;
le N-(3-cyclobutyl-2,3,4,5-tétrahydro-1H-3-benzazépine-7-yl)-1-méthyl-1H-imidazole-4-carboxamide ;
le N-(3-cyclobutyl-2,3,4,5-tétrahydro-1H-3-benzazépine-7-yl)-1-méthyl-1H-1,2,3-triazole-4-carboxamide ;
le N-(3-cyclobutyl-2,3,4,5-tétrahydro-1H-3-benzazépine-7-yl)-5-phényl-1,3-oxazole-4-carboxamide ;
le N-(3-cyclobutyl-2,3,4,5-tétrahydro-1H-3-benzazépine-7-yl)-2-phényl-2H-1,2,3-triazole-4-carboxamide ;
le 2-(2,1,3-benzoxadiazole-5-yl)-N-(3-cyclobutyl-2,3,4,5-tétrahydro-1H-3-benzazépine-7-yl)-1,3-thiazole-4-carboxamide ;
le N-(3-cyclobutyl-2,3,4,5-tétrahydro-1H-3-benzazépine-7-yl)-1,5-diméthyl-1H-pyrazole-3-carboxamide ;
le *N*-(3-cyclobutyl-2,3,4,5-tétrahydro-1*H*-3-benzazépine-7-yl)-1,2,5-thiadiazole-3-carboxamide ;
le N-(3-cyclobutyl-2,3,4,5-tétrahydro-1H-3-benzazépine-7-yl)-6-(1H-1,2,4-triazole-1-yl)-3-pyridinecarboxamide ;
le N-(3-cyclobutyl-2,3,4,5-tétrahydro-1H-3-benzazépine-7-yl)-6-phényl-3-pyridinecarboxamide ;
le N-(3-cyclobutyl-2,3,4,5-tétrahydro-1H-3-benzazépine-7-yl)-3,3'-bipyridine-5-carboxamide ;
le N-(3-cyclobutyl-2,3,4,5-tétrahydro-1H-3-benzazépine-7-yl)-6-oxo-1,6-dihydro-3-pyridinecarboxamide ;
le N-(3-cyclobutyl-2,3,4,5-tétrahydro-1H-3-benzazépine-7-yl)-3-méthyl-5-isoxazolecarboxamide ;
le N-(3-cyclobutyl-2,3,4,5-tétrahydro-1H-3-benzazépine-7-yl)-5-méthyl-2-pyrazinecarboxamide ;
le N-(3-cyclobutyl-2,3,4,5-tétrahydro-1H-3-benzazépine-7-yl)-6-oxo-1,6-dihydro-2-pyridinecarboxamide ;
le N-(3-cyclobutyl-2,3,4,5-tétrahydro-1H-3-benzazépine-7-yl)-2,4-diméthyl-1,3-thiazole-5-carboxamide ;
le N-(3-cyclobutyl-2,3,4,5-tétrahydro-1H-3-benzazépine-7-yl)-2-phényl-1,3-thiazole-4-carboxamide ;
le *N*-(3-cyclobutyl-2,3,4,5-tétrahydro-1*H*-3-benzazépine-7-yl)-4-(1,1-dioxo-2-isothiazolidinyl)benzamide ;
le *N*-(3-cyclobutyl-2,3,4,5-tétrahydro-1*H*-3-benzazépine-7-yl)-4-(2-oxo-1-pyrrolidinyl)benzamide ;
le *N*-(3-cyclobutyl-2,3,4,5-tétrahydro-1*H*-3-benzazépine-7-yl)-4-(2-oxo-1-imidazolidinyl)benzamide ;
le *N*-(3-cyclobutyl-2,3,4,5-tétrahydro-1*H*-3-benzazépine-7-yl)-4-(1*H*-pyrazole-1-yl)benzamide ;
le *N*-(3-cyclobutyl-2,3,4,5-tétrahydro-1*H*-3-benzazépine-7-yl)-4-(1*H*-1,2,4-triazole-1-yl)benzamide ;
le *N*-(3-cyclobutyl-2,3,4,5-tétrahydro-1*H*-3-benzazépine-7-yl)-3-(2-oxo-1-pyrrolidinyl)benzamide ;
le *N*-(3-cyclobutyl-2,3,4,5-tétrahydro-1*H*-3-benzazépine-7-yl)-3-(1,1-dioxo-2-isothiazolidinyl)benzamide ;
le *N*-(3-cyclobutyl-2,3,4,5-tétrahydro-1*H*-3-benzazépine-7-yl)-3-(2-oxo-1-imidazolidinyl)benzamide ;
le *N*-(3-cyclobutyl-2,3,4,5-tétrahydro-1*H*-3-benzazépine-7-yl)-3-(1*H*-pyrazole-1-yl)benzamide ;
le *N*-(3-cyclobutyl-2,3,4,5-tétrahydro-1*H*-3-benzazépine-7-yl)-3-(1*H*-1,2,4-triazole-1-yl)benzamide ;
le *N*-(3-cyclobutyl-2,3,4,5-tétrahydro-1*H*-3-benzazépine-7-yl)-*N*-méthyl-2,5-pyrazinedicarboxamide ;
le *N*-(3-cyclobutyl-2,3,4,5-tétrahydro-1*H*-3-benzazépine-7-yl)-4-(4-pyridinyl)benzamide ;
le 6-(4-cyanophényl)-*N*-(3-cyclobutyl-2,3,4,5-tétrahydro-1*H*-3-benzazépine-7-yl)-3-pyridinecarboxamide ;
le *N*-(3-cyclobutyl-2,3,4,5-tétrahydro-1*H*-3-benzazépine-7-yl)-2,3'-bipyridine-5-carboxamide ;
le *N*-(3-cyclobutyl-2,3,4,5-tétrahydro-1*H*-3-benzazépine-7-yl)-6-(5-pyrimidinyl)-3-pyridinecarboxamide ;
le 6- (3-cyanophényl) -N- (3-cyclobutyl-2,3,4,5-tétrahydro-1*H*-3-benzazépine-7-yl)-3-pyridinecarboxamide ;
le *N*-(3-cyclobutyl-2,3,4,5-tétrahydro-1*H*-3-benzazépine-7-yl)-5-(9-fluorophényl)-2-pyridinecarboxamide ;
le 5-(4-cyanophényl)-N-(3-cyclobutyl-2,3,4,5-tétrahydro-1*H*-3-benzazépine-7-yl)-2-pyridinecarboxamide ;
le 6'-cyano-*N*-(3-cyclobutyl-2,3,4,5-tétrahydro-1*H*-3-benzazépine-7-yl)-3,3'-bipyridine-6-carboxamide ;
le N-(3-cyclobutyl-2,3,4,5-tétrahydro-1H-3-benzazépine-7-yl)-5-(5-pyrimidinyl)-2-pyridinecarboxamide ;
le *N*-(3-cyclobutyl-2,3,4,5-tétrahydro-1*H*-3-benzazépine-7-yl)-5-(2-pyrazinyl)-2-pyridinecarboxamide ;
le *N*-(3-cyclobutyl-2,3,4,5-tétrahydro-1*H*-3-benzazépine-7-yl)-2,3'-bipyridine-6'-carboxamide ;
le *N*-(3-cyclobutyl-2,3,4,5-tétrahydro-1*H*-3-benzazépine-7-yl)-5-(1*H*-pyrazole-1-yl)-2-pyridinecarboxamide
le *N*-(3-cyclobutyl-2,3,4,5-tétrahydro-1*H*-3-benzazépine-7-yl)-5-(1*H*-imidazole-1-yl)-2-pyridinecarboxamide ;
le *N*-(3-cyclobutyl-2,3,4,5-tétrahydro-1*H*-3-benzazépine-7-yl)-5-(1*H*-1,2,4-triazole-1-yl)-2-pyridinecarboxamide ;
le N-(3-cyclobutyl-2,3,4,5-tétrahydro-1*H*-3-benzazépine-7-yl)-5-(4-morpholinyl)-2-pyrazinecarboxamide ;
le *N*-(3-cyclobutyl-2,3,4,5-tétrahydro-1*H*-3-benzazépine-7-yl)-5-(1-pyrrolidinyl)-2-pyrazinecarboxamide ;
le *N*-(3-cyclobutyl-2,3,4,5-tétrahydro-1*H*-3-benzazépine-7-yl)-5-(1-pipéridinyl)-2-pyrazinecarboxamide ;
le *N*-(3-cyclobutyl-2,3,4,5-tétrahydro-1*H*-3-benzazépine-7-yl)-5-(tétrahydro-2H-pyranne-4-yloxy)-2-pyrazinecarboxamide ;
le *N*-(3-cyclobutyl-2,3,4,5-tétrahydro-1*H*-3-benzazépine-7-yl)-5-(phényloxy)-2-pyrazinecarboxamide ;
le 5-(4-cyanophényl)-*N*-(3-cyclobutyl-2,3,4,5-tétrahydro-1*H*-3-benzazépine-7-yl)-2-pyrazinecarboxamide ;
le 5-(3-cyanophényl)-N-(3-cyclobutyl-2,3,4,5-tétrahydro-1*H*-3-benzazépine-7-yl)-2-pyrazinecarboxamide ;
le *N*-(3-cyclobutyl-2,3,4,5-tétrahydro-1*H*-3-benzazépine-7-yl)-4-pyridinecarboxamide ;
le *N*-(3-cyclobutyl-2,3,4,5-tétrahydro-1*H*-3-benzazépine-7-yl)-4-biphénylcarboxamide ;
le 3-cyano-N-(3-cyclobutyl-2,3,4,5-tétrahydro-1H-3-benzazépine-7-yl)benzamide ;
le *N*-(3-cyclobutyl-2,3,4,5-tétrahydro-1*H*-3-benzazépine-7-yl)-1,3-diméthyl-1H-pyrazole-5-carboxamide ;
le *N*-(3-cyclobutyl-2,3,4,5-tétrahydro-1*H*-3-benzazépine-7-yl)-3,5-diméthyl-4-isoxazolecarboxamide ;
le 5-bromo-N-(3-cyclobutyl-2,3,4,5-tétrahydro-1H-3-benzazépine-7-yl)-3-pyridinecarboxamide ;
le N-(3-cyclobutyl-2,3,4,5-tétrahydro-1H-3-benzazépine-7-yl)-5-(1H-imidazole-1-yl)-3-pyridinecarboxamide ;
le *N*-(3-cyclobutyl-2,3,4,5-tétrahydro-1*H*-3-benzazépine-7-yl)-N-méthyl-4-(2-pyrazinyl)benzamide ;
le N-(3-cyclobutyl-2,3,4,5-tétrahydro-1H-3-benzazépine-7-yl)-N-méthyl-4-(2-pyridinyl)benzamide ;
le *N*-(3-cyclobutyl-2,3,4,5-tétrahydro-1*H*-3-benzazépine-7-yl)-*N*-méthyl-4-(5-pyrimidinyl)benzamide ;
le *N*-(3-cyclobutyl-2,3,4,5-tétrahydro-1*H*-3-benzazépine-7-yl)-*N*,6-diméthyl-3-pyridinecarboxamide ;
la *N*-(3-cyclobutyl-2,3,4,5-tétrahydro-1*H*-3-benzazépine-7-yl)-N-phénylurée ;
la *N*-(4-cyanophényl)-*N*-(3-cyclobutyl-2,3,4,5-tétrahydro-1*H*-3-benzazépine-7-yl)urée ;
la *N*-1,3-benzodioxole-5-yl-*N*-(3-cyclobutyl-2,3,4,5-tétrahydro-1*H*-3-benzazépine-7-yl)urée ;
la *N*-(3-cyclobutyl-2,3,4,5-tétrahydro-1*H*-3-benzazépine-7-yl)-N-cyclohexylurée ;
la *N*-(3-cyclobutyl-2,3,4,5-tétrahydro-1*H*-3-benzazépine-7-yl)-*N*-(1-méthyléthyl)urée ;
la *N*-(3-cyclobutyl-2,3,4,5-tétrahydro-1*H*-3-benzazépine-7-yl)-*N*-[2-(méthyloxy)phényl]urée;
la *N*-(3-cyclobutyl-2,3,4,5-tétrahydro-1*H*-3-benzazépine-7-yl)-*N*-8-quinolinylurée ;
la *N*-(3-cyclobutyl-2,3,4,5-tétrahydro-1H-3-benzazépine-7-yl)-*N*-(2,2-difluoro-1,3-benzodioxole-4-yl)urée ;
la *N*-(3-cyclobutyl-2,3,4,5-tétrahydro-1*H*-3-benzazépine-7-yl)-*N*-3-pyridinylurée ;
la *N*-(3-cyclobutyl-2,3,4,5-tétrahydro-1*H*-3-benzazépine-7-yl)-*N*-4-pyridinylurée ;
la *N*-(3-cyclobutyl-2,3,4,5-tétrahydro-1*H*-3-benzazépine-7-yl)-*N*-[2-(éthyloxy)phényl]urée;
la *N*-(3-cyclobutyl-2,3,4,5-tétrahydro-1*H*-3-benzazépine-7-yl)-*N*-{2-[(trifluorométhyl)oxy]phényl}urée ;
la *N*-(3-cyclobutyl-2,3,4,5-tétrahydro-1*H*-3-benzazépine-7-yl)-*N*-[2-méthyl-6-(méthyloxy)phényl]urée ;
la *N*-(3-cyanophényl)-*N*-(3-cyclobutyl-2,3,4,5-tétrahydro-1*H*-3-benzazépine-7-yl)urée ;
la *N*-[5-chloro-2-(méthyloxy)phényl]-*N*-(3-cyclobutyl-2,3,4,5-tétrahydro-1*H*-3-benzazépine-7-yl)urée ;
le *N*-(3-cyclobutyl-2,3,4,5-tétrahydro-1*H*-3-benzazépine-7-yl)4-pyrazine-2-ylpipérazine-1-carboxamide ;
le 4-(5-cyanopyridine-2-yl)-N-(3-cyclobutyl-2,3,4,5-tétrahydro-1*H*-3-benzazépine-7-yl)pipérazine-1-carboxamide ;
le 5-chloro-*N*-(3-cyclobutyl-2,3,4,5-tétrahydro-1*H*-3-benzazépine-7-yl)indoline-1-carboxamide ;
le *N-* (3-cyclobutyl-2, 3,4, 5-tétrahydro-1*H*-3-benzazépine-7-yl)-2,3-dihydro-1*H*-indole-1-carboxamide ;
le *N*-(3-cyclobutyl-2,3,4,5-tétrahydro-1*H*-3-benzazépine-7-yl)-3,4-dihydro-1(2*H*)-quinoléinecarboxamide ;
le *N-* (3-cyclobutyl-2, 3,4, 5-tétrahydro-1*H*-3-benzazépine-7-yl)-1,3-dihydro-2*H*-isoindole-2-carboxamide ;
le *N*-(3-cyclobutyl-2,3,4,5-tétrahydro-1*H*-3-benzazépine-7-yl)-3,4-dihydro-2(1*H*)-isoquinoléinecarboxamide ;
le *N*-(3-cyclobutyl-2,3,4,5-tétrahydro-1*H*-3-benzazépine-7-yl)-4-phényl-1-pipéridinecarboxamide ;
le 4-[(4-cyanophényl)oxy]-N-(3-cyclobutyl-2,3,4,5-tétrahydro-1*H*-3-benzazépine-7-yl)-1-pipéridinecarboxamide ;
le *N*-(3-cyclobutyl-2,3,4,5-tétrahydro-1*H*-3-benzazépine-7-yl)-4-(2-pyridinyl)-1-pipéridinecarboxamide ;
le *N-* (3-cyclobutyl-2, 3,4, 5-tétrahydro-1*H*-3-benzazépine-7-yl)-4-(4-pyridinyl)-1-pipéridinecarboxamide
le *N*-(3-cyclobutyl-2,3,4,5-tétrahydro-1*H*-3-benzazépine-7-yl)-4-phényl-1-pipérazinecarboxamide ;
le 4-(4-cyanophényl)*-N-*(3-cyclobutyl-2,3,4,5-tétrahydro-1*H*-3-benzazépine-7-yl)-1-pipérazinecarboxamide ;
le *N*-(3-cyclobutyl-2,3,4,5-tétrahydro-1*H*-3-benzazépine-7-yl)-1-pipéridinecarboxamide ;
le *N*-(3-cyclobutyl-2,3,4,5-tétrahydro-1*H*-3-benzazépine-7-yl)-1-pyrrolidinecarboxamide ;
le *N*-(3-cyclobutyl-2,3,4,5-tétrahydro-1*H*-3-benzazépine-7-yl)-5-fluoro-2,3-dihydro-1*H*-indole-1-carboxamide ;
le *N*-(3-cyclobutyl-2,3,4,5-tétrahydro-1*H*-3-benzazépine-7-yl)-4-(4-fluorophényl)-1-pipéridinecarboxamide ;
le *N*-(3-cyclobutyl-2,3,4,5-tétrahydro-1*H*-3-benzazépine-7-yl)-2-phényl-1-pyrrolidinecarboxamide ;
le 5-cyano-*N*-(3-cyclobutyl-2,3,4,5-tétrahydro-1*H*-3-benzazépine-7-yl)-2,3-dihydro-1*H*-indole-1-carboxamide ;
le 4-(4-cyanophényl)-*N*-(3-cyclobutyl-2,3,4,5-tétrahydro-1*H*-3-benzazépine-7-yl)-1-pipéridinecarboxamide ;
le *N*-(3-cyclobutyl-2,3,4,5-tétrahydro-1*H*-3-benzazépine-7-yl)-1,2,4,5-tétrahydro-3*H*-3-benzazépine-3-carboxamide ;
le 4-(3-cyano-2-pyrazinyl)-*N*-(3-cyclobutyl-2,3,4,5-tétrahydro-1*H*-3-benzazépine-7-yl)-1-pipérazinecarboxamide ;
le *N*-(3-cyclobutyl-2,3,4,5-tétrahydro-1*H*-3-benzazépine-7-yl)-2,3-dihydro-4*H*-1,4-benzoxazine-4-carboxamide ;
le *N*-(3-cyclobutyl-2,3,4,5-tétrahydro-1*H*-3-benzazépine-7-yl)-4-{[4-(méthyloxy)phényl]oxy}-1-pipéridinecarboxamide ;
le *N*-(3-cyclobutyl-2,3,4,5-tétrahydro-1*H*-3-benzazépine-7-yl)-4-[(4-fluorophényl)oxy]-1-pipéridinecarboxamide ;
le *N*-(3-cyclobutyl-2,3,4,5-tétrahydro-1*H*-3-benzazépine-7-yl)-4-(phényloxy)-1-pipéridinecarboxamide ;
le *N*-(3-cyclobutyl-2,3,4,5-tétrahydro-1*H*-3-benzazépine-7-yl)-4-(2-pyridinyloxy)-1-pipéridinecarboxamide ;
le *N*-(3-cyclobutyl-2,3,4,5-tétrahydro-1*H*-3-benzazépine-7-yl)-4-(3-pyridinyloxy)-1-pipéridinecarboxamide ;
le 7-cyano-*N*-(3-cyclobutyl-2,3,4,5-tétrahydro-1*H*-3-benzazépine-7-yl)-2,3-dihydro-4*H*-1,4-benzoxazine-4-carboxamide ;
la *N*-(4-cyanophényl)-*N*-(3-cyclobutyl-2,3,4,5-tétrahydro-1*H*-3-benzazépine-7-yl)-*N*-méthylurée ;
le *N*-(3-cyclobutyl-2,3,4,5-tétrahydro-1*H*-3-benzazépine-7-yl)-2,3-dihydro-1*H*-pyrrolo[2,3-*b*]pyridine-1-carboxamide
le 5-cyano-*N*-(3-cyclobutyl-2,3,4,5-tétrahydro-1*H*-3-benzazépine-7-yl)-1,3-dihydro-2*H*-isoindole-2-carboxamide ;
la 1-{6-[(3-cyclobutyl-2,3,4,5-tétrahydro-1*H*-3-benzazépine-7-yl)amino]-3-pyridinyl}-2-pyrrolidinone ;
le trifluoracétate de *N*-(3-cyclobutyl-2,3,4,5-tétrahydro-1*H*-3-benzazépine-7-yl)-1,3-benzodioxole-5-carboxamide ;
le trifluoracétate de *N*-(3-cyclobutyl-2,3,4,5-tétrahydro-1*H*-3-benzazépine-7-yl)-2-méthyl-1*H*-benzimidazole-5-carboxamide ;
le trifluoracétate de *N*-(3-cyclobutyl-2,3,4,5-tétrahydro-1*H*-3-benzazépine-7-yl)-2-quinoxalinecarboxamide;
le trifluoracétate de *N*-(3-cyclobutyl-2,3,4,5-tétrahydro-1*H*-3-benzazépine-7-yl)-2-(méthyloxy)benzamide ;
le trifluoracétate de *N*-(3-cyclobutyl-2,3,4,5-tétrahydro-1*H*-3-benzazépine-7-yl)pyrazolo[1,5-a]pyridine-3-carboxamide ;
le trifluoracétate de *N*-(3-cyclobutyl-2,3,4,5-tétrahydro-1*H*-3-benzazépine-7-yl)-5-méthyl-1-[4-(méthyloxy)phényl]-1*H*-pyrazole-4-carboxamide ;
le trifluoracétate de *N*-(3-cyclobutyl-2,3,4,5-tétrahydro-1*H*-3-benzazépine-7-yl)-3-(méthyloxy)benzamide ;
le trifluoracétate de *N*-(3-cyclobutyl-2,3,4,5-tétrahydro-1*H*-3-benzazépine-7-yl)-1-benzothiophène-2-carboxamide;
le trifluoracétate de *N*-(3-cyclobutyl-2,3,4,5-tétrahydro-1*H*-3-benzazépine-7-yl)-2-naphtalènecarboxamide;
le trifluoracétate de 1-(4-chlorophényl)-*N*-(3-cyclobutyl-2,3,4,5-tétrahydro-1*H*-3-benzazépine-7-yl)-5-(trifluorométhyl)-1*H*-pyrazole-4-carboxamide ;
le trifluoracétate de *N*-(3-cyclobutyl-2,3,4,5-tétrahydro-1*H*-3-benzazépine-7-yl)-1-(1-méthyléthyl)-1*H*-1,2,3-benzotriazole-5-carboxamide ;
le trifluoracétate de *N*-(3-cyclobutyl-2,3,4,5-tétrahydro-1*H*-3-benzazépine-7-yl)-1-(1(méthyléthyl)-2-(trifluorométhyl)-1*H-*ben zimidazole-5-carboxamide ;
le trifluoracétate de *N*-(3-cyclobutyl-2,3,4,5-tétrahydro-1*H*-3-benzazépine-7-yl)-2,1-benzisoxazole-3-carboxamide;
le trifluoracétate de *N*-(3-cyclobutyl-2,3,4,5-tétrahydro-1*H*-3-benzazépine-7-yl)-1-méthyl-3-propyl-1*H*-pyrazole-5-carboxamide ;
le trifluoracétate de *N*-(3-cyclobutyl-2,3,4,5-tétrahydro-1*H*-3-benzazépine-7-yl)-1,5-diméthyl-3-oxo-2-phényl-2,3-dihydro-1*H-*pyrazole-4-carboxamide ;
le trifluoracétate de *N-* (3-cyclobutyl-2, 3, 4, 5-tétrahydro-1*H-*3-benzazépine-7-yl)-2-méthyl-2*H*-1,2,3-triazole-4-carboxamide;
le trifluoracétate de *N*-(3-cyclobutyl-2,3,4,5-tétrahydro-1*H*-3-benzazépine-7-yl)-1-benzothiophène-3-carboxamide;
le trifluoracétate de *N-*(3-cyclobutyl-2,3,4,5-tétrahydro-1*H*-3-benzazépine-7-yl)-1-phényl-1*H*-1,2,3-triazole-5-carboxamide;
le trifluoracétate de *N*-(3-cyclobutyl-2,3,4,5-tétrahydro-1*H*-3-benzazépine-7-yl)-3-méthyl-5-[4-(méthyloxy)phényl]-1*H-*pyra zole-4-carboxamide ; ,
le trifluoracétate de *N-*(3-cyclobutyl-2,3, 4, 5-tétrahydro-1*H-*3-benzazépine-7-yl)-5-méthyl-1-phényl-1*H*-pyrazole-3-carboxamide ;
le trifluoracétate de *N-*(3-cyclobutyl-2,3,4,5-tétrahydro-1*H*-3-benzazépine-7-yl)-3-[3-(méthyloxy)phényl]-1-phényl-1*H-*pyrazole-4-carboxamide ;
le trifluoracétate de *N-*(3-cyclobutyl-2,3,4,5-tétrahydro-1*H*-3-benzazépine-7-yl)-3-(1,1-diméthyléthyl)-1-méthyl-1*H*-pyrazole-5-carboxamide ;
le trifluoracétate de *N-*(3-cyclobutyl-2,3,4,5-tétrahydro-1*H*-3-benzazépine-7-yl)-5,7-diméthylpyrazolo[1,5-a]pyrimidine-2-carboxamide ;
le trifluoracétate de *N-*(3-cyclobutyl-2,3,4,5-tétrahydro-1*H*-3-benzazépine-7-yl)-3-méthyl-1-phényl-1*H*-pyrazole-5-carboxamide ;
le trifluoracétate de *N*-(3-cyclobutyl-2,3,4,5-tétrahydro-1*H*-3-benzazépine-7-yl)-1-méthyl-1*H*-1,2,3-benzotriazole-5-carboxamide ;
le trifluoracétate de *N*-(3-cyclobutyl-2,3,4,5-tétrahydro-1*H*-3-benzazépine-7-yl)-5-méthyl-1-[(4-méthylphényl)méthyl]-1*H-*pyrazole-3-carboxamide ;
le trifluoracétate de *N*-(3-cyclobutyl-2,3,4,5-tétrahydro-1*H*-3-benzazépine-7-yl)-1-éthyl-3- (2-thiényl)-1*H*-pyrazole-5-carboxamide ;
le trifluoracétate de *N*-(3-cyclobutyl-2,3,4,5-tétrahydro-1*H-*3-benzazépine-7-yl)-1-éthyl-5-méthyl-1*H*-pyrazole-3-carboxamide ;
le trifluoracétate de *N*-(3-cyclobutyl-2,3,4,5-tétrahydro-1*H*-3-benzazépine-7-yl)-4-[(4-fluorophényl)oxy]-1*H*-1,2,3-triazole-5-carboxamide ;
le trifluoracétate de *N*-(3-cyclobutyl-2,3,4,5-tétrahydro-1*H*-3-benzazépine-7-yl)-4-éthyl-1-phényl-1*H*-1,2,3-triazole-5-carboxamide ;
le trifluoracétate de *N*-(3-cyclobutyl-2,3,4,5-tétrahydro-1*H*-3-benzazépine-7-yl)-5-éthyl-1-phényl-1*H*-1,2,3-triazole-4-carboxamide ;
le trifluoracétate de *N*-(3-cyclobutyl-2,3,4,5-tétrahydro-1*H*-3-benzazépine-7-yl)-2-méthyl-5-phényl-2*H*-1,2,3-triazole-4-carboxamide ;
le trifluoracétate de *N*-(3-cyclobutyl-2,3,4,5-tétrahydro-1*H*-3-benzazépine-7-yl)-5-méthyl-1-phényl-1*H*-1,2,3-triazole-4-carboxamide ;
le trifluoracétate de *N*-(3-cyclobutyl-2,3,4,5-tétrahydro-1*H*-3-benzazépine-7-yl)-2*H*-chromène-3-carboxamide ;
le trifluoracétate de *N*-(3-cyclobutyl-2,3,4,5-tétrahydro-1*H*-3-benzazépine-7-yl)-2-méthyl-4,5,6,7-tétrahydro-2*H*-indazole-3-carboxamide ;
le trifluoracétate de 4-[3,4-bis(méthyloxy)phényl]-*N-*(3-cyclobutyl-2,3,4,5-tétrahydro-1*H*-3-benzazépine-7-yl)-1*H-*1,2,3-triazole-5-carboxamide;
le trifluoracétate de *N*-(3-cyclobutyl-2,3,4,5-tétrahydro-1*H*-3-benzazépine-7-yl)-1-(2,2,2-trifluoréthyl)-1*H*-1,2,3-triazole-4-carboxamide ;
le trifluoracétate de *N*-(3-cyclobutyl-2,3,4,5-tétrahydro-1*H*-3-benzazépine-7-yl)-3,5-diméthyl-1-phényl-1*H*-pyrazole-4-carboxamide ;
le trifluoracétate de *N*-(3-cyclobutyl-2,3,4,5-tétrahydro-1*H*-3-benzazépine-7-yl)-1-méthyl-1*H*-pyrazole-4-carboxamide ;
le trifluoracétate de *N*-(3-cyclobutyl-2,3,4,5-tétrahydro-1*H*-3-benzazépine-7-yl)-1-méthyl-3-(trifluorométhyl)1*H*-pyrazole-4-carboxamide ;
le trifluoracétate de 1-(2-chlorophényl)-*N*-(3-cyclobutyl-2,3,4,5-tétrahydro-1*H*-3-benzazépine-7-yl)-5-propyl-1*H*-pyrazole-4-carboxamide ;
le trifluoracétate de 1-(4-cyanophényl)-*N*-(3-cyclobutyl-2,3,4,5-tétrahydro-1*H*-3-benzazépine-7-yl)-3-(trifluorométhyl)-1*H*-pyrazole-4-carboxamide ;
le trifluoracétate de 1-(4-chlorophényl)-*N*-(3-cyclobutyl-2, 3, 4, 5-tétrahydro-1*H*-3-benzazépine-7-yl) -5-propyl-1*H*-pyrazole-4-carboxamide ;
le trifluoracétate de *N*-(3-cyclobutyl-2,3,4,5-tétrahydro-1*H*-3-benzazépine-7-yl)-3,4-dihydro-2*H*-chromène-3-carboxamide ;
le trifluoracétate de *N*-(3-cyclobutyl-2,3,4,5-tétrahydro-1*H*-3-benzazépine-7-yl)-1,3,5-triméthyl-1*H*-pyrazole-4-carboxamide ;
le trifluoracétate de *N*-(3-cyclobutyl-2,3,4,5-tétrahydro-1*H*-3-benzazépine-7-yl)-1-(4-fluorophényl)-3,5-diméthyl-1*H*-pyrazole-4-carboxamide ;
le trifluoracétate de *N*-(3-cyclobutyl-2,3,4,5-tétrahydro-1*H*-3-benzazépine-7-yl)-1-benzofuranne-5-carboxamide ;
le trifluoracétate de *N*-(3-cyclobutyl-2,3,4,5-tétrahydro-1*H*-3-benzazépine-7-yl)-1*H*-indole-3-carboxamide ;
le trifluoracétate de *N*-(3-cyclobutyl-2,3,4,5-tétrahydro-1*H*-3-benzazépine-7-yl)-1*H*-indazole-3-carboxamide ;
le trifluoracétate de *N*-(3-cyclobutyl-2,3,4,5-tétrahydro-1*H*-3-benzazépine-7-yl)-2,3-dihydro-1-benzofuranne-2-carboxamide ;
le trifluoracétate de *N*-(3-cyclobutyl-2,3,4,5-tétrahydro-1*H*-3-benzazépine-7-yl)-1*H*-indole-5-carboxamide ;
le trifluoracétate de *N*-(3-cyclobutyl-2,3,4,5-tétrahydro-1*H*-3-benzazépine-7-yl)-2,3-dihydro-1-benzofuranne-5-carboxamide ;
le trifluoracétate de *N*-(3-cyclobutyl-2,3,4,5-tétrahydro-1*H*-3 -benzazépine-7-yl)-1,3-benzothiazole-6-carboxamide ;
le trifluoracétate de *N*-(3-cyclobutyl-2,3,4,5-tétrahydro-1*H*-3-benzazépine-7-yl)-1*H*-indole-6-carboxamide ;
le trifluoracétate de *N*-(3-cyclobutyl-2,3,4,5-tétrahydro-1*H*-3-benzazépine-7-yl)-1,2,3-benzothiadiazole-5-carboxamide ;
le trifluoracétate de *N*-(3-cyclobutyl-2,3,4,5-tétrahydro-1*H*-3-benzazépine-7-yl)-2,7-diméthylpyrazolo[1,5-a]pyrimidine-6-carboxamide ;
le trifluoracétate de *N*-(3-cyclobutyl-2,3,4,5-tétrahydro-1*H*-3-benzazépine-7-yl)-1-naphtalènecarboxamide ;
le trifluoracétate de *N*-(3-cyclobutyl-2,3,4,5-tétrahydro-1*H*-3-benzazépine-7-yl)-3-quinoléinecarboxamide ;
le trifluoracétate de *N*-(3-cyclobutyl-2,3,4,5-tétrahydro-1*H*-3-benzazépine-7-yl)-4-(méthyloxy)-2-quinoléinecarboxamide ;
le trifluoracétate de *N*-(3-cyclobutyl-2,3,4,5-tétrahydro-1*H*-3-benzazépine-7-yl)-2,1,3-benzothiadiazole-5-carboxamide ;
le trifluoracétate de *N*-(3-cyclobutyl-2,3,4,5-tétrahydro-1*H*-3-benzazépine-7-yl)-2,3-dihydro-1,4-benzodioxine-2-carboxamide ;
le trifluoracétate de *N*-(3-cyclobutyl-2,3,4,5-tétrahydro-1*H*-3-benzazépine-7-yl)-6-(méthyloxy)-2-naphtalènecarboxamide ;
le trifluoracétate de *N*-(3-cyclobutyl-2,3,4,5-tétrahydro-1*H*-3-benzazépine-7-yl)-3-isoquinoléinecarboxamide ;
le trifluoracétate de *N*-(3-cyclobutyl-2,3,4,5-tétrahydro-1*H*-3-benzazépine-7-yl)-1-méthyl-1*H*-indole-2-carboxamide ;
le trifluoracétate de *N*-(3-cyclobutyl-2,3,4,5-tétrahydro-1*H*-3-benzazépine-7-yl)-1-benzofuranne-2-carboxamide ;
le trifluoracétate de *N*-(3-cyclobutyl-2,3,4,5-tétrahydro-1*H*-3-benzazépine-7-yl)-4-(méthyloxy)benzamide ;
le trifluoracétate de *N*-(3-cyclobutyl-2,3,4,5-tétrahydro-1*H*-3-benzazépine-7-yl)-1,3-diphényl-1*H*-pyrazole-4-carboxamide;
le trifluoracétate de *N*-(3-cyclobutyl-2,3,4,5-tétrahydro-1*H*-3-benzazépine-7-yl)-2-quinoléinecarboxamide;
le trifluoracétate de *N*-(3-cyclobutyl-2,3,4,5-tétrahydro-1*H*-3-benzazépine-7-yl)-2,3-dihydro-1,4-benzodioxine-6-carboxamide;
le trifluoracétate de *N*-(3-cyclobutyl-2,3,4,5-tétrahydro-1*H*-3-benzazépine-7-yl)-4-(méthylsulfonyl)benzamide ;
le trifluoracétate de *N*-(3-cyclobutyl-2,3,4*,*5*-*tétrahydro-1*H*-3-benzazépine-7-yl)-2-(phényloxy)benzamide ;
le trifluoracétate de *N*-(3-cyclobutyl-2,3,4,5-tétrahydro-1*H*-3-benzazépine-7-yl)-3-(phényloxy)benzamide ;
le trifluoracétate de *N*-(3-cyclobutyl-2,3,4,5-tétrahydro-1*H*-3-benzazépine-7-yl)-4-(phényloxy)benzamide ;
le trifluoracétate de *N*-(3-cyclobutyl-2,3,4,5-tétrahydro-1*H*-3-benzazépine-7-yl)-2-(trifluorométhyl)benzamide ;
le trifluoracétate de *N*-(3-cyclobutyl-2,3,4,5-tétrahydro-1*H*-3-benzazépine-7-yl)-3-(trifluorométhyl)benzamide ;
le trifluoracétate de *N*-(3-cyclobutyl-2,3,4,5-tétrahydro-1*H*-3-benzazépine-7-yl)-4-(trifluorométhyl)benzamide ;
le trifluoracétate de *N*-(3-cyclobutyl-2,3,4,5-tétrahydro-1*H*-3-benzazépine-7-yl)-2-biphénylcarboxamide ;
le trifluoracétate de (2*E*)-*N*-(3-cyclobutyl-2,3,4,5-tétrahydro-1*H*-3-benzazépine-7-yl)-3-phényl-2-propénamide ;
le trifluoracétate de *N*-(3-cyclobutyl-2,3,4,5-tétrahydro-1*H*-3-benzazépine-7-yl)-1*H*-benzimidazole-2-carboxamide ;
le trifluoracétate de (2E)-*N*-(3-cyclobutyl-2,3,4,5-tétrahydro-1*H*-3-benzazépine-7-yl)-3-[2-(méthyloxy)phényl]-2-propénamide ;
le trifluoracétate de (2*E*)-*N*-(3-cyclobutyl-2,3,4,5-tétrahydro-1*H*-3-benzazépine-7-yl)-3-[3-(méthyloxy)phényl]-2-propénamide ;
le trifluoracétate de (2*E*)-*N*-(3-cyclobutyl-2,3,4,5-tétrahydro-1*H*-3-benzazépine-7-yl)-3-[4-(méthyloxy)phényl]-2-propénamide ;
le trifluoracétate de (2*E*)-*N*-cyclobutyl-2,3,4,5-tétrahydro-1*H*-3-benzazépine-7-yl)-3-(2-fluorophényl)-2-propénamide;
le trifluoracétate de (2*E*)-*N*-(3-cyclobutyl-2,3,4,5-tétrahydro-1*H*-3-benzazépine-7-yl)-3-(3-fluorophényl)-2-propénamide;
le trifluoracétate de (2*E*)-*N*-(3-cyclobutyl-2,3,4,5-tétrahydro-1*H*-3-benzazépine-7-yl)-3-(4-fluorophényl)-2-propénamide;
le trifluoracétate de 3,5-dichloro-*N*-(3-cyclobutyl-2,3,4,5-tétrahydro-1*H*-3-benzazépine-7-yl)benzamide;
le trifluoracétate de *N*-(3-cyclobutyl-2,3,4,5-tétrahydro-1*H*-3-benzazépine-7-yl)-1-éthyl-3-méthyl-1*H*-pyrazole-5-carboxamide;
le trifluoracétate de *N*-(3-cyclobutyl-2,3,4,5-tétrahydro-1*H*-3-benzazépine-7-yl)-5-(2-furannyl)-1*H*-pyrazole-3-carboxamide ;
le trifluoracétate de *N*-(3-cyclobutyl-2,3,4,5-tétrahydro-1*H*-3-benzazépine-7-yl)-1-(3,4-dichlorophényl)-3,5-diméthyl-1*H*-pyrazole-4-carboxamide ;
le trifluoracétate de *N*-(3-cyclobutyl-2,3,4,5-tétrahydro-1*H*-3-benzazépine-7-yl)-3-(méthyloxy)-2-naphtalènecarboxamide ;
le *N*-(3-cyclobutyl-2,3,4,5-tétrahydro-1*H*-3-benzazépine-7-yl)-1-(4-fluorophényl)-5-phényl-1*H*-pyrazole-3-carboxamide ;
le *N*-(3-cyclobutyl-2,3,4,5-tétrahydro-1*H*-3-benzazépine-7-yl)-1,5-diphényl-1*H*-pyrazole-4-carboxamide ;
le *N*-(3-cyclobutyl-2,3,4,5-tétrahydro-1*H*-3-benzazépine-7-yl)-5-méthyl-2-(4-méthylphényl)-2*H*-1,2,3-triazole-4-carboxamide ;
le *N*-(3-cyclobutyl-2,3,4,5-tétrahydro-1*H*-3-benzazépine-7-yl)-1-phényl-5-(1*H*-pyrrole-1-yl)-1*H*-pyrazole-4-carboxamide ;
le *N*-(3-cyclobutyl-2,3,4,5-tétrahydro-1*H*-3-benzazépine-7-yl)-1-(4-fluorophényl)-5-méthyl-1*H*-pyrazole-4-carboxamide ;
le *N*-(3-cyclobutyl-2,3,4,5-tétrahydro-1*H*-3-benzazépine-7-yl)-1-phényl-5-(trifluorométhyl)-1*H*-pyrazole-4-carboxamide ;
le *N*-(3-cyclobutyl-2,3,4,5-tétrahydro-1*H*-3-benzazépine-7-yl)-1-phényl-5-propyl-1*H*-pyrazole-4-carboxamide ;
le *N*-(3-cyclobutyl-2,3,4,5-tétrahydro-1*H*-3-benzazépine-7-yl)-5-méthyl-1-(2-méthylphényl)-1*H*-pyrazole-4-carboxamide ;
le *N*-(3-cyclobutyl-2,3,4,5-tétrahydro-1*H*-3-benzazépine-7-yl)-1-(méthyloxy)-2-naphtalènecarboxamide ;
le 1-(4-chlorophényl)-N-(3-cyclobutyl-2,3,4,5-tétrahydro-1H-3-benzazépine-7-yl)-5-méthyl-1H-pyrazole-4-carboxamide ;
le 5-méthyl-*N*-[3-(3-méthylcyclopentyl)-2,3,4,5-tétrahydro-1*H-*3-benzazépine-7-yl]-2-phényl-2*H*-1,2,3-triazole-4-carboxamide ;
le *N*-(3-cyclohexyl-2, 3,4, 5-tétrahydro-1*H*-3-benzazépine-7-yl)-5-méthyl-2-phényl-2*H*-1,2,3-triazole-4-carboxamide ;
le trifluoracétate de 5-méthyl-*N*-[3-(2-méthylcyclopentyl)-2,3,4,5-tétrahydro-1*H*-3-benzazépine-7-yl]-2-phényl-2*H*-1,2,3-triazole-4-carboxamide ; ou
le trifluoracétate de *N*-(3-cyclopentyl-2,3,4,5-tétrahydro-1*H-*3-benzazépine-7-yl)-5-méthyl-2-phényl-2H-1,2,3-triazole-4-carboxamide ;
ou un de ses sels ou produits de solvatation pharmaceutiquement acceptables.

3. Composition pharmaceutique, qui comprend le composé de formule (I) suivant la revendication 1 ou la revendication 2, ou un de ses sels ou produits de solvatation pharmaceutiquement acceptables et un support ou excipient pharmaceutiquement acceptable.

4. Composé suivant la revendication 1 ou la revendication 2 ou un de ses sels ou produits de solvatation pharmaceutiquement acceptables, destiné à être utilisé en thérapie.

5. Utilisation d'un composé suivant la revendication 1 ou la revendication 2 ou d'un de ses sels ou produits de solvatation pharmaceutiquement acceptables, dans la production d'un médicament destiné au traitement de maladies neurologiques.
